(19) Europäisches Patentamt
European Patent Office
Office européen des brevets

(11) EP 3 241 903 A1

(12) **EUROPEAN PATENT APPLICATION**
published in accordance with Art. 153(4) EPC

(43) Date of publication:
**08.11.2017 Bulletin 2017/45**

(21) Application number: **15875390.5**

(22) Date of filing: **30.10.2015**

(51) Int Cl.:
***C12N 15/113*** *(2010.01)* ***A61K 31/7088*** *(2006.01)*
***A61K 47/12*** *(2006.01)* ***A61K 48/00*** *(2006.01)*

(86) International application number:
**PCT/JP2015/080849**

(87) International publication number:
**WO 2016/108264 (07.07.2016 Gazette 2016/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **29.12.2014 JP 2014267087**
**10.04.2015 JP 2015081298**

(71) Applicant: **Bonac Corporation**
**Kurume-shi, Fukuoka 839-0861 (JP)**

(72) Inventors:
- **YAMADA, Taimu**
**Kurume-shi**
**Fukuoka 839-0861 (JP)**
- **TOYOFUKU, Hidekazu**
**Kurume-shi**
**Fukuoka 839-0861 (JP)**

(74) Representative: **J A Kemp**
**14 South Square**
**Gray's Inn**
**London WC1R 5JJ (GB)**

(54) **COMPOSITION CONTAINING NUCLEIC ACID MOLECULE STABLY**

(57) The present invention provides a composition containing a nucleic acid molecule and a buffer, and having the features of (a) being in the form of a solution at ambient temperature; and (b) a content of the nucleic acid molecule after storage at 25°C, relative humidity 60% for 4 weeks, of not less than 80% relative to the content at the time of start of the storage.

FIG 1

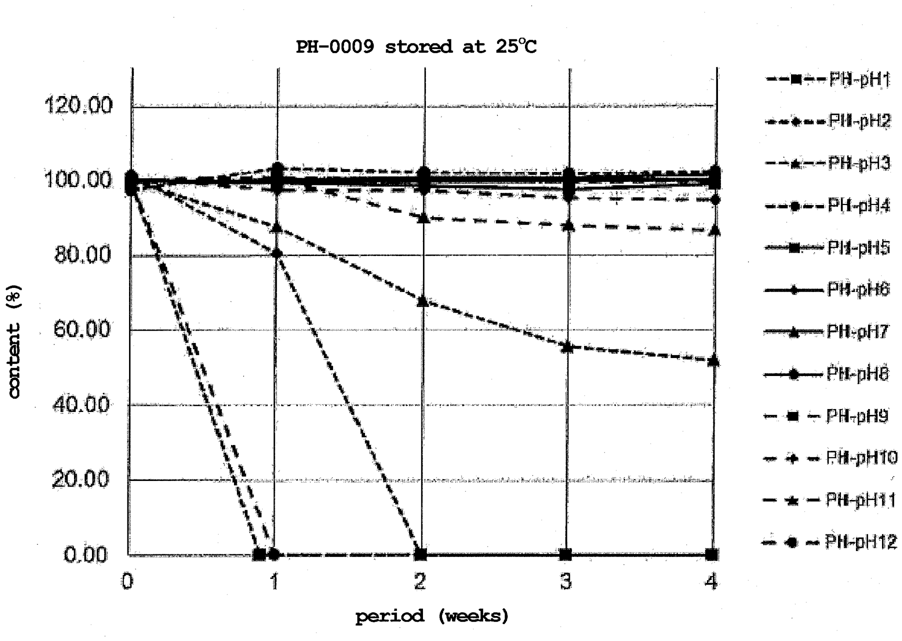


EP 3 241 903 A1

Printed by Jouve, 75001 PARIS (FR)

## Description

[Technical Field]

The present invention relates to a composition containing a nucleic acid molecule having a biological activity, for example, a nucleic acid molecule that controls expression of a target gene or function of a target protein, which is a novel composition, particularly a pharmaceutical composition, showing improved stability of the nucleic acid molecule, as well as a production method thereof, and a method for stabilizing the nucleic acid molecule in a liquid composition.

[Background Art]

As short chain nucleic acid molecules having a biological activity, antisense nucleic acid, siRNA, shRNA, microRNA (miRNA), decoy nucleic acid, ribozyme, aptamer and the like are known, and the development of pharmaceutical products utilizing them is ongoing (e.g., patent documents 1-3).

Since these nucleic acids are susceptible to decomposition in solutions and unstable, handling at ambient temperature was extremely difficult. Therefore, freeze-drying and a method including adding 50% ethanol to a Tris-EDTA (TE) buffer and storing same without freezing at -20°C have generally been adopted.

[Document List]

[Patent documents]

patent document 1: JP-B-2708960
patent document 2: JP-B-3626503
patent document 3: US Patent No. 7,511,131

[SUMMARY OF THE INVENTION]

[Problems to be Solved by the Invention]

Under such circumstances, the development of a stable nucleic acid preparation superior in handleability and capable of stably maintaining a nucleic acid as an active ingredient at ambient temperature has been desired.

The problem of the present invention is to provide a pharmaceutical composition containing the nucleic acid as an active ingredient, which is a novel pharmaceutical composition with improved stability of the active ingredient, and a production method thereof.

[Means of Solving the Problems]

The present inventors have conducted intensive studies in an attempt to solve the aforementioned problem and found that the stability of a nucleic acid can be improved remarkably and unexpectedly by using a buffer capable of adjusting the pH of a nucleic acid molecule solution to fall within a particular range, which resulted in the completion of the present invention.

Accordingly, the present invention is as follows.

[1] A composition comprising a nucleic acid molecule and a buffer, and having the following features:

(a) being in the form of a solution at ambient temperature; and
(b) a content of the nucleic acid molecule after storage at 25°C, relative humidity 60% for 4 weeks, of not less than 80% relative to the content at the time of start of the storage.

[2] The composition of [1], wherein the content of the nucleic acid molecule after storage at 40°C, relative humidity 75% for 4 weeks is not less than 80% relative to the content at the time of start of the storage.
[3] The composition of [1] or [2], wherein the content of the nucleic acid molecule after storage at 60°C for 4 weeks is not less than 60% relative to the content at the time of start of the storage.
[4] The composition of any of [1] to [3], wherein the buffer adjusts the pH of the composition to not less than 4.0 and not more than 9.0.

[5] The composition of any of [1] to [3], wherein the buffer adjusts the pH of the composition to not less than 5.5 and not more than 7.5.
[6] The composition of any of [1] to [3], wherein the buffer adjusts the pH of the composition to not less than 6.0 and not more than 7.0.
[7] The composition of any of [1] to [6], wherein the buffer comprises one or more buffering agents selected from sodium hydrogen phosphate, sodium dihydrogen phosphate, disodium hydrogen phosphate, sodium chloride, arginine hydrochloride, sodium citrate, trisodium citrate dihydrate, monosodium L-glutamate, sodium acetate, sodium carbonate, sodium hydrogen carbonate, sodium lactate, monopotassium phosphate, sodium hydroxide, meglumine, glycine, citric acid, and acetic acid.
[8] The composition of any of [1] to [7], wherein the buffer comprises citric acid and/or phosphoric acid.
[9] The composition of any of [1] to [8], wherein the aforementioned nucleic acid molecule is a single-stranded nucleic acid molecule or a double-stranded nucleic acid molecule.
[10] The composition of any of [1] to [9], wherein the aforementioned nucleic acid molecule is a DNA molecule, an RNA molecule, or a chimeric nucleic acid molecule of DNA and RNA.
[11] The composition of any of [1] to [10], wherein the nucleotide number of the aforementioned nucleic acid molecule is 10 - 300.
[12] The composition of any of [1] to [11], wherein the aforementioned nucleic acid molecule comprises a sequence that controls expression of a target gene or function of a target protein.
[13] The composition of any of [1] to [11], comprising a nucleic acid molecule comprising a sequence that controls expression of a target gene.
[14] The composition of any of [1] to [13], wherein the aforementioned nucleic acid molecule is antisense nucleic acid, siRNA or shRNA, miRNA, ribozyme, decoy nucleic acid or aptamer.
[15] The composition of any of [1] to [14], which is a pharmaceutical composition.
[16] A method of producing the composition of any of [1] to [15], comprising dissolving the aforementioned nucleic acid molecule in a buffer adjusting a pH of the composition to not less than 6.0 and not more than 7.0, and storing the solution at ambient temperature.
[17] A method for stabilizing a nucleic acid molecule in a composition, comprising dissolving the nucleic acid molecule in a buffer adjusting a pH of the composition to not less than 6.0 and not more than 7.0, and storing the solution at ambient temperature.
[18] The method of [16] or [17], wherein the buffer comprises citric acid and/or phosphoric acid.
[19] The method of any of [16] to [18], wherein the composition is a pharmaceutical composition.

[Effect of the Invention]

According to the present invention, a novel composition, particularly a pharmaceutical composition, superior in handlability, wherein a nucleic acid molecule as an active ingredient has improved stability, can be provided.

[Brief Description of the Drawings]

Fig. 1 shows the results of a stability test at 25°C of PH-0009 solution prepared using a buffer at each pH.
Fig. 2 shows the results of a stability test at 40°C of PH-0009 solution prepared using a buffer at each pH.
Fig. 3 shows the results of a stability test at 60°C of PH-0009 solution prepared using a buffer at each pH.
Fig. 4 shows the results of a stability test at 40°C of PH-0009 solution prepared using a citrate buffer at each concentration.
Fig. 5 shows the results of a stability test at 60°C of PH-0009 solution prepared using a citrate buffer at each concentration.
Fig. 6 shows the results of a stability test of a 10 mg/mL PH-0009 solution.
Fig. 7 shows the results of a stability test of NK-7006 solution prepared using a 0.05 M citrate buffer.
Fig. 8 shows the results of a stability test of NK-7007 solution prepared using a 0.05 M citrate buffer.
Fig. 9 shows the results of a stability test of PK-7006 solution prepared using a 0.05 M citrate buffer.
Fig. 10 shows the results of a stability test of PK-7015 solution prepared using a 0.05 M citrate buffer.
Fig. 11 shows the results of a stability test of PH-7069 solution prepared using a 0.05 M citrate buffer.
Fig. 12 shows the results of a stability test of Kynamro-7001 solution prepared using a 0.05 M citrate buffer.
Fig. 13 shows the results of a stability test of PH-7081 solution prepared using a 0.05 M citrate buffer.
Fig. 14 shows the results of a stability test of NI-7001 solution prepared using a 0.05 M citrate buffer.
Fig. 15 shows the results of a stability test of NM-7001 solution prepared using a 0.05 M citrate buffer.

Fig. 16 shows the results of a stability test of Macugen-7001 solution prepared using a 0.05 M citrate buffer.
Fig. 17 shows the results of a stability test at 60°C of PK-7006 solutions prepared using a 0.05 M citrate buffer, a 0.05 M phosphate buffer and a 0.05 M citrate-phosphate (5:5) buffer at each pH.
Fig. 18 shows the results of a stability test at 60°C of NK-7006 solutions prepared using a 0.05 M citrate buffer, a 0.05 M phosphate buffer and a 0.05 M citrate-phosphate (5:5) buffer at each pH.
Fig. 19 shows the results of a stability test at 60°C of PH-7069 solutions prepared using a 0.05 M citrate buffer, and a 0.05 M phosphate buffer and 0.05 M citrate-phosphate (5:5) buffer at each pH.
Fig. 20 shows the results of a stability test at 60°C of NI-7001 solutions prepared using a 0.05 M citrate buffer, a 0.05 M phosphate buffer and a 0.05 M citrate-phosphate (5:5) buffer at each pH.
Fig. 21 shows the results of a stability test at 60°C of NM-7001 solutions prepared using a 0.05 M citrate buffer, a 0.05 M phosphate buffer and a 0.05 M citrate-phosphate (5:5) buffer at each pH.
Fig. 22 shows the results of a stability test at 60°C of Kynamro-7001 solutions prepared using a 0.05 M citrate buffer, a 0.05 M phosphate buffer and a 0.05 M citrate-phosphate (5:5) buffer at each pH.
Fig. 23 shows the results of a stability test at 60°C of Macugen-7001 solutions prepared using a 0.05 M citrate buffer, a 0.05 M phosphate buffer and a 0.05 M citrate-phosphate (5:5) buffer at each pH.

[Description of Embodiments]

The present invention provides a nucleic acid molecule containing composition capable of stably storing a nucleic acid molecule having a biological activity in the form of a solution at ambient temperature (hereinafter to be also referred to as "the composition of the present invention"). As used herein, the "ambient temperature" means a temperature range of 15 - 30°C, and "stably storing" means that not less than 80% of the nucleic acid molecule at the time of start of the storage (on preparation of composition) is stored without decomposition for (1) not less than 4 weeks, preferably (2) not less than 12 weeks (about 3 months), more preferably (3) not less than 200 weeks (about 3.7 years). Such storage stability can be each confirmed or predicted from the results of the following stability test.

(1) The content of nucleic acid molecule in a composition after storage at 25°C, relative humidity 60% for 4 weeks is not less than 80% relative to the content at the time of start of the storage.
(2) The content of nucleic acid molecule in a composition after storage at 40°C, relative humidity 75% for 4 weeks is not less than 80% relative to the content at the time of start of the storage.
(3) The content of nucleic acid molecule in a composition after storage at 60°C for 4 weeks is not less than 60%, preferably not less than 70%, more preferably not less than 80% relative to the content at the time of start of the storage.

As used herein, the content of the nucleic acid molecule in the composition is determined by using a solution (100%) obtained by dissolving nucleic acid molecule in the same amount as a test sample in water for injection, and a solution obtained by mixing said solution and water for injection at a ratio of 9:1, 8:2, 7:3 and 6:4 (90%, 80%, 70% and 60%, respectively) as calibration curve samples, applying 10 μL each of the calibration curve samples to HPLC to measure peak areas, plotting the measured values of respective calibration curve samples with the theoretical content (%) on the horizontal axis (X) and the peak area on the vertical axis (Y), obtaining a regression line (Y=aX+b) (calibration curve) by the least squares method, and applying the peak area of the test sample measured by HPLC under the same conditions to the calibration curve to give a theoretical content (%). The measurement conditions of the above-mentioned HPLC are as follows. detector: ultraviolet absorptiometer (measurement wavelength: 254 nm)

column: X-Bridge OST C18 (2.5 μm, 4.6×50 mm)
column temperature: 40°C
mobile phase A: 50 mM TEAA (pH 7.0), 0.5% Acetonitrile
mobile phase B: 100% Acetonitrile
mobile phase feed: concentration gradient is controlled by

changing the mixing ratio of mobile phase A and mobile phase B as follows.

Table 1

| time after injection (min) | mobile phase A (vol%) | mobile phase B (vol%) |
|---|---|---|
| 0→12 | 100→60 | 0→40 |
| flow: 1.0 mL/min | | |

Preferably, the composition of the present invention has a content of the nucleic acid molecule in the composition after storage at 60°C for 4 weeks of not less than 60%, preferably not less than 70%, more preferably not less than 80%, further preferably not less than 85%, particularly preferably not less than 90%, relative to the content at the time of start of the storage.

1. Nucleic acid molecule

The nucleic acid molecule contained in the composition of the present invention is not particularly limited as long as it is an oligonucleotide or polynucleotide containing deoxyribonucleotide (DNA) and/or ribonucleotide (RNA) as constituent unit(s), and may be constituted of DNA or RNA alone, or a chimeric nucleic acid of DNA and RNA. The nucleic acid molecule may be single stranded or double stranded. When it is double stranded, it may be any of DNA double stranded, RNA double stranded, DNA-RNA hybrid. In addition, it is widely applicable to nucleic acid-derived structures (molecular corpuscle constituted of nucleic acid such as LNA, DNA, RNA and the like, specifically chimeric nucleic acid, hetero double-stranded nucleic acid or triple stranded nucleic acid structure etc.).

The number of bases in the nucleic acid molecule contained in the composition of the present invention is generally 10 - 300, preferably 10 - 200, more preferably 10-150, further preferably 15 - 100, particularly preferably 20 - 80. In the present specification, "siRNA", "shRNA", "miRNA", and "ribozyme" are, unless otherwise specified, names based on function, which may be constituted solely of RNA, or one or more (e.g., 1 - 30, 1 - 20, 1 - 10, 1 - 5 (1, 2, 3, 4, 5)) nucleotides are optionally substituted by DNA.

Preferably, the nucleic acid molecule contained in the composition of the present invention is a molecule having a biological activity, such as a molecule containing a nucleotide sequence that controls expression of target gene or function of target protein and the like. As used herein, "control" encompasses both upregulation (promotion of expression or function) and downregulation (suppression of expression or function). Examples of the nucleic acid molecule containing a nucleotide sequence that controls expression of target gene include antisense nucleic acid, siRNA, shRNA, miRNA, ribozyme and the like. Examples of the nucleic acid molecule that suppresses function of target protein include aptamer, decoy nucleic acid and the like.

An antisense nucleic acid refers to a nucleic acid consisting of target mRNA (or initial transcription product thereof) or a base sequence capable of hybridizing with target miRNA (or initial transcription product thereof) under physiological conditions of a cell that expresses the target mRNA or the target miRNA, and capable of inhibiting translation into a protein encoded by the mRNA by steric hindrance or decomposition of the target mRNA (or inhibiting splicing of initial transcription product thereof), or capable of inhibiting control of gene expression by miRNA by inhibiting or decomposing the target miRNA.

The length of the target region of antisense nucleic acid is not particularly limited as long as translation into a protein and control of gene expression by miRNA can be inhibited by hybridization of the antisense nucleic acid and, for example, a short length is about 10 bases and a long length is the complete sequence of mRNA or initial transcription product. In consideration of problem of easy synthesis, antigenicity, intracellular transferability and the like, about 10 - about 40 base length, particularly about 15 - about 30 base length, is preferable, though the length is not limited thereto.

As the antisense nucleic acid, a nucleic acid targeting any known mRNA can be used. A preferable example is an antisense nucleic acid against mRNA encoding a protein that potentially becomes a drug discovery target for a human disease. Specific examples of the antisense nucleic acid relating to human disease include, but are not limited to, an antisense nucleic acid targeting mRNA (or initial transcription product thereof) of ApoB100 (hypercholesterolemia), dystrophin (muscular dystrophy), STAT3 (malignant lymphoma) and the like, and an antisense nucleic acid targeting miRNA and the like of miR-122 (hepatitis C) and the like.

More specific examples of the antisense nucleic acid include, but are not limited to, mipomersen shown in SEQ ID NO: 1 (trade name: Kynamro; provided that RNA is 2'-O-methoxyethylated and cytosine and uracil are 5-methylated in launched drugs) (antisense nucleic acid against ApoB100 mRNA). 5'-GCCUCagtctgcttcGCACC-3' (SEQ ID NO: 1)
(upper case letters show RNA, and lower case letters show DNA)

An antisense nucleic acid can be prepared by determining a target sequence based on cDNA sequence or genomic DNA sequence, and synthesizing a sequence complementary thereto by using a commercially available DNA/RNA automatic synthesizer (Applied Biosystems, Beckman Instruments etc.).

siRNA is a double-stranded oligo RNA consisting of RNA having a sequence complementary to the nucleotide sequence of mRNA of the target gene or a partial sequence thereof (hereinafter target nucleotide sequence), and a complementary strand thereof. Also, a single-stranded RNA in which a sequence complementary to a target nucleotide sequence (first sequence) and a complementary sequence thereof (second sequence) are linked via a hairpin loop portion, and the double-stranded structure of the first sequence and the second sequence is formed by the hairpin loop type structure (small hairpin RNA: shRNA), is also one of preferable embodiments of siRNA. Furthermore, a dumbbell-type nucleic acid in which both ends of the double-stranded structure of the first sequence and the second sequence are closed with a loop structure is also one of preferable embodiments.

siRNA/shRNA may have an overhang at the 5'-terminus or 3'-terminus of either one or both of the sense strand and the antisense strand. An overhang is formed by the addition of one to several (e.g., 1, 2 or 3) bases to the terminal of a sense strand and/or an antisense strand.

While the base length of siRNA/shRNA is not particularly limited as long as RNA interference can be induced, for example, one side strand has a 10 - 50 base length, preferably 15 - 30 base length, more preferably 21 - 27 base length.

As siRNA/shRNA, siRNA/shRNA targeting any known mRNA can be used and, for example, siRNA/shRNA against mRNA encoding a protein that potentially becomes a drug discovery target for a human disease is preferable. Specific examples of siRNA/shRNA relating to human diseases include, but are not limited to, siRNA/shRNA and the like targeting connective tissue growth factor (CTGF) (fibrosis), respiratory syncytial virus (RSV) nucleocapsid (RSV infections), RTP801 (diabetic macular edema), transthyretin (amyloidosis), collagen-specific chaperone (HSP47) (cirrhosis) and the like.

siRNA can be obtained by chemical synthesis using a conventionally-known method or production using gene recombination technology. It is also possible to use a commercially available nucleic acid as appropriate.

For example, siRNA can be appropriately designed using a commercially available software (e.g., RNAi Designer; Invitrogen) based on the base sequence information of mRNA to be the target. It can be prepared by synthesizing each of the sense strand and antisense strand of a target sequence on mRNA by a commercially available DNA/RNA automatic synthesizer (Applied Biosystems, Beckman Instruments etc.), denaturing them in a suitable annealing buffer at about 90 - about 95°C for about 1 min and annealing them at about 30 - about 70°C for about 1 - about 8 hr.

miRNA is an endogenous non-coding RNA (ncRNA) of about 20 - 25 bases, which is encoded on the genome. It does not cleave target mRNA like siRNA, but controls translation by recognizing the 3' untranslated region (UTR) of the target mRNA. The miRNA in the present invention encompasses an endogenous miRNA that acts on the target mRNA in the cytoplasm and inhibits translation into protein and one that acts in the nucleus and decomposes mRNA in an RNase H-dependent manner by a gapmer structure having RNA oligomers at both ends and a DNA oligomer in the center part.

As miRNA, any known miRNA can be used. Preferred is, for example, miRNA targeting mRNA encoding a protein that potentially becomes a drug discovery target for a human disease or a precursor thereof. Specific examples of miRNA relating to human disease include, but are not limited to, let-7 (lung cancer), miR-15a (B-cell chronic lymphocytic leukemia), miR-143 (colorectal cancer), miR-139 (pancreatic cancer) and precursor thereof and the like.

More specific examples of miRNA include, but are not limited to, human let7a-1 precursor shown in SEQ ID NO: 2.

5'-

UGGGAUGAGGUAGUAGGUUGUAUAGUUUUAGGGUCACACCCACCACUGGGAGAUAACUAUACA

AUCUACUGUCUUUCCUA-3' (SEQ ID NO: 2)

miRNA can be obtained by isolating from a mammalian cell (human cell etc.) by using a conventionally-known method, or chemical synthesis, or production using gene recombination technology. It is also possible to use commercially available nucleic acid as appropriate.

As for miRNA, for example, double-stranded miRNA or single-stranded precursor thereof can be produced by obtaining the base sequence information of the object miRNA from miRBase database etc. and, based on the information, in the same manner as in the chemical synthesis of siRNA.

Aptamer is a nucleic acid molecule having an activity to bind to a target molecule such as protein and the like and control (generally inhibit) the function thereof.

The length of the aptamer is not particularly limited, and may be generally about 16 - about 200 nucleotides. For example, it may be not more than about 100 nucleotides, preferably not more than about 50 nucleotides, more preferably not more than about 40 nucleotides.

As the aptamer, an aptamer targeting any known protein can be used. Preferred is, for example, an aptamer targeting a protein that potentially becomes a drug discovery target for a human disease. Specific examples of the aptamer to a protein relating to a human disease include, but are not limited to, aptamers to vascular endothelium growth factor (VEGF) (age-related macular degeneration), factor IXa (suppression of blood coagulation in coronary artery disease), nerve growth factor (NGF) (pain), basic fibroblast growth factor (FGF2) (rheumatoid arthritis) and the like, and the like.

More specific examples of the aptamer include, but are not limited to, pegaptanib shown in SEQ ID NO: 3 (trade name: macugen (registered trade mark); all pyrimidine nucleotides are 2'-fluorinated and a part of purine nucleotide is 2'-methoxylated) (aptamer to VEGF protein). 5'-CGGAAUCAGUGAAUGCUUAUACAUCCGt-3' (SEQ ID NO: 3) (t is 3',3'-dT)

Aptamer can be obtained, for example, by the following procedures. That is, oligonucleotides (e.g., about 60

bases) are first randomly synthesized using a DNA/RNA automatic synthesizer and an oligonucleotide pool is produced. Then, oligonucleotides binding to the object protein are separated with an affinity column. The separated oligonucleotides are amplified by PCR, and the aforementioned selection process is performed again. This process is repeated about 5 times or more to select aptamers having strong affinity for the object protein.

Ribozyme is a nucleic acid molecule having an enzyme activity to cleave nucleic acid. A ribozyme having the broadest utility is self splicing RNA found in infectious RNA such as viroid, virusoid and the like, and hammerhead type, hairpin type and the like are known. A target mRNA alone can be specifically cleaved by forming a sequence complementary to a desired cleavage site of mRNA, with several bases each of the both ends (about 10 bases in total) adjacent to a part having a hammerhead structure.

Ribozyme can be prepared by determining the target sequence based on the cDNA sequence or genomic DNA sequence, and synthesizing a sequence complementary thereto by using a commercially available DNA/RNA automatic synthesizer (Applied Biosystems, Beckman Instruments etc.).

Decoy nucleic acid is a double-stranded DNA molecule having a base length of about 20 bases and having a nucleotide sequence to which a transcription factor specifically binds, and controls (suppression in the case of transcription activation factor, promotion in the case of transcription suppressing factor) expression of the target gene of the transcription factor by trapping the transcription factor.

As the decoy nucleic acid, a decoy nucleic acid targeting any known transcription factor can be used. Preferred is, for example, a decoy nucleic acid targeting a transcription factor that potentially becomes a drug discovery target for a human disease. Specific examples of the decoy nucleic acid to a transcription factor relating to a human disease include, but are not limited to, decoy nucleic acid to NFκB (atopic dermatitis, vascular restenosis, rheumatoid arthritis) and the like, and the like.

Decoy nucleic acid can be obtained by chemical synthesis using a conventionally-known method.

For example, decoy nucleic acid can be appropriately designed based on the base sequence information of the binding consensus sequence of the transcription factor to be the target. It can be prepared by synthesizing each of the sense strand and antisense strand by a commercially available DNA/RNA automatic synthesizer (Applied Biosystems, Beckman Instruments etc.), denaturing them in a suitable annealing buffer at about 90 - about 95°C for about 1 min, and annealing them at about 30 - about 70°C for about 1 - about 8 hr.

In one preferable embodiment, the nucleic acid molecule contained in the composition of the present invention may be a single-stranded nucleic acid molecule described in WO 2012/017919, WO 2013/103146, WO 2012/005368, WO 2013/077446, WO 2013/133393 and the like.

These single-stranded nucleic acid molecules are nucleic acid molecules in which a region containing a sequence that controls expression of the target gene and a region containing a sequence complementary to the sequence are inked directly or via a linker. Specific examples of the linker include, but are not limited to, a linker having a non-nucleotide structure containing at least one of the pyrrolidine skeleton and piperidine skeleton, a linker constituted of a nucleotide residue and/or a non-nucleotide residue, a linker having a non-nucleotide structure such as an amino acid residue, a polyamine residue, a polycarboxylic acid residue and the like, and the like. Specific examples of the aforementioned single-stranded nucleic acid molecule containing the linker include, but are not limited to, the following.

I. Single-stranded nucleic acid molecule containing sequence controlling expression of target gene (hereinafter sometimes to be abbreviated as expression control sequence), which contains linker having non-nucleotide structure containing at least one of pyrrolidine skeleton and piperidine skeleton.

(1) ssPN molecule

As one embodiment of the aforementioned single-stranded nucleic acid molecule, a single-stranded nucleic acid molecule (hereinafter to be also referred to as "ssPN molecule") having region (X), linker region (Lx) and region (Xc), wherein the aforementioned linker region (Lx) is linked between the aforementioned region (X) and the aforementioned region (Xc),
the aforementioned region (Xc) is complementary to the aforementioned region (X),
at least one of the aforementioned region (X) and the aforementioned region (Xc) contains the aforementioned expression control sequence, and the aforementioned linker region (Lx) contains a non-nucleotide structure containing at least one of the pyrrolidine skeleton and the piperidine skeleton, which is described in WO 2012/017919, can be mentioned.

In the aforementioned ssPN molecule, the aforementioned expression control sequence is a sequence that exhibits, for example, an activity of controlling the expression of the aforementioned target gene when the ssPN molecule is introduced into a cell in vivo or in vitro. The aforementioned expression control sequence is not particularly limited, and can be set as appropriate depending on the kind of a target gene. As the aforementioned expression control sequence, for example, a sequence involved in RNA interference caused by siRNA can be used as appropriate. That is, RNA sequence of a strand of the aforementioned siRNA, which is bound to the target mRNA, can be used as the aforementioned expression control sequence.

The aforementioned expression control sequence is, for example, preferably at least 90% complementary, more preferably 95% complementary, still more preferably 98% complementary, and particularly preferably 100% complementary to a predetermined region of the aforementioned target gene. When such complementarity is satisfied, for example, an off-target effect can be reduced sufficiently.

As specific examples, when the target gene is TGF-β1, for example, a 18-base length sequence shown in SEQ ID NO: 4 can be used as the above-mentioned expression control sequence. 5'-UAUGCUGUGUGUACUCUG-3' (SEQ ID NO: 4)

In the aforementioned ssPN molecule, the aforementioned linker region (Lx) may have, for example, a non-nucleotide structure containing the aforementioned pyrrolidine skeleton, or a non-nucleotide structure containing the aforementioned piperidine skeleton, or both a non-nucleotide structure containing the aforementioned pyrrolidine skeleton and a non-nucleotide structure containing the aforementioned piperidine skeleton. The aforementioned ssPN molecule can suppress, for example, side effects such as interferon induction in vivo and exhibits excellent nuclease resistance.

In the aforementioned ssPN molecule, the aforementioned pyrrolidine skeleton may be, for example, the skeleton of a pyrrolidine derivative wherein one or more carbon atoms constituting the 5-membered ring of pyrrolidine is/are substituted, and when substituted, for example, a carbon atom other than the C-2 carbon is preferable. The aforementioned carbon may be substituted by, for example, a nitrogen atom, an oxygen atom or a sulfur atom. The aforementioned pyrrolidine skeleton may contain, for example, a carbon-carbon double bond or a carbon-nitrogen double bond in the 5-membered ring of pyrrolidine. In the aforementioned pyrrolidine skeleton, the carbon atom and nitrogen atom constituting the 5-membered ring of pyrrolidine may be bonded to, for example, a hydrogen atom or the below-mentioned substituent. The aforementioned linker region (Lx) may be bonded to, for example, the aforementioned region (X) and the aforementioned region (Xc) via any group in the aforementioned pyrrolidine skeleton, which is preferably any one carbon atom or any one nitrogen atom of the aforementioned 5-membered ring, preferably, the 2-position carbon (C-2) atom or nitrogen atom of the aforementioned 5-membered ring. Examples of the aforementioned pyrrolidine skeleton include proline skeleton, prolinol skeleton and the like. Since the aforementioned proline skeleton, prolinol skeleton and the like are, for example, in vivo substances and reduced form thereof, they are also superior in safety.

In the aforementioned ssPN molecule, as the aforementioned piperidine skeleton, for example, the skeleton of a piperidine derivative, wherein one or more carbon atoms constituting the 6-membered ring of piperidine are substituted, can be mentioned. When it is substituted, for example, a carbon atom other than C-2 carbon is preferable. The aforementioned carbon atom may be substituted by, for example, a nitrogen atom, an oxygen atom or a sulfur atom. The aforementioned piperidine skeleton may also contain, for example, in the 6-membered ring of piperidine, for example, a carbon-carbon double bond or a carbon-nitrogen double bond. In the aforementioned piperidine skeleton, the carbon atom and nitrogen atom constituting the 6-membered ring of piperidine may be bonded to, for example, a hydrogen atom or the below-mentioned substituent. The aforementioned linker region (Lx) may also be bonded to, for example, the aforementioned region (X) and the aforementioned region (Xc) via any group of the aforementioned piperidine skeleton, and preferably, the 2-position carbon (C-2) atom and nitrogen atom of the aforementioned 6-membered ring.

The aforementioned linker regions may be composed of, for example, the non-nucleotide residue(s) having the aforementioned non-nucleotide structure only, or may contain the non-nucleotide residue(s) having the aforementioned non-nucleotide structure and the nucleotide residue(s).

In the aforementioned ssPN molecule, the aforementioned linker region is represented, for example, by the following formula (I):

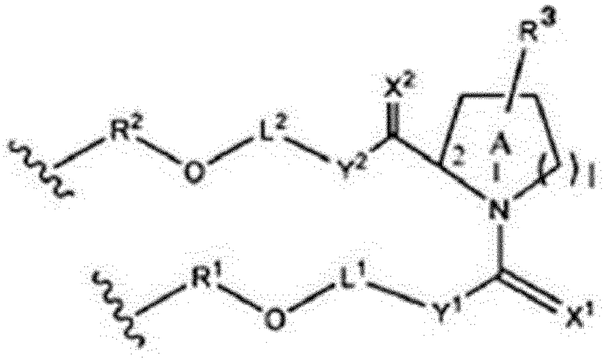

・・・(I)

In the aforementioned formula (I), for example,

$X^1$ and $X^2$ are each independently $H_2$, O, S, or NH;
$Y^1$ and $Y^2$ are each independently a single bond, $CH_2$, NH, O, or S;
$R^3$ is a hydrogen atom or a substituent which is bonded to C-3, C-4, C-5 or C-6 on ring A,
$L^1$ is an alkylene chain having n atoms, and a hydrogen atom on an alkylene carbon atom may or may not be substituted with OH, $OR^a$, $NH_2$, $NHR^a$, $NR^aR^b$, SH, or $SR^a$, or,
$L^1$ is a polyether chain obtained by substituting at least one carbon atom on the aforementioned alkylene chain with oxygen atom,
provided that: when $Y^1$ is NH, O, or S, an atom bound to $Y^1$ in $L^1$ is carbon, an atom bound to $OR^1$ in $L^1$ is carbon, and an oxygen atoms are not adjacent to each other;
$L^2$ is an alkylene chain having m atoms, and a hydrogen atom on an alkylene carbon atom may or may not be substituted with OH, $OR^c$, $NH_2$, $NHR^c$, $NR^cR^d$, SH, or $SR^c$, or
$L^2$ is a polyether chain obtained by substituting at least one carbon atom on the aforementioned alkylene chain with an oxygen atom,
provided that: when $Y^2$ is NH, O, or S, an atom bound to $Y^2$ in $L^2$ is carbon, an atom bound to $OR^2$ in $L^2$ is carbon, and oxygen atoms are not adjacent to each other;
$R^a$, $R^b$, $R^c$, and $R^d$ are each independently a substituent or a protecting group; 1 is 1 or 2;
m is an integer in the range from 0 to 30;
n is an integer in the range from 0 to 30;
in ring A, one carbon atom other than the aforementioned C-2 on the ring A may be substituted by a nitrogen atom, an oxygen atom or a sulfur atom, and may contain, in the aforementioned ring A, a carbon-carbon double bond or a carbon-nitrogen double bond, and
the aforementioned regions (Yc) and (Y) are each linked to the aforementioned linker region (Ly) via $-OR^1-$ or $-OR^2-$, wherein $R^1$ and $R^2$ may or may not be present, and when they are present, $R^1$ and $R^2$ are each independently a nucleotide residue or the aforementioned structure (I).

In the aforementioned formula (I), for example, $X^1$ and $X^2$ are each independently $H_2$, O, S, or NH. In the aforementioned formula (I), "$X^1$ is $H_2$" means that $X^1$ forms $CH_2$ (a methylene group) together with a carbon atom to which $X^1$ binds. The same applies to $X^2$.

In the aforementioned formula (I), $Y^1$ and $Y^2$ are each independently a single bond, $CH_2$, NH, O, or S.

In the aforementioned formula (I), in ring A, 1 is 1 or 2; when 1 = 1, ring A is a 5-membered ring, for example, the aforementioned pyrrolidine skeleton. The aforementioned pyrrolidine skeleton is, for example, proline skeleton, prolinol skeleton or the like, and exemplified by the divalent structures thereof. When 1 = 2, ring A is a 6-membered ring, for example, the aforementioned piperidine skeleton. In ring A, one carbon atom other than C-2 on ring A may be substituted by a nitrogen atom, an oxygen atom or a sulfur atom. Ring A may contain, in ring A, a carbon-carbon double bond or a carbon-nitrogen double bond. Ring A may be, for example, L type or D type.

In the aforementioned formula (I), $R^3$ is a hydrogen atom or substituent bonded to C-3, C-4, C-5 or C-6 on ring A. When $R^3$ is the aforementioned substituent, substituent $R^3$ may be one or more, or may be absent. When $R^3$ is present in plurality, they may be the same or different.

The substituent $R^3$ is, for example, halogen, OH, $OR^4$, $NH_2$, $NHR^4$, $NR^4R^5$, SH, $SR^4$, oxo group (=O) and the like.

$R^4$ and $R^5$ are, for example, each independently a substituent or a protecting group, and may be the same or different. Examples of the aforementioned substituent include halogen, alkyl, alkenyl, alkynyl, haloalkyl, aryl, heteroaryl, arylalkyl, cycloalkyl, cycloalkenyl, cycloalkylalkyl, cyclylalkyl, hydroxyalkyl, alkoxyalkyl, aminoalkyl, heterocyclylalkenyl, heterocyclylalkyl, heteroarylalkyl, silyl, silyloxyalkyl and the like. The same applies hereinafter. The substituent $R^3$ may be selected from the substituents recited above.

The aforementioned protecting group is a functional group that inactivates, for example, a highly-reactive functional group. Examples of the protecting group include known protecting groups. Regarding the aforementioned protecting group, for example, the description in the literature (J. F. W. McOmie, "Protecting Groups in Organic Chemistry", Plenum Press, London and New York, 1973) can be incorporated herein. The aforementioned protecting group is not particularly limited, and examples thereof include a tert-butyldimethylsilyl group (TBDMS), a bis(2-acetoxyethyloxy)methyl group (ACE), a triisopropylsilyloxymethyl group (TOM), a 1-(2-cyanoethoxy)ethyl group (CEE), a 2-cyanoethoxymethyl group (CEM), a tolylsulfonylethoxymethyl group (TEM), and a dimethoxytrityl group (DMTr). When $R^3$ is $OR^4$, the aforementioned protecting group is not particularly limited, and examples thereof include a TBDMS group, an ACE group, a TOM group, a CEE group, a CEM group, and a TEM group. Other examples of the protecting group include silyl-containing groups to be shown later. The same applies hereinafter.

In the aforementioned formula (I), $L^1$ is an alkylene chain consisting of n atoms. A hydrogen atom(s) on the aforementioned alkylene carbon atom(s) may or may not be substituted with, for example, OH, $OR^a$, $NH_2$, $NHR^a$, $NR^aR^b$, SH, or $SR^a$. Alternatively, $L^1$ may be a polyether chain obtained by substituting at least one carbon atom on the aforementioned alkylene chain with an oxygen atom. The aforementioned polyether chain is, for example, polyethylene glycol.

When $Y^1$ is NH, O, or S, an atom bound to $Y^1$ in $L^1$ is carbon, an atom bound to $OR^1$ in $L^1$ is carbon, and oxygen atoms are not adjacent to each other. That is, for example, when $Y^1$ is O, this oxygen atom and the oxygen atom in $L^1$ are not adjacent to each other, and the oxygen atom in $OR^1$ and the oxygen atom in $L^1$ are not adjacent to each other.

In the aforementioned formula (I), $L^2$ is an alkylene chain consisting of m atoms. A hydrogen atom(s) on the aforementioned alkylene carbon atom(s) may or may not be substituted with, for example, OH, $OR^c$, $NH_2$, $NHR^c$, $NR^cR^d$, SH, or $SR^c$. Alternatively, $L^2$ may be a polyether chain obtained by substituting at least one carbon atom on the aforementioned alkylene chain with an oxygen atom. When $Y^2$ is NH, O, or S, an atom bound to $Y^2$ in $L^2$ is carbon, an atom bound to $OR^2$ in $L^2$ is carbon, and oxygen atoms are not adjacent to each other. That is, for example, when $Y^2$ is O, this oxygen atom and the oxygen atom in $L^2$ are not adjacent to each other, and the oxygen atom in $OR^2$ and the oxygen atom in $L^2$ are not adjacent to each other.

n of $L^1$ and m of $L^2$ are not particularly limited, and the lower limit of each of them may be 0, for example, and the upper limit of the same is not particularly limited. For example, n and m can be set as appropriate depending on a desired length of the aforementioned linker region (Lx). For example, from the view point of manufacturing cost, yield, and the like, n and m are each preferably 0 to 30, more preferably 0 to 20, and still more preferably 0 to 15. n and m may be the same (n = m) or different. n + m is, for example, 0 to 30, preferably 0 to 20, and more preferably 0 to 15.

For example, $R^a$, $R^b$, $R^c$ and $R^d$ are each independently a substituent or a protecting group. Examples of the aforementioned substituent and the aforementioned protecting group are the same as above.

In the aforementioned formula (I), hydrogen atoms each independently may be substituted with, for example, a halogen such as Cl, Br, F, or I.

The aforementioned regions (Xc) and (X) are each linked, for example, to the aforementioned linker region (Lx) via $-OR^1$-or $-OR^2$-. $R^1$ and $R^2$ may or may not be present. When $R^1$ and $R^2$ are present, $R^1$ and $R^2$ are each independently a nucleotide residue or the structure represented by the aforementioned formula (I). When $R^1$ and/or $R^2$ are/is the aforementioned nucleotide residue, the aforementioned linker region (Lx) is composed of, for example, the aforementioned non-nucleotide residue having the structure of the aforementioned formula (I) excluding the nucleotide residue $R^1$ and/or $R^2$, and the aforementioned nucleotide residue(s). When $R^1$ and/or $R^2$ are/is the structure represented by the aforementioned formula (I), the structure of the aforementioned linker region (Xc) is such that, for example, two or more of the aforementioned non-nucleotide residues having the structure of the aforementioned formula (I) are linked to each other. The number of the structures of the aforementioned formula (I) may be, for example, 1, 2, 3, or 4. When the linker region (Lx) includes a plurality of the aforementioned structures, the structures of the aforementioned (I) may be linked, for example, either directly or via the aforementioned nucleotide residue(s). On the other hand, when $R^1$ and $R^2$ are not present, the aforementioned linker region (Lx) is composed of, for example, the aforementioned non-nucleotide residue having the structure of the aforementioned formula (I) alone.

The combination of the aforementioned regions (Xc) and (X) with $-OR^1$- and $-OR^2$- is not particularly limited, and may be, for example, any of the following conditions.

Condition (1):

the aforementioned regions (Xc) and (X) are linked to the structure of the aforementioned formula (I) via $-OR^2$- and $-OR^1$-, respectively.

Condition (2):

the aforementioned regions (Xc) and (X) are linked to the structure of the aforementioned formula (I) via $-OR^1$- and $-OR^2$-, respectively.

Examples of the structure of the aforementioned formula (I) include the structures of the following formulae (I-1) to (I-9). In the following formulae, n and m are the same as in the aforementioned formula (I). In the following formulae, q is an integer of 0 - 10.

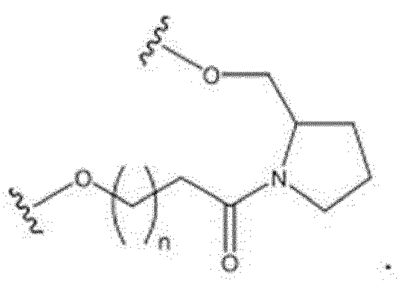
· · · (I-1)

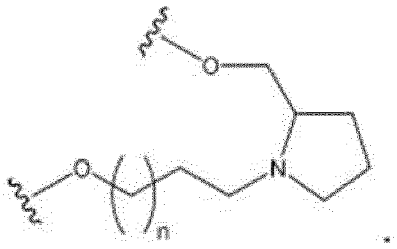
· · · (I-2)

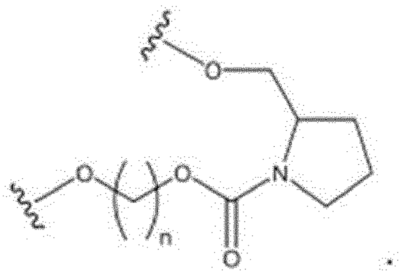
· · · (I-3)

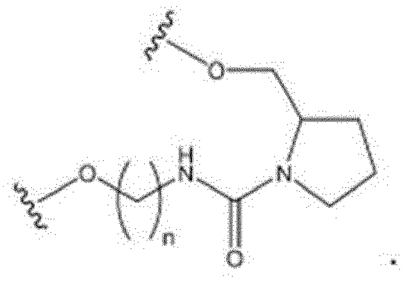
· · · (I-4)

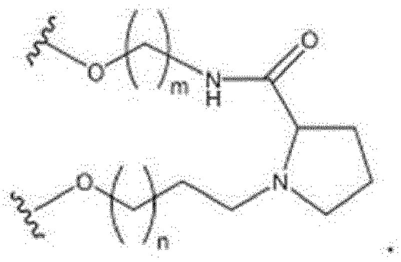
· · · (I-5)

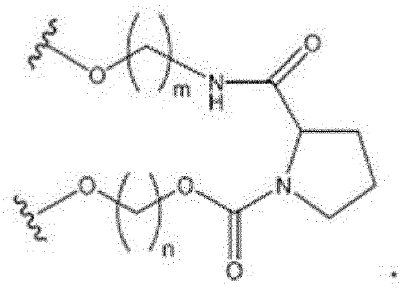
· · · (I-6)

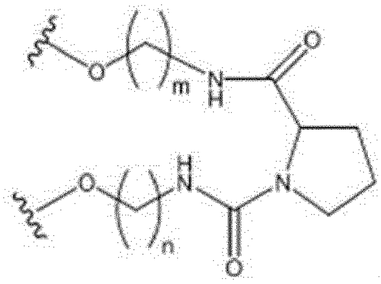
· · · (I-7)

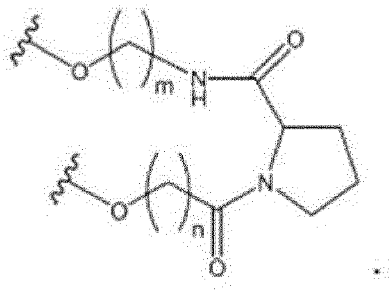
· · · (I-8)

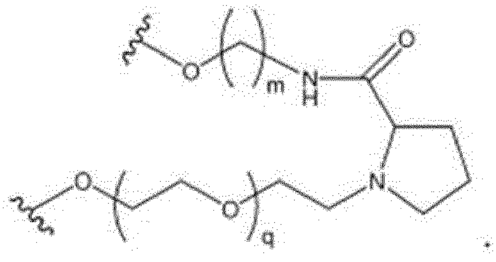
· · · (I-9)

In the aforementioned formulae (I-1) to (I-9), n, m and q are not particularly limited, and are as described above. Specific example thereof is the aforementioned formula (I-1) wherein n = 8, n=3 in the aforementioned (I-2), n=4 or 8 in the aforementioned formula (I-3), n=7 or 8 in the aforementioned (I-4), n=3 and m=4 in the aforementioned formula (I-5), n=8 and m=4 in the aforementioned (I-6), n=8 and m=4 in the aforementioned formula (I-7), n=5 and m=4 in the aforementioned (I-8), and q=1 and m=4 in the aforementioned formula (I-9). One embodiment (n=8) of the aforementioned

formula (I-4) is shown in the following formula (I-4a), and one embodiment (n=5, m=4) of the aforementioned formula (I-8) is shown in the following formula (I-8a).

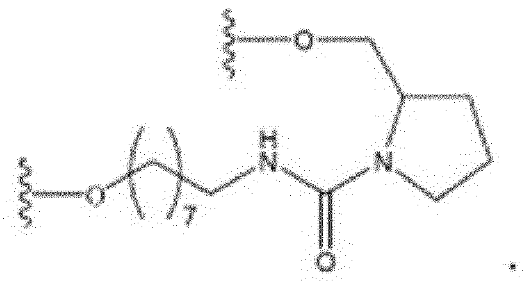

・・・(I-4a)

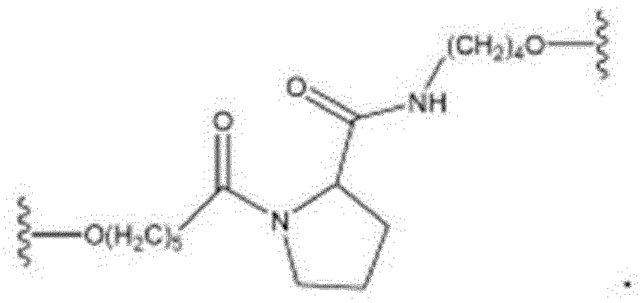

・・・(I-8a)

In the aforementioned ssPN molecule, the aforementioned region (Xc) is complementary to the aforementioned region (X). Thus, in the aforementioned ssPN molecule, a double strand can be formed by fold-back of the aforementioned region (Xc) toward the region (X) and self-annealing of the aforementioned regions (Xc) and (X).

In the aforementioned ssPN molecule, for example, only the aforementioned region (Xc) may fold back to form a double strand with the aforementioned region (X), or another double strand may be formed in another region. Hereinafter, the former ssPN molecule, i.e., the ssPN molecule in which double strand formation occurs at one location is referred to as a "first ssPN molecule", and the latter ssPN molecule, i.e., the ssPN molecule in which double strand formation occurs at two locations is referred to as a "second ssPN molecule". Examples of the aforementioned first and second ssPN molecules are given below. It should be noted, however, that the present invention is not limited to these illustrative examples.

(1-1) First ssPN molecule

The aforementioned first ssPN molecule is, for example, a molecule including the aforementioned region (X), the aforementioned region (Xc), and the aforementioned linker region (Lx).

The aforementioned first ssPN molecule may include the aforementioned region (Xc), the aforementioned linker region (Lx), and the aforementioned region (X) in this order from, for example, the 5'-side to the 3'-side, or may include the aforementioned region (Xc), the aforementioned linker region (Lx), and the aforementioned region (X) in this order from the 3'-side to the 5'-side.

In the aforementioned first ssPN molecule, the aforementioned region (Xc) is complementary to the aforementioned region (X). It is only necessary that the aforementioned region (Xc) has a sequence complementary to the entire region or part of the aforementioned region (X). Preferably, the aforementioned region (Xc) includes or is composed of a sequence complementary to the entire region or part of the region (X). The aforementioned region (Xc) may be, for example, perfectly complementary to the entire region or part of the aforementioned region (X), or one or a few bases in the region (Xc) may be noncomplementary to the same. Preferably, the region (Xc) is perfectly complementary to the same. The aforementioned expression "one or a few bases" means, for example, 1 to 3 bases, preferably 1 base or 2 bases.

In the aforementioned first ssPN molecule, the aforementioned expression control sequence is included in at least one of the aforementioned regions (Xc) and (X), as described above. The aforementioned first ssPN molecule may include, for example, one expression control sequence or two or more expression control sequences mentioned above.

In the latter case, the aforementioned first ssPN molecule may include, for example: two or more identical expression control sequences for the same target gene; two or more different expression control sequences for the same target gene; or two or more different expression control sequences for different target genes. When the aforementioned first ssPN molecule includes two or more expression control sequences mentioned above, the positions of the respective expression control sequences are not particularly limited, and they may be in one region or different regions selected from the aforementioned regions (X) and (Xc). When the aforementioned first ssPN molecule includes two or more expression control sequences mentioned above for different target genes, for example, the aforementioned first ssPN molecule can control the expressions of two or more kinds of different target genes.

One embodiment of the aforementioned first ssPN molecule is shown in WO 2012/017919, Fig. 1, and can be referred to.

In the aforementioned first ssPN molecule, the number of bases in each of the aforementioned regions (Xc) and (X) is not particularly limited. Examples of the lengths of the respective regions are given below. However, it is to be noted that the present invention is by no means limited thereto. In the present invention, "the number of bases" means the "length", for example, and it can also be referred to as the "base length". In the present invention, for example, the numerical range regarding the number of bases discloses all the positive integers falling within that range. For example, the description "1 to 4 bases" disclosed all of "1, 2, 3, and 4 bases" (the same applies hereinafter).

The aforementioned region (Xc) may be, for example, perfectly complementary to the entire region of the aforementioned region (X). In this case, it means that, for example, the aforementioned region (Xc) is composed of a base sequence complementary to the entire region extending from the 5'-terminus to the 3'-terminus of the aforementioned region (X). In other words, it means that the aforementioned region (Xc) has the same base length as the aforementioned region (X), and all the bases in the aforementioned region (Xc) are complementary to all the bases in the aforementioned region (X).

Furthermore, the aforementioned region (Xc) may be, for example, perfectly complementary to part of the aforementioned region (X). In this case, it means that, for example, the aforementioned region (Xc) is composed of a base sequence complementary to the part of the aforementioned region (X). In other words, it means that the aforementioned region (Xc) is composed of a base sequence whose base length is shorter than the base length of the aforementioned region (X) by one or more bases, and all the bases in the aforementioned region (Xc) are complementary to all the bases in the part of the aforementioned region (X). The aforementioned part of the region (X) is preferably a region having a base sequence composed of, for example, successive bases starting from the base at the end (the 1st base) on the aforementioned region (Xc) side in the aforementioned region (X).

In the aforementioned first ssPN molecule, the relationship between the number of bases (X) in the aforementioned region (X) and the number of bases (Xc) in the aforementioned region (Xc) satisfy, for example, the following condition (3) or (5). For example, in the former case, specifically, the following condition (11) is satisfied:

$$X > Xc \quad (3)$$

$$X - Xc = 1 \text{ to } 10, \text{ preferably } 1, 2, \text{ or } 3, \text{ more preferably } 1 \text{ or } 2 \quad (11)$$

$$X = Xc \quad (5)$$

When the aforementioned region (X) and/or the aforementioned region (Xc) include(s) the aforementioned expression control sequence, the aforementioned region may be, for example, a region composed of the aforementioned expression control sequence only or a region including the aforementioned expression control sequence. The number of bases in the aforementioned expression control sequence is, for example, 19 to 30, preferably 19, 20, or 21. In the region(s) including the aforementioned expression control sequence, for example, the aforementioned expression control sequence further may have an additional sequence on its 5'-side and/or 3'-side. The number of bases in the aforementioned additional sequence is, for example, 1 to 31, preferably 1 to 21, and more preferably 1 to 11.

The number of bases in the aforementioned region (X) is not particularly limited. When the aforementioned region (X) includes the aforementioned expression control sequence, the lower limit of the number of bases in the aforementioned region (X) is, for example, 19, and the upper limit of the same is, for example, 50, preferably 30, and more preferably 25. Specifically, the number of bases in the aforementioned region (X) is, for example, 19 to 50, preferably 19 to 30, and more preferably 19 to 25.

The number of bases in the aforementioned region (Xc) is not particularly limited. The lower limit of the number of bases in the aforementioned region (Xc) is, for example, 19, preferably 20, and more preferably 21, and the upper limit of the same is, for example, 50, more preferably 40, and still more preferably 30.

In the aforementioned ssPN molecule, the length of the aforementioned linker region (Lx) is not particularly

limited. The length of the aforementioned linker region (Lx) is preferably such that, for example, the aforementioned regions (X) and (Xc) can form a double strand. When the aforementioned linker region (Lx) includes the aforementioned nucleotide residue(s) in addition to the aforementioned non-nucleotide residue(s), the lower limit of the number of bases in the aforementioned linker region (Lx) is, for example, 1, preferably 2, and more preferably 3, and the upper limit of the same is, for example, 100, preferably 80, and more preferably 50.

The full length of the aforementioned first ssPN molecule is not particularly limited. In the aforementioned first ssPN molecule, the lower limit of the total number of bases (the number of bases in the full length ssPN molecule), is, for example, 38, preferably 42, more preferably 50, still more preferably 51, and particularly preferably 52, and the upper limit of the same is, for example, 300, preferably 200, more preferably 150, still more preferably 100, and particularly preferably 80. In the aforementioned first ssPN molecule, the lower limit of the total number of bases excluding that in the aforementioned linker region (Lx) is, for example, 38, preferably 42, more preferably 50, still more preferably 51, and particularly preferably 52, and the upper limit of the same is, for example, 300, preferably 200, more preferably 150, still more preferably 100, and particularly preferably 80.

(1-2) Second ssPN molecule

The aforementioned second ssPN molecule is a molecule that further includes a region (Y) and a region (Yc) that is complementary to the aforementioned region (Y), in addition to, for example, the aforementioned region (X), the aforementioned linker region (Lx), and the aforementioned region (Xc). In the aforementioned second ssPN molecule, an inner region (Z) is composed of the aforementioned region (X) and the aforementioned region (Y) that are linked to each other. The description regarding the aforementioned first ssPN molecule also applies to the aforementioned second ssPN molecule, unless otherwise stated.

The aforementioned second ssPN molecule may include, for example, the aforementioned region (Xc), the aforementioned linker region (Lx), the aforementioned region (X), the aforementioned region (Y), and the aforementioned region (Yc) in this order from the 5'-side to the 3'-side. In this case, the aforementioned region (Xc) also is referred to as a "5'-side region (Xc)"; the aforementioned region (X) in the aforementioned inner region (Z) also is referred to as an "inner 5'-side region (X)"; the aforementioned region (Y) in the aforementioned inner region (Z) also is referred to as an "inner 3' region (Y)"; and the aforementioned region (Yc) also is referred to as a "3'-side region (Yc)". Alternatively, the aforementioned second ssPN molecule may include, for example, the aforementioned region (Xc), the aforementioned linker region (Lx), the aforementioned region (X), the aforementioned region (Y), and the aforementioned region (Yc) in this order from the 3'-side to the 5'-side. In this case, the aforementioned region (Xc) also is referred to as a "3'-side region (Xc)"; the aforementioned region (X) in the aforementioned inner region (Z) also is referred to as an "inner 3'-side region (X)"; the aforementioned region (Y) in the aforementioned inner region (Z) also is referred to as an "inner 5' region (Y)"; and the aforementioned region (Yc) also is referred to as a "5'-side region (Yc)".

As described above, the aforementioned inner region (Z) is composed of, for example, the aforementioned regions (X) and (Y) that are linked to each other. For example, the aforementioned regions (X) and (Y) are linked directly to each other with no intervening sequence therebetween. The aforementioned inner region (Z) is defined as being "composed of the aforementioned regions (X) and (Y) that are linked to each other" merely to indicate the sequence context between the aforementioned regions (Xc) and (Yc). This definition does not intend to limit that, in the use of the aforementioned ssPN molecule, the aforementioned regions (X) and (Y) in the aforementioned inner region (Z) are discrete independent regions. That is, for example, when the aforementioned expression control sequence is included in the aforementioned inner region (Z), the aforementioned expression control sequence may be arranged to extend across the aforementioned regions (X) and (Y) in the aforementioned inner region (Z).

In the aforementioned second ssPN molecule, the aforementioned region (Xc) is complementary to the aforementioned region (X). It is only necessary that the aforementioned region (Xc) has a sequence complementary to the entire region or part of the aforementioned region (X). Preferably, the aforementioned region (Xc) includes or is composed of a sequence complementary to the entire region or part of the aforementioned region (X). The aforementioned region (Xc) may be, for example, perfectly complementary to the entire region or part of the aforementioned region (X), or one or a few bases in the aforementioned region (Xc) may be noncomplementary to the same. Preferably, the aforementioned region (Xc) is perfectly complementary to the same. The aforementioned expression "one or a few bases" means, for example, 1 to 3 bases, preferably 1 base or 2 bases.

In the aforementioned second ssPN molecule, the aforementioned region (Yc) is complementary to the aforementioned region (Y). It is only necessary that the aforementioned region (Yc) has a sequence complementary to the entire region or part of the aforementioned region (Y). Preferably, the aforementioned region (Yc) includes or is composed of a sequence complementary to the entire region or part of the aforementioned region (Y). The aforementioned region (Yc) may be, for example, perfectly complementary to the entire region or part of the aforementioned region (Y), or one or a few bases in the aforementioned region (Yc) may be noncomplementary to the same. Preferably, the aforementioned region (Yc) is perfectly complementary to the same. The aforementioned expression "one or a few bases" means, for

example, 1 to 3 bases, preferably 1 base or 2 bases.

In the aforementioned second ssPN molecule, at least one of the aforementioned inner region (Z), which is composed of the aforementioned regions (X) and (Y), and the aforementioned region (Xc) includes, for example, the aforementioned expression control sequence. Furthermore, the aforementioned region (Yc) also may include the aforementioned expression control sequence. When the aforementioned inner region (Z) includes the aforementioned expression control sequence, for example, either of the aforementioned regions (X) and (Y) may include the aforementioned expression control sequence, or the aforementioned expression control sequence may be included to extend across the aforementioned regions (X) and (Y). The aforementioned second ssPN molecule may include, for example, one expression control sequence mentioned above, or two or more expression control sequences mentioned above.

When the aforementioned second ssPN molecule includes two or more expression control sequences mentioned above, the positions of the respective expression control sequences are not particularly limited. They may be in either one of the aforementioned inner region (Z) and the aforementioned region (Xc), or may be in one of the aforementioned inner region (Z) and the aforementioned region (Xc), and any region other than these regions.

In the aforementioned second ssPN molecule, for example, the aforementioned regions (Yc) and (Y) may be linked to each other either directly or indirectly. In the former case, for example, the aforementioned regions (Yc) and (Y) may be linked directly by phosphodiester linkage or the like. In the latter case, for example, the aforementioned second ssPN molecule may be configured so that it has a linker region (Ly) between the aforementioned regions (Yc) and (Y) and the aforementioned regions (Yc) and (Y) are linked via the aforementioned linker region (Ly).

When the aforementioned second ssPN molecule has the aforementioned linker region (Ly), for example, the aforementioned linker region (Ly) may be a linker composed of the aforementioned nucleotide residue(s), or a linker having a non-nucleotide structure containing at least one of a pyrrolidine skeleton and a piperidine skeleton such as described above. In the latter case, the aforementioned linker region (Ly) can be represented by the aforementioned formula (I), for example, and all the descriptions regarding the aforementioned formula (I) stated above in connection with the aforementioned linker region (Lx) also apply to the aforementioned linker region (Ly).

The aforementioned regions (Yc) and (Y) are, for example, each linked to the aforementioned linker region (Ly) via $-OR^1$-or $-OR^2$-. In the aforementioned linker region (Ly), $R^1$ and $R^2$ may or may not be present, as in the above-described linker region (Lx).

The combination of the aforementioned regions (Xc) and (X) with aforementioned $-OR^1-$ and $-OR^2-$, and the combination of the aforementioned regions (Yc) and (Y) with aforementioned $-OR^1-$ and $-OR^2-$ are not particularly limited, and may be, for example, any of the following conditions:

Condition (1):

the aforementioned regions (Xc) and (X) are linked to the structure of the aforementioned formula (I) via $-OR^2-$ and $-OR^1-$, respectively; and
the aforementioned regions (Yc) and (Y) are linked to the structure of the aforementioned formula (I) via $-OR^1-$ and $-OR^2-$, respectively.

Condition (2):

the aforementioned regions (Xc) and (X) are linked to the structure of the aforementioned formula (I) via $-OR^2-$ and $-OR^1-$, respectively; and
the aforementioned regions (Yc) and (Y) are linked to the structure of the aforementioned formula (I) via $-OR^2-$ and $-OR^1-$, respectively.

Condition (3):

the aforementioned regions (Xc) and (X) are linked to the structure of the aforementioned formula (I) via $-OR^1-$ and $-OR^2-$, respectively; and
the aforementioned regions (Yc) and (Y) are linked to the structure of the aforementioned formula (I) via $-OR^1-$ and $-OR^2-$, respectively.

Condition (4):

the aforementioned regions (Xc) and (X) are linked to the structure of the aforementioned formula (I) via $-OR^1-$ and $-OR^2-$, respectively; and
the aforementioned regions (Yc) and (Y) are linked to the structure of the aforementioned formula (I) via $-OR^2-$ and $-OR^1-$, respectively.

As regards the aforementioned second ssPN molecule, one embodiment of ssPN molecule having the aforementioned linker region (Ly) is shown in WO 2012/017919, Fig. 2, and can be referred to.

In the aforementioned second ssPN molecule, the number of bases in each of the aforementioned regions (Xc), (X), (Y), and (Yc) is not particularly limited. Examples of the lengths of the respective regions are given below. It is to be noted, however, that the present invention is by no means limited thereto.

As described above, for example, the aforementioned region (Xc) may be complementary to the entire region of the aforementioned region (X). In this case, it is preferable that, for example, the aforementioned region (Xc) has the same base length as the aforementioned region (X), and is composed of a base sequence complementary to the entire region of the aforementioned region (X). It is more preferable that the aforementioned region (Xc) has the same base length as the aforementioned region (X) and all the bases in the aforementioned region (Xc) are complementary to all the bases in the aforementioned region (X), i.e., for example, the region (Xc) is perfectly complementary to the region (X). It is to be noted, however, that the configuration of the region (Xc) is not limited thereto, and one or a few bases in the region (Xc) may be noncomplementary to the corresponding bases in the region (X), for example, as described above.

Furthermore, as described above, the aforementioned region (Xc) may be complementary to, for example, a part of the aforementioned region (X). In this case, it is preferable that, for example, the aforementioned region (Xc) has the same base length as the part of the aforementioned region (X), i.e., the aforementioned region (Xc) is composed of a base sequence whose base length is shorter than the base length of the aforementioned region (X) by one or more bases. It is more preferable that the aforementioned region (Xc) has the same base length as the part of the aforementioned region (X) and all the bases in the aforementioned region (Xc) are complementary to all the bases in the part of the aforementioned region (X), i.e., for example, the region (Xc) is perfectly complementary to the part of the region (X). The part of the aforementioned region (X) is preferably a region having a base sequence composed of, for example, successive bases starting from the base at the end (the 1st base) on the aforementioned region (Xc) side in the aforementioned region (X) .

As described above, the aforementioned region (Yc) may be complementary to, for example, the entire region of the aforementioned region (Y). In this case, it is preferable that, for example, the aforementioned region (Yc) has the same base length as the aforementioned region (Y), and is composed of a base sequence complementary to the entire region of the aforementioned region (Y). It is more preferable that the aforementioned region (Yc) has the same base length as the aforementioned region (Y) and all the bases in the aforementioned region (Yc) are complementary to all the bases in the aforementioned region (Y), i.e., for example, the region (Yc) is perfectly complementary to the region (Y). It is to be noted, however, that the configuration of the region (Yc) is not limited thereto, and one or a few bases in the region (Yc) may be noncomplementary to the corresponding bases in the region (Y), for example, as described above.

Furthermore, as described above, the aforementioned region (Yc) may be complementary to, for example, a part of the aforementioned region (Y). In this case, it is preferable that, for example, the aforementioned region (Yc) has the same base length as the part of the aforementioned region (Y), i.e., the aforementioned region (Yc) is composed of a base sequence whose base length is shorter than the base length of the aforementioned region (Y) by one or more bases. It is more preferable that the aforementioned region (Yc) has the same base length as the part of the aforementioned region (Y) and all the bases in the aforementioned region (Yc) are complementary to all the bases in the part of the aforementioned region (Y), i.e., for example, the region (Yc) is perfectly complementary to the part of the region (Y). The part of the aforementioned region (Y) is preferably a region having a base sequence composed of, for example, successive bases starting from the base at the end (the 1st base) on the aforementioned region (Yc) side in the aforementioned region (Y).

In aforementioned the second ssPN molecule, the relationship of the number of bases (Z) in the aforementioned inner region (Z) with the number of bases (X) in the aforementioned region (X) and the number of bases (Y) in the aforementioned region (Y) and the relationship of the number of bases (Z) in the aforementioned inner region (Z) with the number of bases (X) in the aforementioned region (X) and the number of bases (Xc) in the aforementioned region (Xc) satisfy, for example, the conditions of the following expressions (1) and (2).

$$Z = X + Y \quad (1)$$

$$Z \geq Xc + Yc \quad (2)$$

In the aforementioned second ssPN molecule, the relationship between the number of bases (X) in the aforementioned region (X) and the number of bases (Y) in the aforementioned region (Y) is not particularly limited, and satisfy, for example, any of the conditions of the following expressions:

$$X = Y \quad (19)$$

$$X < Y \quad (20)$$

$$X > Y \quad (21).$$

In the second ssPN molecule, the relationship between the number of bases (X) in the aforementioned region (X) and the number of bases (Xc) in the aforementioned region (Xc), and the relationship between the number of bases (Y) in the aforementioned region (Y) and the number of bases (Yc) in the aforementioned region (Yc) satisfy, for example, any of the following conditions (a) to (d):

(a) Conditions of the following expressions (3) and (4) are satisfied.

$$X > Xc \quad (3)$$

$$Y = Yc \quad (4)$$

(b) Conditions of the following expressions (5) and (6) are satisfied.

$$X = Xc \quad (5)$$

$$Y > Yc \quad (6)$$

(c) Conditions of the following expressions (7) and (8) are satisfied.

$$X > Xc \quad (7)$$

$$Y > Yc \quad (8)$$

(d) Conditions of the following expressions (9) and (10) are satisfied.

$$X = Xc \quad (9)$$

$$Y = Yc \quad (10)$$

In the above-described conditions (a) to (d), for example, the difference between the number of bases (X) in the aforementioned region (X) and the number of bases (Xc) in the aforementioned region (Xc), and the difference between the number of bases (Y) in the aforementioned region (Y) and the number of bases (Yc) in the aforementioned region (Yc) preferably satisfy the following conditions.

(a) Conditions of the following expressions (11) and (12) are satisfied.

$$X - Xc = 1 \text{ to } 10\text{, preferably } 1, 2, 3, \text{ or } 4, \text{ more preferably } 1, 2, \text{ or } 3 \quad (11)$$

$$Y - Yc = 0 \quad (12)$$

(b) Conditions of the following expressions (13) and (14) are satisfied.

$$X - Xc = 0 \quad (13)$$

$$Y - Yc = 1 \text{ to } 10\text{, preferably } 1, 2, 3, \text{ or } 4, \text{ more preferably } 1, 2, \text{ or } 3 \quad (14)$$

(c) Conditions of the following expressions (15) and (16) are satisfied.

$$X - Xc = 1 \text{ to } 10\text{, preferably, } 1, 2, \text{ or } 3, \text{ more preferably } 1 \text{ or } 2 \quad (15)$$

$$Y - Yc = 1 \text{ to } 10\text{, preferably, } 1, 2, \text{ or } 3, \text{ more preferably } 1 \text{ or } 2 \quad (16)$$

(d) Conditions of the following expressions (17) and (18) are satisfied.

$$X - Xc = 0 \quad (17)$$

$$Y - Yc = 0 \quad (18)$$

As regards the second ssPN molecules of the aforementioned (a) - (d), one embodiment of each structure is shown in WO 2012/017919, Fig. 3, and can be referred to.

The ssPN molecules of the above-mentioned (a) to (c) are configurations having a base not aligned with both the aforementioned regions (Xc) and (Yc) in the aforementioned inner region (Z) since, for example, the aforementioned regions (Xc) and (X), and regions (Yc) and (Y) each form a double strand. They may also be said configurations having a base not forming a double strand. In the aforementioned inner region (Z), the aforementioned base that is not aligned (also referred to as a base that does not form a double strand) is hereinafter to be referred to as an "unpaired base". In FIG. 3 of WO 2012/017919, the region of the aforementioned unpaired base is shown by "F". The number of the bases in the aforementioned region (F) is not particularly limited. The number of the bases (F) in the aforementioned region (F) is, for example, the number of the bases of "X - Xc" for the ssPN molecule of the aforementioned (a); the number of the bases of "Y - Yc" for the ssPN molecule of the above-mentioned (b); and the total of the number of the bases of "X - Xc" and the number of the bases of "Y - Yc" for the ssPN molecule of the aforementioned (c).

On the other hand, the ssPN molecule satisfying the aforementioned condition (d) is configured so that, for example, the entire region of the aforementioned inner region (Z) is aligned with the aforementioned regions (Xc) and (Yc), in other words, the entire region of the aforementioned inner region (Z) forms a double strand. In the ssPN molecule satisfying the aforementioned condition (d), the 5'-terminus of the aforementioned region (Xc) and the 3'-terminus of the aforementioned region (Yc) are not linked to each other.

The total number of the bases in the aforementioned region (Xc), the bases in the aforementioned region (Yc), and the aforementioned unpaired bases (F) in the aforementioned inner region (Z) is equal to the number of the bases in the aforementioned inner region (Z). Thus, the length of the aforementioned region (Xc) and the length of the aforementioned region (Yc) can be determined as appropriate depending on, for example, the length of the aforementioned inner region (Z), the number of the aforementioned unpaired bases, and the positions of the unpaired bases.

The number of the bases in the aforementioned inner region (Z) is, for example, 19 or more. The lower limit of the number of the bases is, for example, 19, preferably 20, and more preferably 21. The upper limit of the number of the aforementioned bases is, for example, 50, preferably 40, and more preferably 30. A specific example of the number of the bases in the aforementioned inner region (Z) is 19, 20, 21, 22, 23, 24, 25, 26, 27, 28, 29, or 30. When the aforementioned inner region (Z) includes the aforementioned expression control sequence, for example, such conditions

are preferable.

When the aforementioned inner region (Z) includes the aforementioned expression control sequence, the aforementioned inner region (Z) may be, for example, a region composed of the aforementioned expression control sequence only or a region including the aforementioned expression control sequence. The number of bases of the aforementioned expression control sequence is, for example, 19 to 30, preferably 19, 20, or 21. When the aforementioned inner region (Z) includes the aforementioned expression control sequence, the aforementioned expression control sequence further may have an additional sequence on its 5'-side and/or 3'-side. The number of bases in the aforementioned additional sequence is, for example, 1 to 31, preferably 1 to 21, more preferably 1 to 11, and still more preferably 1 to 7.

The number of bases in the aforementioned region (Xc) is, for example, 1 to 29, preferably 1 to 11, more preferably 1 to 7, still more preferably 1 to 4, and particularly preferably 1, 2, or 3. When the aforementioned inner region (Z) or the aforementioned region (Yc) includes the aforementioned expression control sequence, for example, the number of bases as described above is preferable. A specific example is as follows: when the number of bases in the aforementioned inner region (Z) is 19 to 30 (e.g., 19), the number of bases in the aforementioned region (Xc) is, for example, 1 to 11, preferably 1 to 7, more preferably 1 to 4, and still more preferably 1, 2, or 3.

When the aforementioned region (Xc) includes the aforementioned expression control sequence, the aforementioned region (Xc) may be, for example, a region composed of the aforementioned expression control sequence only or a region including the aforementioned expression control sequence. For example, the length of the aforementioned expression control sequence is as described above. When the aforementioned region (Xc) includes the aforementioned expression control sequence, the aforementioned expression control sequence further may have an additional sequence on its 5'-side and/or 3'-side. The number of bases in the aforementioned additional sequence is, for example, 1 to 11, preferably 1 to 7.

The number of bases in the aforementioned region (Yc) is, for example, 1 to 29, preferably 1 to 11, more preferably 1 to 7, still more preferably 1 to 4, and particularly preferably 1, 2, or 3. When the aforementioned inner region (Z) or the aforementioned region (Xc) includes the aforementioned expression control sequence, for example, the number of bases as described above is preferable. A specific example is as follows: when the number of bases in the aforementioned inner region (Z) is 19 to 30 (e.g., 19), the number of bases in the aforementioned region (Yc) is, for example, 1 to 11, preferably 1 to 7, more preferably 1, 2, 3, or 4, and still more preferably 1, 2, or 3.

When the aforementioned region (Yc) includes the aforementioned expression control sequence, the aforementioned region (Yc) may be, for example, a region composed of the aforementioned expression control sequence only or a region including the aforementioned expression control sequence. The length of the aforementioned expression control sequence is, for example, as described above. When the aforementioned region (Yc) includes the aforementioned expression control sequence, the aforementioned expression control sequence further may have an additional sequence on its 5'-side and/or 3'-side. The number of bases in the aforementioned additional sequence is, for example, 1 to 11, preferably 1 to 7.

As described above, the relationship among the number of bases in the aforementioned inner region (Z), the number of bases in the aforementioned region (Xc), and the number of bases in the aforementioned region (Yc) can be expressed by, for example, the aforementioned expression (2): "$Z \geq Xc + Yc$". Specifically, the number of bases represented by "$Xc + Yc$" is, for example, equal to the number of bases in the aforementioned inner region (Z), or lower than the number of bases in the aforementioned inner region (Z). In the latter case, "$Z - (Xc + Yc)$" is, for example, 1 to 10, preferably 1 to 4, and more preferably 1, 2, or 3. The aforementioned "$Z - (Xc + Yc)$" corresponds, for example, to the number of bases (F) in the unpaired base region (F) in the aforementioned inner region (Z).

In the aforementioned second ssPN molecule, the lengths of the aforementioned linker regions (Lx) and (Ly) are not particularly limited. The aforementioned linker region (Lx) is as described above. When the constitutional unit of the aforementioned linker region (Ly) include a base(s), the lower limit of the number of bases in the aforementioned linker region (Ly) is, for example, 1, preferably 2, and more preferably 3, and the upper limit of the same is, for example, 100, preferably 80, and more preferably 50. The number of bases in each of the aforementioned linker regions is specifically 1 to 50, 1 to 30, 1 to 20, 1 to 10, 1 to 7, or 1 to 4, for example, but it is not limited to these examples.

The aforementioned linker region (Ly) may be, for example, the same as or different from the aforementioned linker region (Lx) .

The full length of the aforementioned second ssPN molecule is not particularly limited. In the aforementioned second ssPN molecule, the lower limit of the total number of bases (the number of bases in the full length ssPN molecule), is, for example, 38, preferably 42, more preferably 50, still more preferably 51, and particularly preferably 52, and the upper limit of the same is, for example, 300, preferably 200, more preferably 150, still more preferably 100, and particularly preferably 80. In the aforementioned second ssPN molecule, the lower limit of the total number of bases excluding those in the aforementioned linker regions (Lx) and (Ly) is, for example, 38, preferably 42, more preferably 50, still more preferably 51, and particularly preferably 52, and the upper limit of the same is, for example, 300, preferably 200, more preferably 150, still more preferably 100, and particularly preferably 80.

In the aforementioned ssPN molecule, it is only necessary that the aforementioned linker region (Lx) has the

aforementioned non-nucleotide structure, as described above, and other constitutional units are not particularly limited. Examples of the aforementioned constitutional units include nucleotide residues. Examples of the aforementioned nucleotide residues include a ribonucleotide residue and a deoxyribonucleotide residue. The aforementioned nucleotide residue may be, for example, the one that is not modified (unmodified nucleotide residue) or the one that has been modified (modified nucleotide residue). By configuring the aforementioned ssPN molecule to include the aforementioned modified nucleotide residue, for example, the resistance of the ssPN molecule to nuclease can be improved, thereby allowing the stability of the ssPN molecule to be improved. Furthermore, the aforementioned ssPN molecule further may include, for example, a non-nucleotide residue in addition to the aforementioned nucleotide residue.

The aforementioned nucleotide residue is preferable as the constitutional unit of each of the aforementioned region (Xc), the aforementioned region (X), the aforementioned region (Y) and the aforementioned region (Yc). Each of the aforementioned regions is composed of, for example, any of the following residues (1) to (3):

(1) an unmodified nucleotide residue(s)
(2) a modified nucleotide residue(s)
(3) an unmodified nucleotide residue(s) and a modified nucleotide residue(s).

The aforementioned linker region (Lx) may be composed of, for example, the aforementioned non-nucleotide residue(s) only, or may be composed of the aforementioned non-nucleotide(s) and the aforementioned nucleotide residue(s). The aforementioned linker region (Lx) is composed of, for example, any of the following residues (4) to (7):

(4) a non-nucleotide residue(s)
(5) a non-nucleotide residue(s) and an unmodified nucleotide residue(s)
(6) a non-nucleotide residue(s) and a modified nucleotide residue(s)
(7) a non-nucleotide residue(s), an unmodified nucleotide residue(s), and a modified nucleotide residue(s).

The constitutional units of the aforementioned linker region (Ly) are not particularly limited, and examples thereof include the aforementioned nucleotide residues and the aforementioned non-nucleotide residues, as described above. Each of the aforementioned linker regions (Ly) may be composed of, for example, the aforementioned nucleotide residue(s) only, the aforementioned non-nucleotide residue(s) only, or both the aforementioned nucleotide residue(s) and the aforementioned non-nucleotide residue(s). Each of the aforementioned linker regions (Ly) is composed of, for example, any of the following residues (1) to (7):

(1) an unmodified nucleotide residue(s)
(2) a modified nucleotide residue(s)
(3) an unmodified nucleotide residue(s) and a modified nucleotide residue(s)
(4) a non-nucleotide residue(s)
(5) a non-nucleotide residue(s) and an unmodified nucleotide residue(s)
(6) a non-nucleotide residue(s) and a modified nucleotide residue(s)
(7) a non-nucleotide residue(s), an unmodified nucleotide residue(s), and a modified nucleotide residue(s).

Examples of the aforementioned ssPN molecule, excluding the aforementioned linker region (Lx), include molecules composed of the aforementioned nucleotide residues only; and molecules including the aforementioned non-nucleotide residue(s) in addition to the aforementioned nucleotide residues. In the aforementioned ssPN molecule, for example, the aforementioned nucleotide residues may be the aforementioned unmodified nucleotide residues only; the aforementioned modified nucleotide residues only; or both the aforementioned unmodified nucleotide residue(s) and the aforementioned modified nucleotide residue(s), as described above. When the aforementioned ssPN molecule includes both the aforementioned unmodified nucleotide residue(s) and the aforementioned modified nucleotide residue(s), the number of the aforementioned modified nucleotide residue(s) is not particularly limited, and is, for example, "one to several", specifically, for example, 1 to 5, preferably 1 to 4, more preferably 1 to 3, and most preferably 1 or 2. When the aforementioned ssPN molecule includes the aforementioned non-nucleotide residue(s), the number of the aforementioned non-nucleotide residue(s) is not particularly limited, and is, for example, "one to several", specifically, for example, 1 or 2.

In the aforementioned ssPN molecule, for example, the aforementioned nucleotide residue is preferably a ribonucleotide residue. In this case, for example, the aforementioned ssPN molecule also is referred to as an "ssRNA molecule" or "P-ssRNA molecule". Examples of the aforementioned ssRNA molecule, excluding the aforementioned linker region (Lx), include molecules composed of the aforementioned ribonucleotide residues only; and a molecule including the aforementioned non-nucleotide residue(s) in addition to the aforementioned ribonucleotide residues. As described above, as the aforementioned ribonucleotide residues, for example, the aforementioned ssRNA molecule

may include: the aforementioned unmodified ribonucleotide residues only; the aforementioned modified ribonucleotide residues only; or both the aforementioned unmodified ribonucleotide residue(s) and the aforementioned modified ribonucleotide residue(s).

When the aforementioned ssRNA molecule includes, for example, the aforementioned modified ribonucleotide residue(s) in addition to the aforementioned unmodified ribonucleotide residues, the number of the aforementioned modified ribonucleotide residue(s) is not particularly limited, and is, for example, "one to several", specifically, for example, 1 to 5, preferably 1 to 4, more preferably 1 to 3, and most preferably 1 or 2. The aforementioned modified ribonucleotide residue as contrasted to the aforementioned unmodified ribonucleotide residue may be, for example, the aforementioned deoxyribonucleotide residue obtained by substituting a ribose residue with a deoxyribose residue. When the aforementioned ssRNA molecule includes, for example, the aforementioned deoxyribonucleotide residue(s) in addition to the aforementioned unmodified ribonucleotide residue(s), the number of the aforementioned deoxyribonucleotide residue(s) is not particularly limited, and is, for example, "one to several", specifically, for example, 1 to 5, preferably 1 to 4, more preferably 1 to 3, and most preferably 1 or 2.

The aforementioned ssPN molecule may include, for example, a labeling substance, and may be labeled with the aforementioned labeling substance. The aforementioned labeling substance is not particularly limited, and may be, for example, a fluorescent substance, a dye, an isotope, or the like. Examples of the aforementioned labeling substance include: fluorophores such as pyrene, TAMRA, fluorescein, a Cy3 dye, and a Cy5 dye. Examples of the aforementioned dye include Alexa dyes such as Alexa 488. Examples of the aforementioned isotope include stable isotopes and radioisotopes. Among them, stable isotopes are preferable. For example, the aforementioned stable isotopes have a low risk of radiation exposure and they require no dedicated facilities. Thus, stable isotopes are excellent in handleability and can contribute to cost reduction. Moreover, for example, the aforementioned stable isotope does not change the physical properties of a compound labeled therewith and thus has an excellent property as a tracer. The aforementioned stable isotope is not particularly limited, and examples thereof include $^{2}H$, $^{13}C$, $^{15}N$, $^{17}O$, $^{18}O$, $^{33}S$, $^{34}S$, and $^{36}S$.

In the present invention, the term "alkyl" encompasses, for example, straight-chain and branched alkyl groups. The number of carbon atoms in the aforementioned alkyl is not particularly limited, and is, for example, 1 to 30, preferably 1 to 6 or 1 to 4. Examples of the aforementioned alkyl group include: methyl, ethyl, n-propyl, isopropyl, n-butyl, isobutyl, sec-butyl, tert-butyl, n-pentyl, isopentyl, neopentyl, n-hexyl, isohexyl, n-heptyl, n-octyl, n-nonyl, and n-decyl. Among them, for example, methyl, ethyl, n-propyl, isopropyl, n-butyl, isobutyl, sec-butyl, tert-butyl, n-pentyl, isopentyl, neopentyl, n-hexyl, isohexyl, and the like are preferable.

In the present invention, the term "alkenyl" encompasses, for example, straight-chain and branched alkenyls. Examples of the aforementioned alkenyl include the aforementioned alkyls having one or more double bonds. The number of carbon atoms in the aforementioned alkenyl is not particularly limited, and is, for example, the same as that in the aforementioned alkyl, preferably 2 to 8. Examples of the aforementioned alkenyl include vinyl, 1-propenyl, 2-propenyl, 1-butenyl, 2-butenyl, 3-butenyl, 1,3-butadienyl, and 3-methyl-2-butenyl.

In the present invention, the term "alkynyl" encompasses, for example, straight-chain and branched alkynyls. Examples of the aforementioned alkynyl include the aforementioned alkyls having one or more triple bonds. The number of carbon atoms in the aforementioned alkynyl is not particularly limited, and is, for example, the same as that in the aforementioned alkyl, preferably 2 to 8. Examples of the aforementioned alkynyl include ethynyl, propynyl, and butynyl. The aforementioned alkynyl may further include, for example, one or more double bonds.

In the present invention, the term "aryl" encompasses, for example, monocyclic aromatic hydrocarbon groups and polycyclic aromatic hydrocarbon groups. Examples of the aforementioned monocyclic aromatic hydrocarbon group include phenyl. Examples of the aforementioned polycyclic aromatic hydrocarbon group include 1-naphthyl, 2-naphthyl, 1-anthryl, 2-anthryl, 9-anthryl, 1-phenanthryl, 2-phenanthryl, 3-phenanthryl, 4-phenanthryl, and 9-phenanthryl. Among them, for example, phenyl, naphthyls such as 1-naphthyl and 2-naphthyl, and the like are preferable.

In the present invention, the term "heteroaryl" encompasses, for example, monocyclic aromatic heterocyclic groups and condensed aromatic heterocyclic groups. Examples of the aforementioned heteroaryl include furyls (e.g., 2-furyl, 3-furyl), thienyls (e.g., 2-thienyl, 3-thienyl), pyrrolyls (e.g., 1-pyrrolyl, 2-pyrrolyl, 3-pyrrolyl), imidazolyls (e.g., 1-imidazolyl, 2-imidazolyl, 4-imidazolyl), pyrazolyls (e.g., 1-pyrazolyl, 3-pyrazolyl, 4-pyrazolyl), triazolyls (e.g., 1,2,4-triazol-1-yl, 1,2,4-triazol-3-yl, 1,2,4-triazol-4-yl), tetrazolyls (e.g., 1-tetrazolyl, 2-tetrazolyl, 5-tetrazolyl), oxazolyls (e.g., 2-oxazolyl, 4-oxazolyl, 5-oxazolyl), isoxazolyls (e.g., 3-isoxazolyl, 4-isoxazolyl, 5-isoxazolyl), thiazolyls (e.g., 2-thiazolyl, 4-thiazolyl, 5-thiazolyl), thiadiazolyls, isothiazolyls (e.g., 3-isothiazolyl, 4-isothiazolyl, 5-isothiazolyl), pyridyls (e.g., 2-pyridyl, 3-pyridyl, 4-pyridyl), pyridazinyls (e.g., 3-pyridazinyl, 4-pyridazinyl), pyrimidinyls (e.g., 2-pyrimidinyl, 4-pyrimidinyl, 5-pyrimidinyl), furazanyls (e.g., 3-furazanyl), pyrazinyls (e.g., 2-pyrazinyl), oxadiazolyls (e.g., 1,3,4-oxadiazol-2-yl), benzofuryls (e.g., 2-benzo[b]furyl, -benzo[b]furyl, 4-benzo[b]furyl, 5-benzo[b]furyl, 6-benzo[b]furyl, 7-benzo[b]furyl), benzothienyls (e.g., 2-benzo[b]thienyl, 3-benzo[b]thienyl, 4-benzo[b]thienyl, 5-benzo[b]thienyl, 6-benzo[b]thienyl, 7-benzo[b]thienyl), benzimidazolyls (e.g., 1-benzimidazolyl, 2-benzimidazolyl, 4-benzimidazolyl, 5-benzimidazolyl), dibenzofuryls, benzoxazolyls, benzothiazolyls, quinoxalinyls (e.g., 2-quinoxalinyl, 5-quinoxalinyl, 6-quinoxalinyl), cinnolinyls (e.g., 3-cinnolinyl, 4-cinnolinyl, 5-cinnolinyl, 6-cinnolinyl, 7-cinnolinyl, 8-cinnolinyl), quinazolinyls (e.g., 2-quinazolinyl, 4-quinazolinyl, 5-quinazolinyl, 6-quinazolinyl, 7-quinazolinyl, 8-quinazolinyl), quinolyls (e.g., 2-quinolyl, 3-quinolyl, 4-quinolyl, 5-quinolyl, 6-quinolyl, 7-quinolyl, 8-quinolyl), phthalazinyls (e.g., 1-phthalazinyl, 5-phthalazinyl, 6-phthalazinyl), isoquinolyls (e.g., 1-isoquinolyl, 3-isoquinolyl, 4-isoquinolyl, 5-isoquinolyl, 6-isoquinolyl, 7-isoquinolyl, 8-isoquinolyl), puryls, pteridinyls (e.g., 2-pteridinyl, 4-pteridinyl, 6-pteridinyl, 7-pteridinyl), carbazolyls, phenanthridinyls, acridinyls (e.g., 1-acridinyl, 2-acridinyl, 3-acridinyl, 4-acridinyl, 9-acridinyl), indolyls (e.g., 1-indolyl, 2-indolyl, 3-indolyl, 4-indolyl, 5-indolyl, 6-indolyl, 7-indolyl), isoindolyls, phenazinyls (e.g., 1-phenazinyl, 2-phenazinyl), and phenothiazinyls (e.g., 1-phenothiazinyl, 2-phenothiazinyl, 3-phenothiazinyl, 4-phenothiazinyl).

In the present invention, for example, the term "cycloalkyl" refers to cyclic saturated hydrocarbon groups and the number of carbon atoms in the cycloalkyl is, for example, 3 to 15. Examples of the aforementioned cycloalkyl include cyclopropyl, cyclobutyl, cyclopentyl, cyclohexyl, cycloheptyl, cyclooctyl, bridged cyclic hydrocarbon groups, and spiro hydrocarbon groups. Among them, cyclopropyl, cyclobutyl, cyclopentyl, cyclohexyl, bridged cyclic hydrocarbon groups, and the like are preferable.

In the present invention, examples of the "bridged cyclic hydrocarbon groups" include bicyclo[2.1.0]pentyl, bicyclo[2.2.1]heptyl, bicyclo[2.2.2]octyl, and bicyclo[3.2.1]octyl, tricyclo[2.2.1.0]heptyl, bicyclo[3.3.1]nonane, 1-adamantyl, and 2-adamantyl.

In the present invention, examples of the "spiro hydrocarbon groups" include spiro[3.4]octyl.

In the present invention, the term "cycloalkenyl" encompasses, for example, unsaturated cyclic aliphatic hydrocarbon groups and the number of carbon atoms in the cycloalkenyl is, for example, 3 to 7. Examples of the aforementioned group include cyclopropenyl, cyclobutenyl, cyclopentenyl, cyclohexenyl, and cycloheptenyl. Among them, cyclopropenyl, cyclobutenyl, cyclopentenyl, cyclohexenyl, and the like are preferable. The aforementioned term "cycloalkenyl" also encompasses, for example, bridged cyclic hydrocarbon groups and spiro hydrocarbon groups having an unsaturated bond in their rings.

In the present invention, examples of the "arylalkyl" include benzyl, 2-phenethyl, and naphthalenylmethyl. Examples of the "cycloalkylalkyl" and "cyclylalkyl" include cyclohexylmethyl and adamantylmethyl. Examples of the "hydroxyalkyl" include hydroxymethyl and 2-hydroxyethyl.

In the present invention, the "alkoxy" encompasses, for example, groups composed of any of the aforementioned alkyls and oxygen (alkyl-O-groups) and examples thereof include methoxy, ethoxy, n-propoxy, isopropoxy, and n-butoxy. Examples of the "alkoxyalkyl" include methoxymethyl. Examples of the "aminoalkyl" include 2-aminoethyl.

In the present invention, examples of the "heterocyclyl" include 1-pyrrolinyl, 2-pyrrolinyl, 3-pyrrolinyl, 1-pyrrolidinyl, 2-pyrrolidinyl, 3-pyrrolidinyl, pyrrolidinone, 1-imidazoliny, 2-imidazoliny, 4-imidazoliny, 1-imidazolidinyl, 2-imidazolidinyl, 4-imidazolidinyl, imidazolidinone, 1-pyrazolinyl, 3-pyrazolinyl, 4-pyrazolinyl, 1-pyrazolidinyl, 3-pyrazolidinyl, 4-pyrazolidinyl, piperidinone, piperidino, 2-piperidinyl, 3-piperidinyl, 4-piperidinyl, 1-piperazinyl, 2-piperazinyl, piperazinone, 2-morpholinyl, 3-morpholinyl, morpholino, tetrahydropyranyl, and tetrahydrofuranyl.

In the present invention, examples of the "heterocyclylalkyl" include piperidinylmethyl and piperazinylmethyl. Examples of the "heterocyclylalkenyl" include 2-piperidinylethenyl. Examples of the "heteroarylalkyl" include pyridylmethyl and quinolin-3-ylmethyl.

In the present invention, the term "silyl" encompasses groups represented by the formula $R_3Si$-, where R independently can be selected from the aforementioned alkyls, aryls, and cycloalkyls. Examples of the silyl include a trimethylsilyl group and a tert-butyldimethylsilyl group. Examples of the "silyloxy" include a trimethylsilyloxy group. Examples of the "silyloxyalkyl" include trimethylsilyloxymethyl.

In the present invention, examples of the "alkylene" include methylene, ethylene, and propylene.

In the present invention, the above-described various groups may be substituted. Examples of the aforementioned substituent include hydroxy, carboxy, halogen, alkyl halide (e.g., $CF_3$, $CH_2CF_3$, $CH_2CCl_3$), nitro, nitroso, cyano, alkyl (e.g., methyl, ethyl, isopropyl, tert-butyl), alkenyl (e.g., vinyl), alkynyl (e.g., ethynyl), cycloalkyl (e.g., cyclopropyl, adamantyl), cycloalkylalkyl (e.g., cyclohexylmethyl, adamantylmethyl), cycloalkenyl (e.g., cyclopropenyl), aryl (e.g., phenyl, naphthyl), arylalkyl (e.g., benzyl, phenethyl), heteroaryl (e.g., pyridyl, furyl), heteroarylalkyl (e.g., pyridylmethyl), heterocyclyl (e.g., piperidyl), heterocyclylalkyl (e.g., morpholylmethyl), alkoxy (e.g., methoxy, ethoxy, propoxy, butoxy), halogenated alkoxy (e.g., $OCF_3$), alkenyloxy (e.g., vinyloxy, allyloxy), aryloxy (e.g., phenyloxy), alkyloxycarbonyl (e.g., methoxycarbonyl, ethoxycarbonyl, tert-butoxycarbonyl), arylalkyloxy (e.g., benzyloxy), amino [alkylamino (e.g., methylamino, ethylamino, dimethylamino), acylamino (e.g., acetylamino, benzoylamino), arylalkylamino (e.g., benzylamino, tritylamino), hydroxyamino], alkylaminoalkyl (e.g., diethylaminomethyl), sulfamoyl, oxo, and the like.

(2) Nucleotide residue

The nucleotide residue constituting the aforementioned nucleic acid molecule contained in the composition of the present invention includes, for example, a sugar, a base, and a phosphate as its components. The aforementioned nucleotide residue may be, for example, a ribonucleotide residue or a deoxyribonucleotide residue, as described above. The aforementioned ribonucleotide residue has, for example, a ribose residue as the sugar; and adenine (A), guanine

(G), cytosine (C), or uracil (U) as the base. The aforementioned deoxyribose residue has, for example, a deoxyribose residue as the sugar; and adenine (A), guanine (G), cytosine (C), or thymine (T) as the base.

The aforementioned nucleotide residue may be, for example, an unmodified nucleotide residue or a modified nucleotide residue. The aforementioned components of the aforementioned unmodified nucleotide residue are the same or substantially the same as, for example, the components of a naturally-occurring nucleotide residue. Preferably, the components are the same or substantially the same as the components of a nucleotide residue occurring naturally in a human body.

The aforementioned modified nucleotide residue is, for example, a nucleotide residue obtained by modifying the aforementioned unmodified nucleotide residue. For example, the aforementioned modified nucleotide may be such that any of the components of the aforementioned unmodified nucleotide residue is modified. In the present invention, "modification" means, for example, substitution, addition, and/or deletion of any of the aforementioned components; and substitution, addition, and/or deletion of an atom(s) and/or a functional group(s) in the aforementioned component(s). It can also be referred to as "alteration". Examples of the aforementioned modified nucleotide residue include naturally-occurring nucleotide residues and artificially-modified nucleotide residues. Regarding the aforementioned naturally-derived modified nucleotide residues, for example, Limbach et al. (Limbach et al., 1994, Summary: the modified nucleosides of RNA, Nucleic Acids Res. 22: pp. 2183 to 2196) can be referred to. The aforementioned modified nucleotide residue may be, for example, a residue of an alternative of the aforementioned nucleotide.

Examples of the modification of the aforementioned nucleotide residue include modification of a ribose-phosphate backbone (hereinafter referred to as a "ribophosphate backbone").

In the aforementioned ribophosphate backbone, for example, a ribose residue may be modified. In the aforementioned ribose residue, for example, the 2'-position carbon can be modified. Specifically, a hydroxyl group bound to, for example, the 2'-position carbon can be substituted with hydrogen or fluoro. By substituting the hydroxyl group bound to the aforementioned 2'-position carbon with hydrogen, it is possible to substitute the ribose residue with deoxyribose. The aforementioned ribose residue can be substituted with its stereoisomer, for example, and may be substituted with, for example, an arabinose residue.

The aforementioned ribophosphate backbone may be substituted with, for example, a non-ribophosphate backbone having a non-ribose residue and/or a non-phosphate. The aforementioned non-ribophosphate backbone may be, for example, the aforementioned ribophosphate backbone modified to be uncharged. Examples of an alternative obtained by substituting the ribophosphate backbone with the aforementioned non-ribophosphate backbone in the aforementioned nucleotide include morpholino, cyclobutyl, and pyrrolidine. Other examples of the aforementioned alternative include artificial nucleic acid monomer residues. Specific examples thereof include PNA (Peptide Nucleic Acid), LNA (Locked Nucleic Acid), and ENA (2'-0,4'-C-Ethylenebridged Nucleic Acids). Among them, PNA is preferable.

In the aforementioned ribophosphate backbone, for example, a phosphate group can be modified. In the aforementioned ribophosphate backbone, a phosphate group in the closest proximity to the sugar residue is called an "$\alpha$-phosphate group". The aforementioned $\alpha$-phosphate group is charged negatively, and the electric charges are distributed evenly over two oxygen atoms that are not linked to the sugar residue. Among the four oxygen atoms in the aforementioned $\alpha$-phosphate group, the two oxygen atoms not linked to the sugar residue in the phosphodiester linkage between the nucleotide residues hereinafter are referred to as "non-linking oxygens". On the other hand, two oxygen atoms that are linked to the sugar residue in the phosphodiester linkage between the aforementioned nucleotide residues hereinafter are referred to as "linking oxygens". For example, the aforementioned $\alpha$-phosphate group is preferably modified to be uncharged, or to render the charge distribution between the aforementioned non-linking atoms asymmetric.

In the aforementioned phosphate group, for example, the aforementioned non-linking oxygen(s) may be substituted. The aforementioned oxygen(s) can be substituted with, for example, any atom selected from S (sulfur), Se (selenium), B (boron), C (carbon), H (hydrogen), N (nitrogen), and OR (R is, for example, an alkyl group or an aryl group) and substitution with S is preferable. It is preferable that both the aforementioned non-linking oxygens are substituted, for example, and it is more preferable that both the non-linking oxygens are substituted with S. Examples of the aforementioned modified phosphate group include phosphorothioates, phosphorodithioates, phosphoroselenates, boranophosphates, boranophosphate esters, hydrogen phosphonates, phosphoroamidates, alkyl or aryl phosphonates, and phosphotriesters. In particular, phosphorodithioate in which both of the aforementioned two non-linking oxygens are substituted with S is preferable.

In the aforementioned phosphate group, for example, the aforementioned linking oxygen(s) may be substituted. The aforementioned oxygen(s) can be substituted with, for example, any atom selected from S (sulfur), C (carbon), and N (nitrogen). Examples of the aforementioned modified phosphate group include: bridged phosphoroamidates resulting from the substitution with N; bridged phosphorothioates resulting from the substitution S; and bridged methylenephosphonates resulting from the substitution C. Preferably, substitution of the aforementioned linking oxygen(s) is performed in, for example, at least one of the 5'-terminus nucleotide residue and the 3'-terminus nucleotide residue of the aforementioned ssPN molecule. When the substitution is performed on the 5'-side, substitution with C is preferable. When the substitution is performed on the 3'-side, substitution with N is preferable.

The aforementioned phosphate group may be substituted with, for example, the aforementioned phosphate-free linker. The aforementioned linker may contain siloxane, carbonate, carboxymethyl, carbamate, amide, thioether, ethylene oxide linker, sulfonate, sulfonamide, thioformacetal, formacetal, oxime, methyleneimino, methylenemethylimino, methylenehydrazo, methylenedimethylhydrazo, methyleneoxymethylimino, or the like. Preferably, the linker may contain a methylenecarbonylamino group and a methylenemethylimino group.

In the aforementioned ssPN molecule, for example, at least one of a nucleotide residue at the 3'-terminus and a nucleotide residue at the 5'-terminus may be modified. For example, the nucleotide residue at either one of the 3'-terminus and the 5'-terminus may be modified, or the nucleotide residues at both the 3'-terminus and the 5'-terminus may be modified. The aforementioned modification may be, for example, as described above, and it is preferable to modify a phosphate group(s) at the end(s). For example, the entire aforementioned phosphate group may be modified, or one or more atoms in the aforementioned phosphate group may be modified. In the former case, for example, the entire phosphate group may be substituted or deleted.

Modification of the aforementioned nucleotide residue(s) at the end(s) may be, for example, addition of any other molecule. Examples of the aforementioned other molecule include functional molecules such as labeling substances as described above and protecting groups. Examples of the aforementioned protecting groups include S (sulfur), Si (silicon), B (boron), and ester-containing groups. The functional molecules such as the aforementioned labeling substances can be used, for example, in the detection and the like of the aforementioned ssPN molecule.

The aforementioned other molecule may be, for example, added to the phosphate group of the aforementioned nucleotide residue or may be added to the aforementioned phosphate group or the aforementioned sugar residue via a spacer. For example, the terminus atom of the aforementioned spacer can be added to or substituted for either one of the aforementioned linking oxygens of the aforementioned phosphate group, or O, N, S, or C of the sugar residue. The binding site in the aforementioned sugar residue preferably is, for example, C at the 3'-position, C at the 5'-position, or any atom bound thereto. For example, the aforementioned spacer can also be added to or substituted for a terminus atom of the aforementioned nucleotide alternative such as PNA.

The aforementioned spacer is not particularly limited, and examples thereof include $-(CH_2)_n-$, $-(CH_2)_nN-$, $-(CH_2)_nO-$, $(CH_2)_nS-$, $O(CH_2CH_2O)_nCH_2CH_2OH$, abasic sugars, amide, carboxy, amine, oxyamine, oxyimine, thioether, disulfide, thiourea, sulfonamide, and morpholino, and also biotin reagents and fluorescein reagents. In the aforementioned formulae, n is a positive integer, and n = 3 or 6 is preferable.

Other examples of the aforementioned molecule to be added to the end include dyes, intercalating agents (e.g., acridines), crosslinking agents (e.g., psoralen, mitomycin C), porphyrins (TPPC4, texaphyrin, sapphyrin), polycyclic aromatic hydrocarbons (e.g., phenazine, dihydrophenazine), artificial endonucleases (e.g., EDTA), lipophilic carriers (e.g., cholesterol, cholic acid, adamantane acetic acid, 1-pyrenebutyric acid, dihydrotestosterone, 1,3-Bis-O (hexadecyl)glycerol, a geranyloxyhexyl group, hexadecylglycerol, borneol, menthol, 1,3-propanediol, a heptadecyl group, palmitic acid, myristic acid, O3-(oleoyl)lithocholic acid, 03-(oleoyl)cholic acid, dimethoxytrityl, or phenoxathiine), peptide complexes (e.g., Antennapedia peptide, Tat peptide), alkylating agents, phosphate, amino, mercapto, PEG (e.g., PEG-40K), MPEG, $[MPEG]_2$, polyamino, alkyl, substituted alkyl, radiolabeled markers, enzymes, haptens (e.g., biotin), transport/absorption facilitators (e.g., aspirin, vitamin E, folic acid), and synthetic ribonucleases (e.g., imidazole, bisimidazole, histamine, imidazole clusters, acridine-imidazole complexes, $Eu^{3+}$ complexes of tetraazamacrocycles).

In the aforementioned ssPN molecule, for example, the aforementioned 5'-terminus may be modified with a phosphate group or a phosphate group analog. Examples of the aforementioned phosphorylation include:

> 5'-monophosphate ($(HO)_2(O)P$-O-5'); 5'-diphosphate ($(HO)_2(O)P$-OP (HO) (O)-O-5'); 5'-triphosphate ($(HO)_2(O)P$-O-(HO)(O)P-OP(HO)(O)-O-5'); 5'-guanosine cap (7-methylated or non-methylated, 7m-G-O-5'-(HO)(O)P-O-(HO)(O)P-O-P(HO)(O)-O-5'); 5'-adenosine cap (Appp); any modified or unmodified nucleotide cap structure (N-O-5'-(HO)(O)P-O-(HO)(O)P-O-P(HO)(O)-O-5'); 5'-monothiophosphate (phosphorothioate: $(HO)_2(S)P$-O-5'); 5'-monodithiophosphate (phosphorodithioate: (HO)(HS)(S)P-O-5');
> 5'-phosphorothiolate ($(HO)_2(O)P$-S-5'); sulfur substituted monophosphate, diphosphate, and triphosphates (e.g., 5'-$\alpha$-thiotriphosphate, 5'-$\gamma$-thiotriphosphate, and the like); 5'-phosphoramidates ($(HO)_2(O)P$-NH-5', $(HO)(NH_2)(O)P$-O-5'); 5'-alkylphosphonates (e.g., RP(OH)(O)-O-5', $(OH)_2(O)P$-5'-$CH_2$, where R is alkyl (e.g., methyl, ethyl, isopropyl, propyl, or the like)); and 5'-alkyletherphosphonates (e.g., RP(OH)(O)-O-5', where R is alkylether (e.g., methoxymethyl, ethoxymethyl, or the like)).

In the aforementioned nucleotide residue, the aforementioned base is not particularly limited. The aforementioned base may be, for example, a natural base or a non-natural base. The aforementioned base may be, for example, a naturally-derived base or a synthetic base. As the aforementioned base, for example, a common base, a modified analog thereof, and the like can be used.

Examples of the aforementioned base include: purine bases such as adenine and guanine; and pyrimidine bases such as cytosine, uracil, and thymine. Other examples of the aforementioned base include inosine, thymine,

xanthine, hypoxanthine, nubularine, isoguanisine, and tubercidine. Examples of the aforementioned base also include: 2-aminoadenine, alkyl derivatives such as 6-methylated purine; alkyl derivatives such as 2-propylated purine; 5-halouracil and 5-halocytosine; 5-propynyluracil and 5-propynylcytosine; 6-azouracil, 6-azocytosine, and 6-azothymine; 5-uracil (pseudouracil), 4-thiouracil, 5-halouracil, 5-(2-aminopropyl)uracil, 5-aminoallyluracil; 8-halogenated, aminated, thiolated, thioalkylated, hydroxylated, and other 8-substituted purines; 5-trifluoromethylated and other 5-substituted pyrimidines; 7-methylguanine; 5-substituted pyrimidines; 6-azapyrimidines; N-2, N-6, and 0-6 substituted purines (including 2-aminopropyladenine); 5-propynyluracil and 5-propynylcytosine; dihydrouracil; 3-deaza-5-azacytosine; 2-aminopurine; 5-alkyluracil; 7-alkylguanine; 5-alkylcytosine; 7-deazaadenine; N6,N6-dimethyladenine; 2,6-diaminopurine; 5-amino-allyl-uracil; N3-methyluracil; substituted 1,2,4-triazoles; 2-pyridinone; 5-nitroindole; 3-nitropyrrole; 5-methoxyuracil; uracil-5-oxyacetic acid; 5-methoxycarbonylmethyluracil; 5-methyl-2-thiouracil; 5-methoxycarbonylmethyl-2-thiouracil; 5-methylaminomethyl-2-thiouracil; 3-(3-amino-3-carboxypropyl)uracil; 3-methylcytosine; 5-methylcytosine; $N^4$-acetylcytosine; 2-thiocytosine; N6-methyladenine; N6-isopentyladenine; 2-methylthio-N6-isopentenyladenine; N-methylguanine; and O-alkylated bases. Examples of the purines and pyrimidines include those disclosed in U.S. Patent No. 3,687,808, "Concise Encyclopedia of Polymer Science and Engineering", pp. 858 to 859, edited by Kroschwitz J. I, John Wiley & Sons, 1990, and Englisch et al, Angewandte Chemie, International Edition, 1991, vol. 30, p. 613.

Specific examples of the aforementioned ssPN molecule to be used in the present invention include, but are not limited to, ssPN molecules shown by the below-mentioned PH-0009, PK-7006, PK-7015, PH-7069, and PH-7081.

II. Single-stranded nucleic acid molecule containing sequence controlling expression of target gene, which contains linker constituted of nucleotide residue and/or non-nucleotide residue

(1) ssNc molecule

As the aforementioned single-stranded nucleic acid molecule, a single-stranded nucleic acid molecule containing a linker constituted of a nucleotide residue(s) and/or a non-nucleotide residue(s) can be mentioned. One embodiment thereof is, for example, the single-stranded nucleic acid molecule described in WO 2012/005368, which comprises, from the 5'-side to the 3'-side, a 5'-side region (Xc), an inner region (Z), and a 3'-side region (Yc) in this order, wherein
the aforementioned inner region (Z) is constituted by linkage of an inner 5'-side region (X) and an inner 3'-side region (Y), the aforementioned 5'-side region (Xc) is complementary to the aforementioned inner 5'-side region (X),
the aforementioned 3'-side region (Yc) is complementary to the aforementioned inner 3'-side region (Y), and
at least one of the aforementioned inner region (Z), the aforementioned 5'-side region (Xc) and the aforementioned 3'-side region (Yc) comprises the aforementioned expression control sequence (hereinafter to be also referred to as "ssNc molecule"). It is needless to say that this is a mere example, and those of ordinary skill in the art obviously know that any nucleic acid molecule can be applied. Therefore, those of ordinary skill in the art can appropriately prepare a desired nucleic acid molecule based on a known technique and the common technical knowledge in the field.

In the aforementioned ssNc molecule, the aforementioned expression control sequence is a sequence that exhibits an activity of suppressing the expression of the aforementioned target gene when the ssNc molecule of the present invention is introduced into a cell in vivo or in vitro. The aforementioned expression control sequence is not particularly limited, and can be set as appropriate depending on the kind of a target gene. As the aforementioned expression control sequence, for example, a sequence involved in RNA interference caused by siRNA can be used as appropriate. That is, RNA sequence of the strand of the aforementioned siRNA, which is bound to the target mRNA, can be used as the aforementioned expression control sequence.

In the aforementioned ssNc molecule, the aforementioned 5'-side region (Xc) is complementary to the aforementioned inner 5'-side region (X) and the aforementioned 3'-side region (Yc) is complementary to the aforementioned inner 3'-side region (Y). Thus, on the 5'-side, a double strand can be formed by fold-back of the aforementioned region (Xc) toward the region (X) and self-annealing of the aforementioned regions (Xc) and (X) and, on the 3'-side, a double strand can be formed by fold-back of the aforementioned region (Yc) toward the region (Y) and self-annealing of the aforementioned regions (Yc) and (Y). Thus, the aforementioned ssNc molecule can form a double strand in a molecule and is a structure clearly different from one in which two separate single-stranded RNAs form a double-stranded RNA by annealing, such as siRNA used for conventional RNA interference.

The aforementioned expression control sequence is, for example, preferably at least 90% complementary, more preferably 95% complementary, still more preferably 98% complementary, and particularly preferably 100% complementary to a predetermined region of the aforementioned target gene. When such complementarity is satisfied, for example, an off-target effect can be reduced sufficiently.

As a specific example, when the target gene is Luciferase gene, for example, the sequence shown in SEQ ID NO: 5 can be used as the aforementioned expression control sequence and, when the target gene is mouse GAPDH gene, the sequence shown in SEQ ID NO: 6 can be used as the aforementioned expression control sequence.

5'-UCGAAGUACUCGGCGUAGG-3' (SEQ ID NO: 5)
5'-GUUGUCAUAUUUCUCGUGG-3' (SEQ ID NO: 6)

In the aforementioned ssNc molecule, the aforementioned expression control sequence is included in at least one of the aforementioned inner region (Z), the aforementioned 5'-side region (Xc) and the aforementioned 3'-side region (Yc), as described above. The aforementioned ssNc molecule may include, for example, one expression control sequence or two or more expression control sequences mentioned above.

In the latter case, the aforementioned ssNc molecule may include, for example: two or more identical expression control sequences for the same target gene; two or more different expression control sequences for the same target gene; or two or more different expression control sequences for different target genes. When the aforementioned ssNc molecule includes two or more expression control sequences mentioned above, the positions of the respective expression control sequences are not particularly limited, and they may be in one region or different regions selected from the aforementioned inner region (Z), the aforementioned 5'-side region (Xc) and the aforementioned 3'-side region (Yc). When the aforementioned ssNc molecule includes two or more expression control sequences mentioned above for different target genes, for example, the aforementioned ssNc molecule can control the expressions of two or more kinds of different target genes.

As described above, the aforementioned inner region (Z) is composed of, the aforementioned inner 5' region (X) and the aforementioned inner 3' region (Y) that are linked to each other. For example, the aforementioned regions (X) and (Y) are linked directly to each other with no intervening sequence therebetween. The aforementioned inner region (Z) is represented as being "composed of the aforementioned inner 5'-side region (X) and the aforementioned inner 3'-side region (Y) that are linked to each other" merely to indicate the sequence context between the aforementioned 5'-side region (Xc) and the aforementioned 3'-side region (Yc). This definition does not intend to limit that, for example, in the use of the aforementioned ssNc molecule, the aforementioned 5'-side region (Xc) and the aforementioned 3'-side region (Xc) in the aforementioned inner region (Z) are discrete independent regions. That is, for example, when the aforementioned expression control sequence is included in the aforementioned inner region (Z), the aforementioned expression control sequence may be arranged to extend across the aforementioned regions (X) and (Y) in the aforementioned inner region (Z).

In the aforementioned ssNc molecule, the aforementioned 5'-side region (Xc) is complementary to the aforementioned inner 5'-side region (X). It is only necessary that the aforementioned region (Xc) has a sequence complementary to the entire region or part of the aforementioned region (X). Specifically, for example, the aforementioned region (Xc) includes or is preferably composed of a sequence complementary to the entire region or part of the region (X). The aforementioned region (Xc) may be, for example, perfectly complementary to the entire region or part of the aforementioned region (X), or one or a few bases in the region (Xc) may be noncomplementary to the same. Preferably, the region (Xc) is perfectly complementary to the same. In the aforementioned ssNc molecule, the aforementioned 3'-side region (Yc) is complementary to the aforementioned inner 3'-side region (Y). It is only necessary that the aforementioned region (Yc) has a sequence complementary to the entire region or part of the aforementioned region (Y). Specifically, for example, the aforementioned region (Yc) includes or is preferably composed of a sequence complementary to the entire region or part of the aforementioned region (Y). The aforementioned region (Yc) may be, for example, perfectly complementary to the entire region or part of the aforementioned region (Y), or one or a few bases in the aforementioned region (Yc) may be noncomplementary to the same. Preferably, the aforementioned region (Yc) is perfectly complementary to the same. The aforementioned expression "one or a few bases" means, for example, 1 to 3 bases, preferably 1 base or 2 bases.

In the aforementioned ssNc molecule, the aforementioned 5'-side region (Xc) and the aforementioned inner 5'-side region (X) may be, for example, linked to each other either directly or indirectly. In the former case, for example, the aforementioned regions (Xc) and (X) may be linked directly by phosphodiester linkage or the like. In the latter case, for example, an embodiment wherein a linker region (Lx) is configured between the aforementioned region (Xc) and the aforementioned region (X) and the aforementioned regions (Xc) and (X) are linked via the aforementioned linker region (Lx) can be mentioned.

In the aforementioned ssNc molecule, for example, the aforementioned 3'-side region (Yc) and the aforementioned inner 3'-side region (Y) may be linked to each other either directly or indirectly. In the former case, for example, the aforementioned regions (Yc) and (Y) may be linked directly by phosphodiester linkage or the like. In the latter case, for example, a linker region (Ly) is present between the aforementioned regions (Yc) and (Y) and the aforementioned regions (Yc) and (Y) are linked via the aforementioned linker region (Ly).

The aforementioned ssNc molecule may have, for example, both or either one of the aforementioned linker region (Lx) and the aforementioned linker region (Ly). In the latter case, for example, a configuration having the aforementioned linker region (Lx) between the aforementioned 5'-side region (Xc) and the aforementioned inner 5'-side region (X), and free of the aforementioned linker region (Ly) between the aforementioned 3'-side region (Yc) and the aforementioned inner 3'-side region (Y), wherein the aforementioned region (Yc) and the aforementioned region (Y) are

directly linked, can be mentioned. In the latter case, for example, a configuration having the aforementioned linker region (Ly) between the aforementioned 3'-side region (Yc) and the aforementioned inner 3'-side region (Y), and free of the aforementioned linker region (Lx) between the aforementioned 5'-side region (Xc) and the aforementioned inner 5'-side region (X), wherein the aforementioned region (Xc) and the aforementioned region (X) are directly linked, can be mentioned.

The aforementioned linker region (Lx) and the aforementioned linker region (Ly) each preferably have a structure free of self-annealing inside the very region.

As regards the aforementioned ssNc molecule, one embodiment of ssNc molecule free of the aforementioned linker region is shown in WO 2012/005368, Fig. 1, and can be referred to.

As regards the aforementioned ssNc molecule, one embodiment of ssNc molecule having the aforementioned linker region is shown in WO 2012/005368, Fig. 2, and can be referred to.

In the aforementioned ssNc molecule, the base number of the aforementioned 5'-side region (Xc), the aforementioned inner 5'-side region (X), the aforementioned inner 3'-side region (Y) and the aforementioned 3'-side region (Yc) is not particularly limited and it is, for example, as described below. In the present invention, "the number of bases" means the "length", for example, and it can also be referred to as the "base length".

As described above, for example, the aforementioned 5'-side region (Xc) may be complementary to the entire region of the aforementioned inner 5'-side region (X). In this case, the aforementioned region (Xc) is as explained for the aforementioned second ssPN molecule.

Furthermore, as described above, the aforementioned 5'-side region (Xc) may be complementary to, for example, a part of the aforementioned inner 5'-side region (X). In this case, the aforementioned region (Xc) is as explained for the aforementioned second ssPN molecule.

As described above, the aforementioned 3'-side region (Yc) may be complementary to, for example, the entire region of the aforementioned inner 3'-side region (Y). In this case, the aforementioned region (Yc) is as explained for the aforementioned second ssPN molecule.

Furthermore, as described above, the aforementioned 3'-side region (Yc) may be complementary to, for example, a part of the aforementioned inner 3'-side region (Y). In this case, the aforementioned region (Yc) is as explained for the aforementioned second ssPN molecule.

In the aforementioned ssNc molecule, the relationship of the number of bases (Z) in the aforementioned inner region (Z) with the number of bases (X) in the aforementioned inner 5'-side region (X) and the number of bases (Y) in the aforementioned inner 3'-side region (Y) and the relationship of the number of bases (Z) in the aforementioned inner region (Z) with the number of bases (X) in the aforementioned inner 5'-side region (X) and the number of bases (Xc) in the aforementioned 5'-side region (Xc), is as explained for the aforementioned second ssPN molecule.

In the aforementioned ssNc molecule, the relationship between the number of bases (X) in the aforementioned inner 5'-side region (X) and the number of bases (Y) in the aforementioned inner 3'-side region (Y) is as explained for the aforementioned second ssPN molecule.

In the aforementioned ssNc molecule, the relationship between the number of bases (X) in the aforementioned inner 5'-side region (X) and the number of bases (Xc) in the aforementioned 5'-side region (Xc), the number of bases (Y) in the aforementioned inner 3'-side region (Y) and the number of bases (Yc) in the aforementioned 3'-side region (Yc) is as explained for the aforementioned second ssPN molecule.

In the aforementioned ssNc molecule, while the length of each region is as explained for the aforementioned second ssPN molecule, the present invention is not limited thereto. In the present invention, for example, the numerical range of the base number discloses all positive integers that fall within the range and, for example, "1 to 4 bases" means all of "1, 2, 3, and 4 bases" (hereinafter the same).

In the aforementioned ssNc molecule, the lengths of the aforementioned linker regions (Lx) and (Ly) are not particularly limited. The aforementioned linker region (Lx) preferably has, for example, a length permitting the aforementioned inner 5'-side region (X) and the aforementioned 5'-side region (Xc) to form a double strand, and the aforementioned linker region (Ly) preferably has, for example, a length permitting the aforementioned inner 3'-side region (Y) and the aforementioned 3'-side region (Yc) to form a double strand. When the constitutional units of the aforementioned linker region (Lx) and the aforementioned linker region (Ly) include a base(s), the base number of the aforementioned linker region (Lx) and the aforementioned linker region (Ly) may be the same or different, and also, the base sequences thereof may be the same or different. The lower limit of the number of bases in the aforementioned linker region (Lx) and the aforementioned linker region (Ly) is, for example, 1, preferably 2, and more preferably 3, and the upper limit thereof is, for example, 100, preferably 80, and more preferably 50. The number of bases in each of the aforementioned linker regions is specifically, for example, 1 to 50, 1 to 30, 1 to 20, 1 to 10, 1 to 7, or 1 to 4, but it is not limited thereto.

The full length of the aforementioned ssNc molecule is not particularly limited. In the aforementioned ssNc molecule, the lower limit of the total number of bases (the number of bases in the full length ssPN molecule), is, for example, 38, preferably 42, more preferably 50, still more preferably 51, and particularly preferably 52, and the upper limit of the same is, for example, 300, preferably 200, more preferably 150, still more preferably 100, and particularly

preferably 80. In the aforementioned ssNc molecule, the lower limit of the total number of bases excluding those in the aforementioned linker regions (Lx) and (Ly) is, for example, 38, preferably 42, more preferably 50, still more preferably 51, and particularly preferably 52, and the upper limit of the same is, for example, 300, preferably 200, more preferably 150, still more preferably 100, and particularly preferably 80.

Examples of the constitutional units of the aforementioned ssNc molecule are not particularly limited and include, for example, nucleotide residues. The aforementioned nucleotide residue may be, for example, a ribonucleotide residue or a deoxyribonucleotide residue. The aforementioned nucleotide residue may be, for example, the one that is not modified (unmodified nucleotide residue) or the one that has been modified (modified nucleotide residue). By configuring the aforementioned ssNc to include the aforementioned modified nucleotide residue, for example, the resistance of the aforementioned ssNc molecule to nuclease can be improved, thereby allowing the stability of the ssPN molecule to be improved. Furthermore, the aforementioned ssNc molecule further may include, for example, a non-nucleotide residue in addition to the aforementioned nucleotide residue.

In the aforementioned ssNc molecule, the aforementioned nucleotide residue is preferable as the constitutional unit of each of the aforementioned inner region (Z), the aforementioned 5'-side region (Xc) and the aforementioned 3'-side region (Yc). Each of the aforementioned regions is composed of, for example, any of the following residues (1) to (3):

(1) an unmodified nucleotide residue(s)
(2) a modified nucleotide residue(s)
(3) an unmodified nucleotide residue(s) and a modified nucleotide residue(s).

In the aforementioned ssNc molecule, the constitutional units of the aforementioned linker region (Lx) and the aforementioned linker region (Ly) are not particularly limited, and examples thereof include the aforementioned nucleotide residues and the aforementioned non-nucleotide residues. Each of the aforementioned linker regions may be composed of, for example, the aforementioned nucleotide residue(s) only, the aforementioned non-nucleotide residue(s) only, or both the aforementioned nucleotide residue(s) and the aforementioned non-nucleotide residue(s). Each of the aforementioned linker regions is composed of, for example, any of the following residues (1) to (7):

(1) an unmodified nucleotide residue(s)
(2) a modified nucleotide residue(s)
(3) an unmodified nucleotide residue(s) and a modified nucleotide residue(s)
(4) a non-nucleotide residue(s)
(5) a non-nucleotide residue(s) and an unmodified nucleotide residue(s)
(6) a non-nucleotide residue(s) and a modified nucleotide residue(s)
(7) a non-nucleotide residue(s), an unmodified nucleotide residue(s), and a modified nucleotide residue(s).

When the aforementioned ssNc molecule has both the aforementioned linker region (Lx) and the aforementioned linker region (Ly), for example, the both constitutional units may be the same or different. Specific examples thereof include a form wherein the constitutional unit of the both linker regions is the aforementioned nucleotide residues, a form wherein the constitutional unit of the both linker regions is the aforementioned non-nucleotide residues, a form wherein the constitutional unit of one region is the aforementioned nucleotide residues and the constitutional unit of the other linker region is a non-nucleotide residues and the like.

Examples of the aforementioned ssNc molecule include molecules composed of the aforementioned nucleotide residues only; and molecules including the aforementioned non-nucleotide residue(s) in addition to the aforementioned nucleotide residues. In the aforementioned ssNc molecule, for example, the aforementioned nucleotide residues may be the aforementioned unmodified nucleotide residues only; the aforementioned modified nucleotide residues only; or both the aforementioned unmodified nucleotide residue(s) and the aforementioned modified nucleotide residue(s), as described above. When the aforementioned ssNc molecule includes both the aforementioned unmodified nucleotide residue(s) and the aforementioned modified nucleotide residue(s), the number of the aforementioned modified nucleotide residue(s) is not particularly limited, and is, for example, "one to several", specifically, for example, 1 to 5, preferably 1 to 4, more preferably 1 to 3, and most preferably 1 or 2. When the aforementioned ssNc molecule include the aforementioned non-nucleotide residue(s), the number of the aforementioned non-nucleotide residue(s) is not particularly limited, and is, for example, "one to several", specifically, for example, 1 - 8, 1 - 6, 1 - 4, 1, 2 or 3.

In the aforementioned ssNc molecule, the aforementioned nucleotide residue is preferably, for example, ribonucleotide residue. In this case, the aforementioned ssNc molecule is also referred to as an "RNA molecule" or "ssRNA molecule". Examples of the aforementioned ssRNA molecule include molecules composed of the aforementioned ribonucleotide residues only; and a molecule including the aforementioned non-nucleotide residue(s) in addition to the aforementioned ribonucleotide residues. As described above, as the aforementioned ribonucleotide residues, for example, the aforementioned ssRNA molecule may include: the aforementioned unmodified ribonucleotide residues only; the

aforementioned modified ribonucleotide residues only; or both the aforementioned unmodified ribonucleotide residue(s) and the aforementioned modified ribonucleotide residue(s).

When the aforementioned ssRNA molecule includes, for example, the aforementioned modified ribonucleotide residue(s) in addition to the aforementioned unmodified ribonucleotide residues, the number of the aforementioned modified ribonucleotide residue(s) is as explained for the aforementioned second ssPN molecule.

The aforementioned ssNc molecule may include, for example, a labeling substance, and may be labeled with the aforementioned labeling substance. The aforementioned labeling substance is as explained for the aforementioned second ssPN molecule.

(2) Nucleotide residue

The aforementioned nucleotide residue is as explained for the aforementioned ssPN molecule.

(3) Non-nucleotide residue

The aforementioned non-nucleotide residue is not particularly limited. The aforementioned ssNc molecule may have, as the aforementioned non-nucleotide residue, for example, a non-nucleotide structure containing a pyrrolidine skeleton or a piperidine skeleton. The aforementioned non-nucleotide residue(s) is(are) preferably present at, for example, at least one of the aforementioned linker region (Lx) and the aforementioned linker region (Ly). The aforementioned non-nucleotide residue(s) may be present at, for example, the aforementioned linker region (Lx) or the aforementioned linker region (Ly) or both of the aforementioned linker regions. The aforementioned linker region (Lx) and the aforementioned linker region (Ly) may be, for example, the same or different.

The aforementioned pyrrolidine skeleton is as explained for the pyrrolidine skeleton in the aforementioned ssPN molecule.

The aforementioned piperidine skeleton is as explained for the piperidine skeleton in the aforementioned ssPN molecule.

The aforementioned linker regions may be composed of, for example, non-nucleotide residue(s) having the aforementioned non-nucleotide structure only, or may contain non-nucleotide residue(s) having the aforementioned non-nucleotide structure and nucleotide residue(s).

The aforementioned linker region is represented, for example, by the following formula (I). The aforementioned linker region is as explained for the linker region in the aforementioned ssPN molecule.

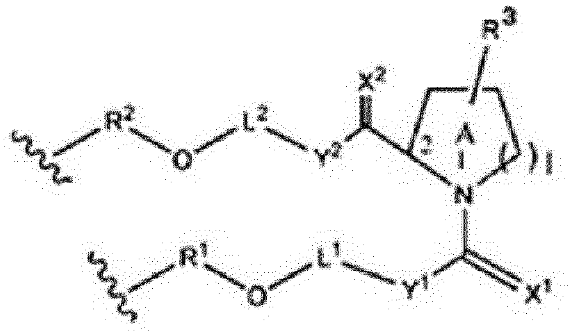

・・・(I)

When the aforementioned linker region (Ly) is represented by the aforementioned formula (I), for example, the aforementioned linker region (Ly) is as explained for the aforementioned linker region (Lx).

In the aforementioned ssNc molecule, the aforementioned linker region (Lx) may contain at least one selected from the group consisting of an amino acid residue, a polyamine residue and a polycarboxylic acid residue. The aforementioned linker region may or may not contain a residue other than the amino acid residue, polyamine residue and polycarboxylic acid residue. For example, the aforementioned linker region may contain any of a polycarboxylic acid residue, a terephthalic acid residue and an amino acid residue.

In the present invention, the "polyamine" means any compound containing a plurality of (two, three or more) amino groups. The aforementioned "amino group" is not limited to an $-NH_2$ group and also includes an imino group (-NH-). In the present invention, the aforementioned polyamine is not particularly limited, and examples thereof include 1,4-diaminobenzene, 1,3-diaminobenzene, 1,2-diaminobenzene and the like. In the present invention, moreover, the "polycarboxylic acid" means any compound containing a plurality of (two, three or more) carboxy groups. In the present

invention, the aforementioned polycarboxylic acid is not particularly limited, and examples thereof include 1,4-dicarboxybenzene (terephthalic acid), 1,3-dicarboxybenzene (isophthalic acid), 1,2-dicarboxybenzene (phthalic acid) and the like. In the present invention, moreover, the "amino acid" means any organic compound containing one or more amino groups and one or more carboxy groups in a molecule, as mentioned below. The aforementioned "amino group" is not limited to an $-NH_2$ group and also includes an imino group (-NH-).

In the aforementioned ssNc molecule, the aforementioned amino acid residue may be a plurality of interlinked amino acid residues. In the present invention, the amino acid residue that is a plurality of interlinked amino acid residues is, for example, a residue containing a peptide structure. More specifically, the aforementioned amino acid residue that is a plurality of interlinked amino acid residues is, for example, an amino acid residue of the below-mentioned chemical formula (I) wherein the below-mentioned chemical formula (Ia) is a peptide (e.g., glycine dimer or glycine trimer etc.).

In the aforementioned ssNc molecule, the aforementioned amino acid residue may be a glycine residue, a terephthalamide residue, a proline residue or a lysine residue. Also, the aforementioned amino acid residue may be a modified amino acid residue or an amino acid derivative.

In the aforementioned ssNc molecule, the aforementioned linker region residue is represented by, for example, the following chemical formula (I-0)

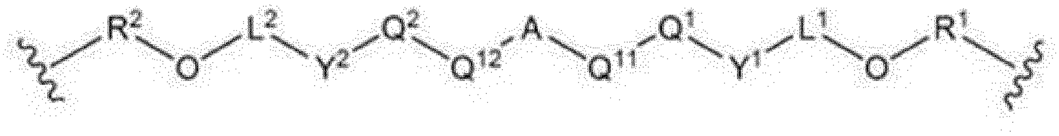

(I−0)

in the aforementioned chemical formula (I-0),

$Q^{11}$ and $Q^{12}$ are each independently a single bond, $CH_2$ (a methylene group), NH (an imino group), C=O (a carbonyl group), C=S (a thiocarbonyl group), C=NH (an iminomethylene group), O, or S,
$Q^1$ and $Q^2$ are each independently a single bond, $CH_2$ (a methylene group), NH (an imino group), C=O (a carbonyl group), C=S (a thiocarbonyl group), C=NH (an iminomethylene group), O, or S, $Y^1$ and $Y^2$ are each independently a single bond, $CH_2$, NH, O, or S;
$Y^1$ and $Y^2$ are each independently a single bond, $CH_2$, NH, O, or S;
$L^1$ is an alkylene chain having n carbon atoms, and a hydrogen atom on an alkylene carbon atom may or may not be substituted with OH, $OR^a$, $NH_2$, $NHR^a$, $NR^aR^b$, SH, or $SR^a$, or,
$L^1$ is a polyether chain obtained by substituting at least one carbon atom on the aforementioned alkylene chain with an oxygen atom,
provided that: when $Y^1$ is NH, O, or S, an atom bound to $Y^1$ in $L^1$ is carbon, an atom bound to $OR^1$ in $L^1$ is carbon, and oxygen atoms are not adjacent to each other;
$L^2$ is an alkylene chain having m carbon atoms, and a hydrogen atom on an alkylene carbon atom may or may not be substituted with OH, $OR^c$, $NH_2$, $NHR^c$, $NR^cR^d$, SH, or $SR^c$, or
$L^2$ is a polyether chain obtained by substituting at least one carbon atom on the aforementioned alkylene chain with an oxygen atom,
provided that: when $Y^2$ is NH, O, or S, an atom bound to $Y^2$ in $L^2$ is carbon, an atom bound to $OR^2$ in $L^2$ is carbon, and oxygen atoms are not adjacent to each other;
$R^a$, $R^b$, $R^c$, and $R^d$ are each independently a substituent or a protecting group;
m is an integer in the range from 0 to 30;
n is an integer in the range from 0 to 30;
the aforementioned X region and the aforementioned Y region are each linked to the aforementioned linker residue via $-OR^1-$ or $-OR^2-$,
where $R^1$ and $R^2$ may or may not be present and, when $R^1$ and $R^2$ are present, they are each independently a nucleotide residue or the aforementioned structure (I-0), and
A is any atomic group.

The combination of the aforementioned X region and the aforementioned Y region with $-OR^1-$ and $-OR^2-$ is not particularly limited, and may be, for example, any of the following conditions.

Condition (1):

the aforementioned X region is linked to the structure of the aforementioned formula (I) via $-OR^2-$ and the aforementioned Y region is linked thereto via $-OR^1-$.

Condition (2):

the aforementioned X region is linked to the structure of the aforementioned formula (I) via $-OR^1-$ and the aforementioned Y region is linked thereto via $-OR^2-$.

In the aforementioned chemical formula (I-0), for example, $Q^{11}$ may be C=O (a carbonyl group), and $Q^1$ may be NH (an imino group). In addition, for example, $Q^{11}$ may be NH (an imino group), and $Q^1$ may be C=O (a carbonyl group). Furthermore, for example, $Q^{12}$ may be C=O (a carbonyl group), and $Q^2$ may be NH (an imino group). Moreover, for example, $Q^{12}$ may be NH (an imino group), and $Q^2$ may be C=O (a carbonyl group).

In the aforementioned chemical formula (I-0), each of $Q^{11}$ and $Q^{12}$ may be, for example, a carbonyl group. In this case, each of $Q^1$ and $Q^2$ is preferably an imino group. In addition, in this case, the structure of the following chemical formula (Iα) is more preferably represented by the following chemical formula (Iα2).

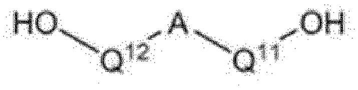

(Iα)

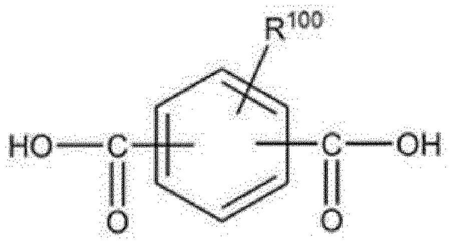

(Iα2)

In the aforementioned chemical formula (Iα2),
$R^{100}$ is any substituent, which may or may not be present. When it is present, it may be present singly or in plurality. When it is present in plurality, they may be the same or different from each other. Examples of the aforementioned any substituent for $R^{100}$ include the below-mentioned substituents exemplified as the aforementioned $R^a$, $R^b$, $R^c$ and $R^d$. More specific examples thereof include halogen, hydroxy, alkoxy, amino, carboxy, sulfo, nitro, carbamoyl, sulfamoyl, alkyl, alkenyl, alkynyl, haloalkyl, aryl, arylalkyl, alkylaryl, cycloalkyl, cycloalkenyl, cycloalkylalkyl, cyclylalkyl, hydroxyalkyl, alkoxyalkyl, aminoalkyl, silyl, silyloxyalkyl, pyrrolyl, imidazolyl and the like. The structure of the aforementioned chemical formula (Iα2) is more preferably represented by the following chemical formula (Iα3).

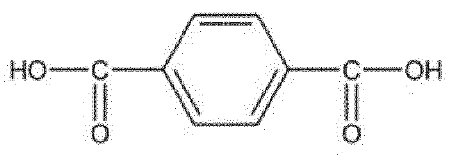

(Iα3)

When $Q^{11}$ and $Q^{12}$ are carbonyl groups, and $Q^1$ and $Q^2$ are imino groups, the linker residue of the aforementioned chemical formula (I-0) can be a carboxylic acid amide residue or a carboxylic acid residue. For example, the "TPA" structure can be a terephthalamide residue or a terephthalic acid residue represented by the aforementioned chemical formula (Iα3).

In the aforementioned chemical formula (I-0), each of $Q^{11}$ and $Q^{12}$ may be an imino group. In this case, each of $Q^1$ and $Q^2$ is preferably a carbonyl group. In this case, the structure of the following chemical formula (Iβ) is more

preferably represented by the following chemical formula (Iβ2)

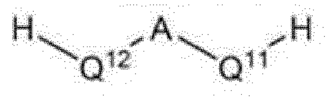

(I β)

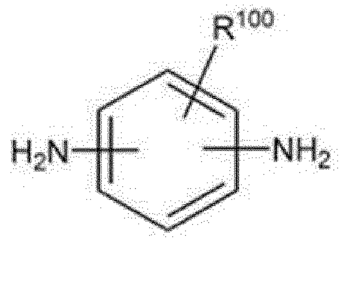

(I β 2)

In the aforementioned chemical formula (Iβ2),
$R^{100}$ is any substituent, which may or may not be present. When it is present, it may be present singly or in plurality. When it is present in plurality, they may be the same or different from each other. Specifically, for example, it is the same as $R^{100}$ in the aforementioned chemical formula (Iα2). In addition, the structure of the aforementioned chemical formula (Iβ2) is more preferably represented by the following chemical formula (Iβ3).

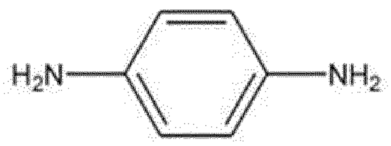

(I β 3)

In the aforementioned ssNc molecule, when the aforementioned linker residue is an amino acid residue, the aforementioned amino acid residue is represented by, for example, the following chemical formula (I). The structure of the following chemical formula (I) is one example of the structure represented by the aforementioned chemical formula (I-0).

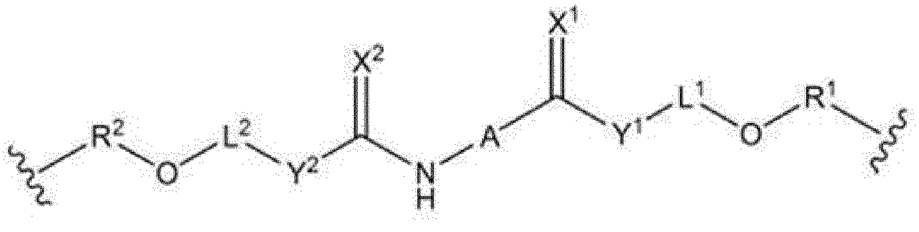

(I)

In the aforementioned formula (I), for example, $X^1$, $X^2$, $Y^1$, $Y^2$, $L^1$ and $L^2$ are as defined above.
The sequence complementary to the expression control sequence is each bound to the aforementioned amino acid residue via $-OR^1-$ or $-OR^2-$,
**[0224]** $R^1$ and $R^2$ may or may not be present, and when they are present, $R^1$ and $R^2$ are each independently a

nucleotide residue or the aforementioned structure (I), and

A is any atomic group, the following chemical formula (Ia) is an amino acid or peptide.

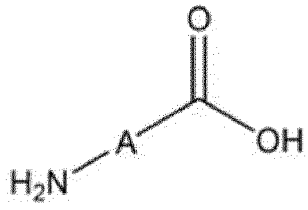

(I a)

The atomic group A in the aforementioned chemical formula (I), (Iα) or (Ia) may or may not contain, for example, at least one selected from the group consisting of chain atomic group, alicyclic atomic group, aromatic atomic group, heteroaromatic atomic group, and heteroalicyclic atomic group. While the aforementioned chain atomic group is not particularly limited, for example, alkyl, alkenyl, alkynyl, haloalkyl, hydroxyalkyl, alkoxyalkyl, aminoalkyl, silyl, silyloxyalkyl and the like can be mentioned. While the aforementioned alicyclic atomic group is not particularly limited, for example, cycloalkyl, cycloalkenyl, cycloalkylalkyl, cyclylalkyl and the like can be mentioned. While the aforementioned aromatic atomic group is not particularly limited, for example, aryl, arylalkyl, alkylaryl, condensed-ring aryl, condensed-ring arylalkyl, condensed-ring alkylaryl and the like can be mentioned. While the aforementioned heteroaromatic atomic group is not particularly limited, for example, heteroaryl, heteroarylalkyl, alkylheteroaryl, condensed ring system heteroaryl, condensed ring system heteroarylalkyl, condensed ring system alkylheteroaryl and the like can be mentioned. In the atomic group A in the aforementioned chemical formula (I), (Iα) or (Ia), each of the aforementioned atomic groups may or may not further have a substituent or a protecting group. When the aforementioned substituent or protecting group is in plurality, they may be the same or different. The aforementioned substituents are, for example, those exemplified for the aforementioned $R^a$, $R^b$, $R^c$ and $R^d$, more specifically, for example, halogen, hydroxy, alkoxy, amino, carboxy, sulfo, nitro, carbamoyl, sulfamoyl, alkyl, alkenyl, alkynyl, haloalkyl, aryl, arylalkyl, alkylaryl, cycloalkyl, cycloalkenyl, cycloalkylalkyl, cyclylalkyl, hydroxyalkyl, alkoxyalkyl, aminoalkyl, silyl, silyloxyalkyl, pyrrolyl, imidazolyl, and the like. The aforementioned protecting groups are, for example, the same as those exemplified for the aforementioned $R^a$, $R^b$, $R^c$ and $R^d$.

In the present invention, the "amino acid" refers to any organic compound containing at least one amino group and at least one carboxy group in a molecule, as mentioned above. The aforementioned "amino group" is not limited to an $-NH_2$ group and also includes an imino group (-NH-). For example, proline, hydroxyproline and the like do not contain an $-NH_2$ group in a molecule, but contains an imino group (-NH-), and is included in the definition of the "amino acid" in the present invention. In the present invention, the aforementioned "amino acid" may be, as mentioned below, a natural amino acid or an artificial amino acid. For example, a compound represented by the below-mentioned chemical formula (Ia2) or (Ia3) also contains an amino group and a carboxy group in the molecule, and therefore, it is included in the definition of the "amino acid" in the present invention. Therefore, for example, the structure of the aforementioned chemical formula (I), wherein atomic group A is represented by the below-mentioned chemical formula (A2) or chemical formula (A2a), is included in the definition of the "amino acid residue" in the present invention. In addition, for example, the "TPA" structure in the below-mentioned Example is also included in the definition of the "amino acid residue" in the present invention. In the present invention, moreover, the "peptide" refers to an organic compound having a structure wherein not less than 2 molecules of amino acid are bonded via a peptide bond. The aforementioned peptide bond may be an acid amide structure or an acid imide structure. When plural amino groups are present in the amino acid or peptide molecule represented by the aforementioned chemical formula (Ia), the amino group clearly shown in the aforementioned chemical formula (Ia) may be any amino group. In addition, when plural carboxy groups are present in the amino acid or peptide molecule represented by the aforementioned chemical formula (Ia), the carboxy group clearly shown in the aforementioned chemical formula (Ia) may be any carboxy group.

In the aforementioned amino acid residue of the aforementioned ssNc molecule, the aforementioned amino acid may be, for example, as mentioned above, natural amino acid or artificial amino acid. In the present invention, the "natural amino acid" refers to an amino acid having a naturally-occurring structure or an optical isomer thereof. The production method of the aforementioned natural amino acid is not particularly limited and, for example, it may be extracted from the nature, or may be synthesized. In the present invention, moreover, the "artificial amino acid" refers to an amino acid having a structure not occurring naturally. That is, the aforementioned artificial amino acid is an amino acid, i.e., a carboxylic acid derivative containing an amino group (organic compound containing at least one amino group and at least one carboxy group in a molecule) and having a structure not occurring naturally. The aforementioned artificial amino acid preferably does not contain, for example, a hetero ring. The aforementioned amino acid may be an amino acid constituting, for example, a protein. The aforementioned amino acid may be, for example, at least one kind selected from the group consisting of glycine, α-alanine, arginine, asparagine, aspartic acid, cysteine, cystine, glutamine, glutamic

acid, histidine, isoleucine, leucine, lysine, hydroxylysine, methionine, phenylalanine, serine, threonine, tyrosine, valine, proline, 4-hydroxyproline, tryptophan, β-alanine, 1-amino-2-carboxycyclopentane, aminobenzoic acid, aminopyridinecarboxylic acid and amino acid represented by the following chemical formula (Ia2), and may or may not further have a substituent or a protecting group. Examples of the aforementioned substituent include the substituents exemplified for the aforementioned $R^a$, $R^b$, $R^c$ and $R^d$. More specifically, for example, halogen, hydroxy, alkoxy, amino, carboxy, sulfo, nitro, carbamoyl, sulfamoyl, alkyl, alkenyl, alkynyl, haloalkyl, aryl, arylalkyl, alkylaryl, cycloalkyl, cycloalkenyl, cycloalkylalkyl, cyclylalkyl, hydroxyalkyl, alkoxyalkyl, aminoalkyl, silyl, silyloxyalkyl, pyrrolyl, imidazolyl, and the like can be mentioned. The aforementioned protecting group is the same as, for example, the protecting groups exemplified for the aforementioned $R^a$, $R^b$, $R^c$ and $R^d$. When the amino acid of the aforementioned formula (Ia), which is not peptide, contains isomers such as optical isomer, geometric isomer, stereoisomer and the like, any isomer can be used.

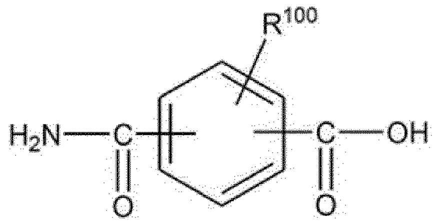

(I a 2)

In the aforementioned chemical formula (Ia2),
$R^{100}$ is any substituent, and may or may not be present. When it is present, it may be present singly or in plurality. When it is present in plurality, they may be the same or different from each other. Examples of the aforementioned any substituent for $R^{100}$ include those exemplified as the aforementioned $R^a$, $R^b$, $R^c$ or $R^d$. More specific examples thereof include halogen, hydroxy, alkoxy, amino, carboxy, sulfo, nitro, carbamoyl, sulfamoyl, alkyl, alkenyl, alkynyl, haloalkyl, aryl, arylalkyl, alkylaryl, cycloalkyl, cycloalkenyl, cycloalkylalkyl, cyclylalkyl, hydroxyalkyl, alkoxyalkyl, aminoalkyl, silyl, silyloxyalkyl, pyrrolyl, imidazolyl, and the like. In addition, the structure of the aforementioned chemical formula (Ia2) may be, for example, the following chemical formula (Ia3).

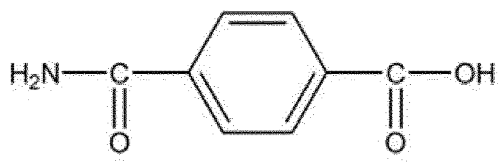

(I a 3)

When the structure of the aforementioned chemical formula (Ia) is the aforementioned chemical formula (Ia2), the structure of atomic group A in the aforementioned chemical formula (I) is represented by the following chemical formula (A2). $R^{100}$ in the following chemical formula (A2) is the same as $R^{100}$ in the aforementioned chemical formula (Ia2). In addition, when the structure of the aforementioned chemical formula (Ia) is the aforementioned chemical formula (Ia3), the structure of atomic group A in the aforementioned chemical formula (I) is represented by the following chemical formula (A2a).

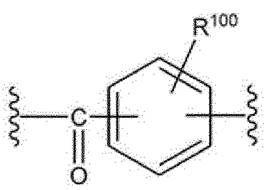

(A 2)

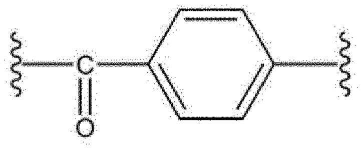

(A 2 a)

Examples of the structure of the aforementioned chemical formula (I) include the following chemical formulas (1-1) - (I-7). In the following chemical formulas (I-1) - (I-7), n and m are the same as in the aforementioned chemical formula (I).

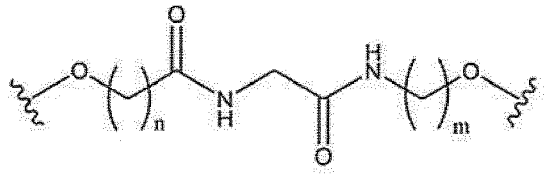

(I − 1)

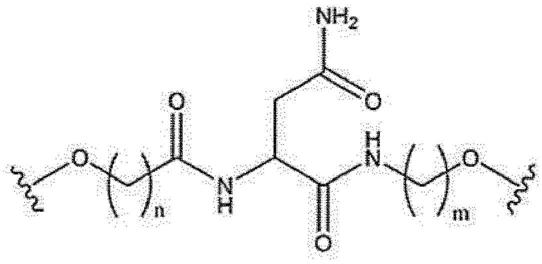

(I − 2)

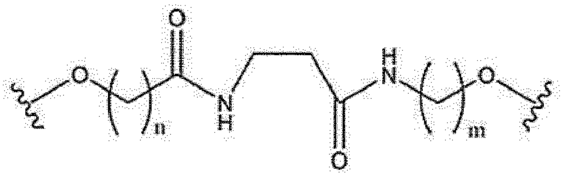

(I − 3)

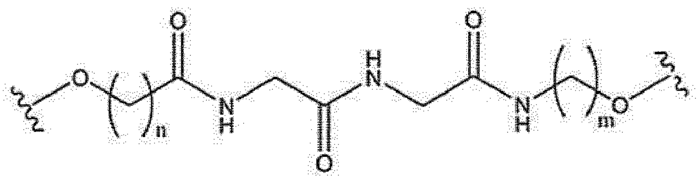

(I − 4)

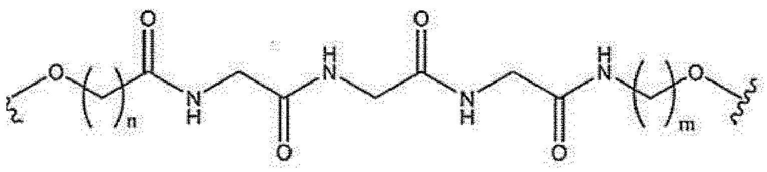

(I − 5)

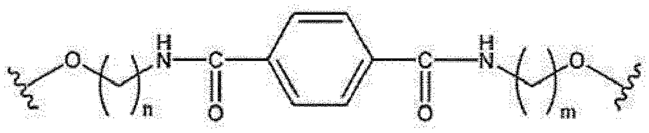

(I-6)

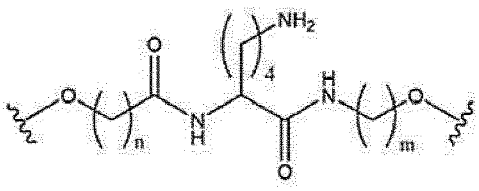

(I-7)

In the aforementioned chemical formulae (I-1) to (I-7), n and m are not particularly limited, and are as described above. Specific examples thereof include n=11 and m=12 or n=5 and m=4 in the aforementioned chemical formula (I-1), n=5 and m=4 in the aforementioned chemical formula (I-4), n=4 and m=4 in the aforementioned chemical formula (I-6), and n=5 and m=4 in the aforementioned chemical formula (1-7). The structures are shown by the following chemical formulas (I-1a), (I-1b)(I-4a), (I-6a) and (I-7a).

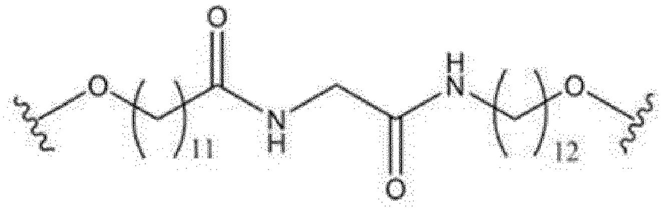

(I-1a)

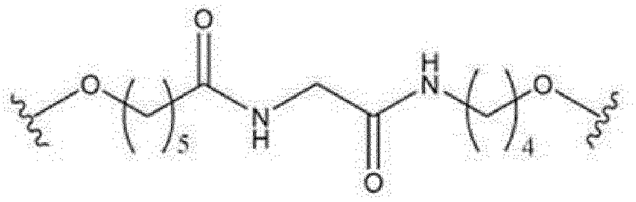

(I-1b)

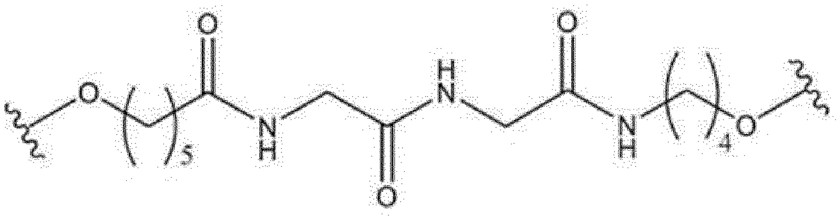

(I-4a)

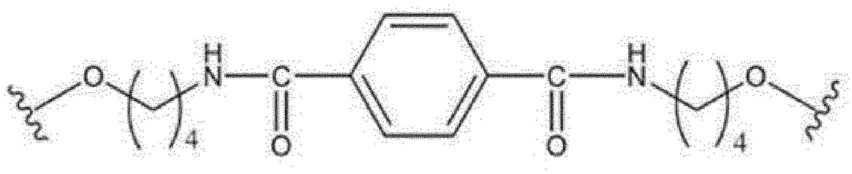

(I-6a)

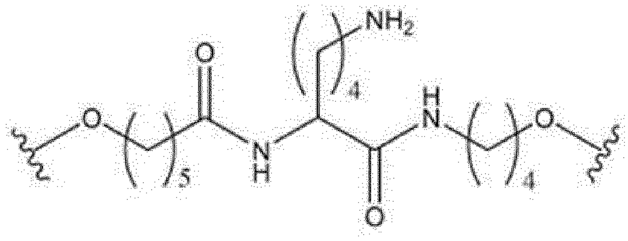

(I-7a)

Examples of the aforementioned ssNc molecule to be used in the present invention include ssNc molecules shown by the below-mentioned NK-7006 and NK-7007.

The nucleic acid molecule to be contained in the composition of the present invention can be produced by a method known per se. For example, it can be produced according to the method described in WO 2012/017919, WO 2013/103146, WO 2012/005368 or WO 2013/077446.

While the content of the nucleic acid molecule in the composition of the present invention is not particularly limited, when the composition is a pharmaceutical composition, it is generally 0.0001 - 60 wt%, preferably 0.001 - 15 wt%, further preferably 0.01 - 1 wt%, relative to the whole pharmaceutical composition.

2. Buffer

The composition of the present invention contains a buffer. In the present invention, the buffer refers to a solution (particularly aqueous solution) having a buffering action, and is constituted by containing a buffering agent. The buffering agent in the present invention means a stabilizer of the pH of an aqueous solution, and one generally used in the field of medicament production can be selected.

In the present invention, decomposition of the nucleic acid molecule in the composition can be prevented by using a buffer.

As a buffer to be used in the present invention, a buffer that adjusts the pH of the composition to not less than 4.0 and not more than 9.0 can be mentioned. A buffer that adjusts the pH of the composition to not less than 5.5 and not more than 7.5 is preferable, and a buffer that adjusts the pH of the composition to not less than 6.0 and not more than 7.0 is more preferable. Furthermore, a buffer that adjusts the pH of the composition to not less than 6.1 and not more than 6.9 is preferable, a buffer that adjusts the pH of the composition to not less than 6.2 and not more than 6.8 is preferable, a buffer that adjusts the pH of the composition to not less than 6.3 and not more than 6.7 is more preferable, a buffer that adjusts the pH of the composition to not less than 6.4 and not more than 6.6 is further preferable and a buffer that sets the pH of the composition to 6.5 is particularly preferable.

As a buffering agent to be used in the present invention, specifically, one or more buffering agents selected from ascorbic acid, magnesium L-aspartate, sodium sulfite, L-arginine, L-arginine hydrochloride, benzoic acid, sodium benzoate, epsilon-aminocaproic acid, ammonium chloride, potassium chloride, sodium chloride, glucosamine chloride, hydrochloric acid triethanolamine, dilute hydrochloric acid, citric acid, anhydrous citric acid, anhydrous sodium citrate, citric acid, sodium citrate hydrate, sodium dihydrogen citrate, disodium citrate, trisodium citrate, trisodium citrate dihydrate, potassium citrate, glycylglycine, glycine, glucono-σ-lactone, gluconic acid, calcium gluconate hydrate, L-glutamic acid, monosodium L-glutamate, creatinine, chlorobutanol, disodium hydrogen phosphate, sodium dihydrogen phosphate, succinic acid, disodium succinate hexahydrate, acetic acid, ammonium acetate, potassium acetate, sodium acetate hydrate, diisopropanolamine, diethanolamine, tartaric acid, sodium L-tartarate, potassium hydroxide, sodium hydroxide, taurine, sodium carbonate, sodium carbonate hydrate, sodium hydrogen carbonate, triisopropanolamine, triethanolamine, trometamol, carbon dioxide, lactic acid, calcium lactate hydrate, sodium lactate solution, L-histidine, 4-(2-hydroxyethyl), glacial acetic acid, glucose, monosodium fumarate, sodium propionate, benzalkonium chloride, aromatic

hydrocarbon mixed solvent, ammonium borate, maleic acid, anhydrous sodium acetate, anhydrous sodium carbonate, disodium hydrogen phosphate anhydrate, trisodium phosphate anhydrate, sodium dihydrogen phosphate anhydrate, sodium metaphosphate, methanesulfonic acid, sulfuric acid, aluminum sulfate potassium hydrate, phosphoric acid, sodium monohydrogen phosphate heptahydrate, trisodium phosphate, dibasic sodium phosphate hydrate, disodium hydrogen phosphate hydrate, sodium dihydrogen phosphatehydrate, potassium dihydrogen phosphate, sodium dihydrogen phosphate, sodium dihydrogen phosphate monohydrate can be mentioned. Of these, citric acid is preferable.

Therefore, as a buffer to be used for the composition of the present invention, a buffer containing citric acid can be preferably mentioned.

In the present invention, the buffer preferably contains an acid exemplified as the above-mentioned buffering agent and a salt thereof, or salts of two or more kinds of acids exemplified as the above-mentioned buffering agent. More preferably, a buffer containing citric acid and a salt thereof (e.g., citric acid and sodium citrate, citric acid, trisodium citrate and the like), a buffer containing phosphoric acid and a salt thereof (e.g., phosphoric acid, sodium dihydrogen phosphate and the like), and a buffer containing two kinds of phosphates (e.g., disodium hydrogen phosphate and sodium dihydrogen phosphate) can be mentioned. Particularly preferred is a buffer containing citric acid and a salt thereof.

The amount of a buffer to be used for the composition of the present invention may be any as long as it can adjust to a desired pH range. For example, it can be appropriately determined to make the content of the buffering agent in the composition fall within the following range. That is, the content of the buffering agent in the composition of the present invention is generally 0.0001 - 40 wt%, preferably 0.0005 - 20 wt%, further preferably 0.001 - 10 wt%, relative to the whole composition.

3. Other additive

The composition of the present invention may further contain a solvent. Examples of the solvent include pharmaceutically acceptable organic solvents (e.g., ethanol, propylene glycol, polyethylene glycol, glycerol etc.), water, water for injection, saline, glucose solution and the like. One or more kinds of solvent may be used in combination.

In the present invention, a nucleic acid molecule is preferably dissolved in a solvent and mixed with a buffer, since the nucleic acid molecule can be dissolved in a short time. As the solvent, water is preferable. In the present specification, unless otherwise specified, that "nucleic acid molecule is dissolved in a buffer" means not only that a nucleic acid molecule as a solid is directly dissolved in a buffer but that, as mentioned above, a nucleic acid molecule is once dissolved in a solvent such as water and the like and the obtained solution is mixed with a buffer.

In the present invention, the content of the solvent is generally not less than 0.0001 wt% and less than 100 wt%, preferably not less than 0.001 wt% and less than 100 wt%, further preferably not less than 0.005 wt% and less than 100 wt%, as the total amount relative to the whole composition.

When the composition of the present invention is a pharmaceutical composition, the composition can be formulated as, for example, inhalant liquid, injection, liquid and the like by a known method, and administered by parenteral administration (e.g., transnasal administration, intravenous administration, instillation, intramuscular administration, subcutaneous administration etc.). In addition, it can be orally administered in a suitable dosage form (e.g., capsule etc.).

The pharmaceutical composition of the present invention may also contain, besides the above-mentioned components, a pharmaceutically acceptable additive as necessary. When the pharmaceutical composition of the present invention is an injection, examples of the additive include isotonicity agent (e.g., glucose, D-sorbitol, sodium chloride, glycerol, D-mannitol etc.), soothing agent (e.g., benzyl alcohol etc.), preservative (e.g., methyl benzoate, paraoxybenzoates, chlorobutanol, benzyl alcohol etc.) and the like. A preferable additive is methyl benzoate.

When the pharmaceutical composition of the present invention is an injection, it can also be produced as a liposome preparation encapsulating a nucleic acid molecule, by dissolving the nucleic acid molecule in a buffer and contacting the obtained solution with a constituent molecule of lipid membrane. The liposome preparation can be preferably used as an injection for systemic administration, such as intravenous injection, intramuscular injection and the like.

When the pharmaceutical composition of the present invention is formulated as an inhalant, for example, a solution obtained by dissolving a nucleic acid molecule in a solvent such as water and the like is mixed with an aqueous solution added with a buffering agent (e.g., citric acid and a salt thereof, phosphoric acid and a salt thereof), the mixture is filtered for bacterial elimination, and the obtained drug solution is filled in a tightly-sealed container such as vial, ampoule and the like to produce an inhalant. For example, a nucleic acid molecule is mixed with an aqueous solution containing water and a buffering agent (e.g., citric acid and a salt thereof, phosphoric acid and a salt thereof), dissolved by sonication and the like, filtered for bacterial elimination, and the obtained drug solution is filled in a tightly-sealed container such as vial, ampoule and the like to produce an inhalant. While a tightly-sealed container to be used is generally a colorless and transparent borosilicate glass container, a container in which a liquid contact part on the glass inner part has quartz-like surface property can also be used.

In the pharmaceutical composition of the present invention, the nucleic acid molecule as the active ingredient is useful for the treatment or prophylaxis of various diseases.

For example, administration to a patient with a disease caused by a gene can control the expression of the aforementioned gene, thereby treating the aforementioned disease. In the present invention, the term "treatment" encompasses, for example, prevention of the aforementioned diseases; improvement of the diseases; and improvement in prognosis, as mentioned above and it can mean any of them.

A specific example is as follows. By setting the TGF-β1 gene as the aforementioned target gene and incorporating an expression suppressive sequence (e.g., nucleotide sequence shown in SEQ ID NO: 4) for the aforementioned gene into the aforementioned ssPN molecule, it can be used for the treatment of diseases or pathology for which a treatment effect is expected by suppressing TGF-β1.

The method of using the pharmaceutical composition of the present invention is not particularly limited. For example, the aforementioned pharmaceutical composition may be administered to a subject having the aforementioned target gene.

Examples of the aforementioned subject to which the pharmaceutical composition of the present invention is administered include cells, tissues, organs and the like. Examples of the aforementioned subject also include humans, nonhuman animals and the like such as nonhuman mammals, i.e., mammals excluding humans. The aforementioned administration may be performed, for example, in vivo or in vitro. The aforementioned cells are not particularly limited, and examples thereof include: various cultured cells such as HeLa cells, 293 cells, NIH3T3 cells, and COS cells; stem cells such as ES cells and hematopoietic stem cells; and cells isolated from living organisms, such as primary cultured cells.

Since the pharmaceutical composition of the present invention is low toxic, it can be safely administered to mammals (e.g., human, mouse, rat, rabbit, dog, cat, bovine, horse, swine, monkey), particularly human.

The dose of the pharmaceutical composition of the present invention also varies depending on the subject of administration, administration route, disease and the like. For example, when it is administered as a therapeutic agent for idiopathic pulmonary fibrosis as an inhalant liquid to an adult, the dose of the nucleic acid molecule as an active ingredient is about 0.001 to about 20 mg/kg body weight, preferably about 0.005 to about 5 mg/kg body weight, more preferably about 0.01 to about 1 mg/kg body weight, which can be administered in one to several portions per day.

The present invention also relates to a method for stabilizing the nucleic acid molecule in the composition, which comprises adding a buffer to the nucleic acid molecule, or a production method of a stable composition containing nucleic acid molecule. As a buffer used for this method, those similar to the aforementioned examples of the composition of the present invention can be mentioned, and a similar one is preferable.

The amount of the buffer to be added in the stabilizing/production method of the present invention may be any as long as the pH can be adjusted to a desired range. For example, the amount of the buffering agent can be appropriately determined to fall within the following range. That is, the amount of the buffering agent to be added in the stabilizing/production method of the present invention is generally 0.0001 - 40 wt%, preferably 0.0005 - 20 wt%, further preferably 0.001 - 10 wt%, relative to the whole composition obtained by the method.

The present invention is explained in more detail in the following by referring to Examples, which are not to be construed as limitative.

[Examples]

Production Example 1 (synthesis of single-stranded nucleic acid molecule)

The single-stranded nucleic acid molecule shown below was synthesized by a nucleic acid synthesizer (trade name: ABI Expedite (registered trademark) 8909 Nucleic Acid Synthesis System, Applied Biosystems) based on the phosphoramidite method. For the aforementioned synthesis, RNA Phosphoramidites (2'-O-TBDMSi, trade name, Samchully Pharm. Co., Ltd.) was used as RNA amidite (hereinafter the same). The aforementioned amidite was deprotected by a conventional method, and the synthesized RNA was purified by HPLC. Each RNA after purification was freeze-dried.

As the single-stranded nucleic acid molecule of Examples 1 - 4, PH-0009 (PshRNA) was synthesized as mentioned above. In PshRNA, Lx is linker region Lx, and the following structural formula was formed using L-proline diamide amidite. The underline shows an expression suppressive sequence of human TGF-β1 gene.

PshRNA (PH-0009)

5'-GCAGAGUACACACAGCAUAUACC-Lx-GGUAUAUGCUGUGUGUACUCUGCUU-3' (SEQ ID NO: 7)

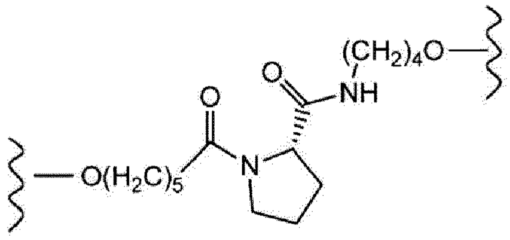

Example 1 (evaluation of influence of pH on storage temperature)

Example 1-1 (preparation of test composition)

The thermal stability of PH-0009-containing composition of a prototype for inhalation of nucleic acid was evaluated. The following test compositions 1 - 12 were prepared by a method generally used in this field.

test composition 1: PH-0009 formulation 19 (0.04 M Britton-Robinson buffer (pH 2.0)), (0.1 mg/mL)
test composition 2: PH-0009 formulation 20 (0.04 M Britton-Robinson buffer (pH 3.0)), (0.1 mg/mL)
test composition 3: PH-0009 formulation 21 (0.04 M Britton-Robinson buffer (pH 4.0)), (0.1 mg/mL)
test composition 4: PH-0009 formulation 22 (0.04 M Britton-Robinson buffer (pH 5.0)), (0.1 mg/mL)
test composition 5: PH-0009 formulation 23 (0.04 M Britton-Robinson buffer (pH 6.0)), (0.1 mg/mL)
test composition 6: PH-0009 formulation 24 (0.04 M Britton-Robinson buffer (pH 7.0)), (0.1 mg/mL)
test composition 7: PH-0009 formulation 25 (0.04 M Britton-Robinson buffer (pH 8.0)), (0.1 mg/mL)
test composition 8: PH-0009 formulation 26 (0.04 M Britton-Robinson buffer (pH 9.0)), (0.1 mg/mL)
test composition 9: PH-0009 formulation 27 (0.04 M Britton-Robinson buffer (pH 10.0)), (0.1 mg/mL)
test composition 10: PH-0009 formulation 28 (0.04 M Britton-Robinson buffer (pH 11.0)), (0.1 mg/mL)
test composition 11: PH-0009 formulation 29 (0.04 M Britton-Robinson buffer (pH 12.0)), (0.1 mg/mL)
test composition 12: PH-0009 formulation 30 (0.04 M
hydrochloric acid-potassium chloride buffer (pH 1.5)), (0.1 mg/mL)

Example 1-2 (test method and diagnostic criteria)

The test compositions 1 - 12 were each stored in a stability test chamber at 25°C/60%RH, 40°C/75%RH and 60°C. Each stored product was taken out every week, the content was calculated by ion exchange HPLC, and the stability was evaluated based on a decrease in the content ratio (%) relative to the content at the time of start of the storage. The storage period and number of products stored are shown in Table 2 - Table 5.

Changes in the content ratio (%) relative to the content at the time of start of the storage were confirmed up to 4 weeks under each storage condition, and the evaluation was continued for the formulations judged to be superior in stability.

The test compositions 1 - 12 stored for 1 week, 2 weeks, 3 weeks and 4 weeks at 25°C/60%RH, 40°C/75%RH and 60°C, respectively were used as storage samples.

Separately, PH-0009 was prepared at 0.1 mg/mL by using water for injection and used as a calibration curve sample (100%). The calibration curve sample (100%) was taken by 90 μL, water for injection (10 μL) was added to 100 μL and used as a calibration curve sample (90%). The calibration curve sample (100%) was taken by 80 μL, water for injection (20 μL) was added to 100 μL and used as a calibration curve sample (80%). The calibration curve sample (100%) was taken by 70 μL, water for injection (30 μL) was added to 100 μL and used as a calibration curve sample (70%). The calibration curve sample (100%) was taken by 60 μL, water for injection (40 μL) were added to 100 μL and used as a calibration curve sample (60%).

Each calibration curve sample (60% - 100%) and each storage sample (each 10 μL) were measured by HPLC. With regard to the peak areas obtained by calibration curve samples (60% - 100%), the regression line (Y=aX+b) and correlation coefficient (r) thereof were determined by the least-squares method with the theoretical content (%) on the horizontal axis (X) and the peak area on the vertical axis (Y), and the content ratio (%) of each sample relative to the content at the time of start of the storage was calculated (excel 2013).

Table 2

| storage period and number of products stored | | | | |
|---|---|---|---|---|
| sample | formulation | formulation 19 | formulation 20 | formulation 21 |
| | | 0.04 M Britton-Robinson buffer (pH 2.0) | 0.04 M Britton-Robinson buffer (pH 3.0) | 0.04 M Britton-Robinson buffer (pH 4.0) |
| | nucleic acid | PH-0009 | PH-0009 | BPH-0009 |
| | mg/mL | 0.1 | 0.1 | 0.1 |
| test composition No. | | 1 | 2 | 3 |
| Found | 0 | 1 | 1 | 1 |
| 40°C | 1 week | 1 | 1 | 1 |
| | 2 weeks | 1 | 1 | 1 |
| | 3 weeks | 1 | 1 | 1 |
| | 4 weeks | 1 | 1 | 1 |
| 60°C | 1 week | 1 | 1 | 1 |
| | 2 weeks | 1 | 1 | 1 |
| | 3 weeks | 1 | 1 | 1 |
| | 4 weeks | 1 | 1 | 1 |

Table 3

| storage period and number of products stored | | | | |
|---|---|---|---|---|
| sample | formulation | formulation 22 | formulation 23 | formulation 24 |
| | | 0.04 M Britton-Robinson buffer (pH 5.0) | 0.04 M Britton-Robinson buffer (pH 6.0) | 0.04 M Britton-Robinson buffer (pH 7.0) |
| | nucleic acid | PH-0009 | PH-0009 | PH-0009 |
| | mg/mL | 0.1 | 0.1 | 0.1 |
| test composition No. | | 4 | 5 | 6 |
| Found | 0 | 1 | 1 | 1 |
| 40°C | 1 week | 1 | 1 | 1 |
| | 2 weeks | 1 | 1 | 1 |
| | 3 weeks | 1 | 1 | 1 |
| | 4 weeks | 1 | 1 | 1 |
| 60°C | 1 week | 1 | 1 | 1 |
| | 2 weeks | 1 | 1 | 1 |
| | 3 weeks | 1 | 1 | 1 |
| | 4 weeks | 1 | 1 | 1 |

[Table 4]

| storage period and number of products stored | | | | |
|---|---|---|---|---|
| sample | formulation | formulation 25 | formulation 26 | formulation 27 |
| | | 0.04 M Britton-Robinson buffer (pH 8.0) | 0.04 M Britton-Robinson buffer (pH 9.0) | 0.04 M Britton-Robinson buffer (pH 10.0) |
| | nucleic acid | B | B | B |
| | | PH-0009 | PH-0009 | PH-0009 |
| | mg/mL | 0.1 | 0.1 | 0.1 |
| test composition No. | | 7 | 8 | 9 |
| Found | 0 | 1 | 1 | 1 |
| 40°C | 1 week | 1 | 1 | 1 |
| | 2 weeks | 1 | 1 | 1 |
| | 3 weeks | 1 | 1 | 1 |
| | 4 weeks | 1 | 1 | 1 |
| 60°C | 1 week | 1 | 1 | 1 |
| | 2 weeks | 1 | 1 | 1 |
| | 3 weeks | 1 | 1 | 1 |
| | 4 weeks | 1 | 1 | 1 |

[Table 5]

| storage period and number of products stored | | | | |
|---|---|---|---|---|
| sample | formulation | formulation 28 | formulation 29 | formulation 30 |
| | | 0.04 M Britton-Robinson buffer (pH 11.0) | 0.04 M Britton-Robinson buffer (pH 12.0) | 0.04 M hydrochloric acid-potassium chloride buffer (pH 1.5) |
| | nucleic acid | PH-0009 | PH-0009 | PH-0009 |
| | mg/mL | 0.1 | 0.1 | 0.1 |
| test composition No. | | 10 | 11 | 12 |
| Found | 0 | 1 | 1 | 1 |
| 40°C | 1 week | 1 | 1 | 1 |
| | 2 weeks | 1 | 1 | 1 |
| | 3 weeks | 1 | 1 | 1 |
| | 4 weeks | 1 | 1 | 1 |
| 60°C | 1 week | 1 | 1 | 1 |
| | 2 weeks | 1 | 1 | 1 |
| | 3 weeks | 1 | 1 | 1 |
| | 4 weeks | 1 | 1 | 1 |

Measurement method

The calibration curve samples (60% - 100%) and respective samples were measured under the following measurement conditions.

detector: ultraviolet absorptiometer (measurement wavelength: 254 nm)
column: X-Bridge OST C18 (2.5 μm, 4.6×50 mm)
column temperature: 40°C
mobile phase A: 50 mM TEAA (pH 7.0), 0.5% Acetonitrile
mobile phase B: 100% Acetonitrile
mobile phase feed: The mixing ratio of mobile phase A and
mobile phase B was changed as follows to control concentration gradient (Table 6).

[Table 6]

| time after injection (min) | mobile phase A (vol%) | mobile phase B (vol%) |
|---|---|---|
| 0→12 | 100→60 | 0→40 |
| flow: 1.0 mL/min | | |

Example 1-3 (results)

The results are shown in Fig. 1 - Fig. 3. Since a clear decrease in the content ratio (%) relative to the content at the time of start of the storage was not observed within the pH range of 5 - 7 under the severest conditions of 60°C, 4 weeks storage, the pH range was set to 5 - 7 for the PH-0009-containing compositions.

In general, nucleic acid is easily influenced by temperature and storage thereof at ambient temperature or above for a long time is said to be impossible. The results have shown that single-stranded nucleic acid can be stored for a long term even at ambient temperature or above by controlling the pH of the solution.

Example 2 (citrate buffer and evaluation of stability at concentration thereof)

Example 2-1 (test composition)

The thermal stability of PH-0009-containing composition of a prototype for inhalation of nucleic acid was evaluated.

Using 0.05 M citrate buffer (pH 6.8) and 0.005 M citrate buffer (pH 6.8) as base formulations, the thermal stability of the following test compositions 13 and 14 in each solution was evaluated.

The test composition 13 was prepared as follows.

Citric acid hydrate (21.0 g) was dissolved in water for injection (1 L) to give 0.1 M citric acid solution. Similarly, trisodium citrate dihydrate (29.4 g) was dissolved in water for injection (1 L) to give 0.1 M sodium citrate solution. The 0.1 M citric acid solution was added to the 0.1 M sodium citrate solution to adjust the pH to 6.8, and the mixture was used as 0.1 M citrate buffer (pH 6.8).

Separately, nucleic acid (PH-0009) (10 mg) was dissolved in water for injection (0.5 mL). This solution (0.2 mL) was mixed with water for injection (20 mL). Thereto was added 0.1 M citrate buffer (pH 6.8) (20 mL) and the mixture was stirred and passed through a 0.22 μm polyvinylidene difluoride (PVDF) filter to give 4 mg/40 mL (0.1 mg/mL) PH-0009-containing composition.

The test composition 14 was prepared as follows.

Citric acid hydrate (21.0 g) was dissolved in water for injection (1 L) to give 0.1 M citric acid solution. Similarly, trisodium citrate dihydrate (29.4 g) was dissolved in water for injection (1 L) to give 0.1 M sodium citrate solution. The 0.1 M citric acid solution was added to the 0.1 M sodium citrate solution to adjust the pH to 6.8, and the mixture was used as 0.1 M citrate buffer (pH 6.8). 0.1 M citric acid solution (18 mL), 0.1 M sodium citrate solution (82 mL), and water for injection (900 mL) were mixed, and adjusted to pH6.8 with 1N NaOH to give 0.01 M citrate buffer (pH 6.8).

Separately, nucleic acid (PH-0009) (13.9 mg) was dissolved in water for injection (1 mL). This solution (0.0719 mL) was mixed with water for injection (4.9281 mL). Thereto was added 0.01 M citrate buffer (pH 6.8) (5 mL) and the mixture was stirred and passed through a 0.22 μm polyvinylidene difluoride (PVDF) filter to give 1 mg/10 mL (0.1 mg/mL) PH-0009-containing composition.

test composition 13: PH-0009 formulation 3 (0.05 M citrate buffer (pH 6.8)), (0.1 mg/mL)
test composition 14: PH-0009 formulation 7 (0.005 M citrate buffer (pH 6.8)), (0.1 mg/mL)

Example 2-2 (test method and diagnostic criteria)

The test compositions 13 and 14 were each stored in a stability test chamber at 40°C/75%RH and 60°C. Each stored product was taken out every week, the content was calculated by ion exchange HPLC, and the stability was evaluated based on a decrease in the content ratio (%) relative to the content at the time of start of the storage. The storage period and number of products stored are shown in Table 7.

Changes in the content ratio (%) relative to the content at the time of start of the storage were confirmed up to 4 weeks under each storage condition, and the evaluation was continued for the formulations judged to be superior in stability.

The test compositions 13 and 14 stored for 1 week, 2 weeks, 3 weeks and 4 weeks at 40°C/75%RH and 60°C, respectively were used as storage samples.

Separately, PH-0009 was prepared at 0.1 mg/mL by using water for injection and used as a calibration curve sample (100%). The calibration curve sample (100%) was taken by 90 μL, water for injection (10 μL) was added to 100 μL and used as a calibration curve sample (90%). The calibration curve sample (100%) was taken by 80 μL, water for injection (20 μL) was added to 100 μL and used as a calibration curve sample (80%). The calibration curve sample (100%) was taken by 70 μL, water for injection (30 μL) was added to 100 μL and used as a calibration curve sample (70%). The calibration curve sample (100%) was taken by 60 μL, water for injection (40 μL) were added to 100 μL and used as a calibration curve sample (60%).

Each calibration curve sample (60% - 100%) and each storage sample (each 10 μL) were measured by HPLC. With regard to the peak areas obtained by calibration curve samples (60% - 100%), the regression line (Y=aX+b) and correlation coefficient (r) thereof were determined by the least-squares method with the theoretical content (%) on the horizontal axis (X) and the peak area on the vertical axis (Y), and the content ratio (%) of each sample relative to the content at the time of start of the storage was calculated (excel 2013).

[Table 7]

| storage period and number of products stored | | | |
|---|---|---|---|
| sample | formulation | formulation 3 | formulation 7 |
| | | 0.05 M citrate buffer (pH 6.8) | 0.005 M citrate buffer (pH 6.8) |
| | nucleic acid | PH-0009 | PH-0009 |
| | mg/mL | 0.1 | 0.1 |
| test composition No. | | 13 | 14 |
| Found | 0 | 1 | 1 |
| 40°C | 1 week | 1 | 1 |
| | 2 weeks | 1 | 1 |
| | 3 weeks | 1 | 1 |
| | 4 weeks | 1 | 1 |
| 60°C | 1 week | 1 | 1 |
| | 2 weeks | 1 | 1 |
| | 3 weeks | 1 | 1 |
| | 4 weeks | 1 | 1 |

Measurement method

The calibration curve samples (60% - 100%) and respective samples were measured under the following measurement conditions.

detector: ultraviolet absorptiometer (measurement wavelength: 254 nm)
column: X-Bridge OST C18 (2.5 μm, 4.6×50 mm)
column temperature: 40°C
mobile phase A: 50 mM TEAA (pH 7.0), 0.5% Acetonitrile

mobile phase B: 100% Acetonitrile
mobile phase feed: The mixing ratio of mobile phase A and
mobile phase B was changed as follows to control concentration gradient (Table 8).

[Table 8]

| time after injection (min) | mobile phase A (vol%) | mobile phase B (vol%) |
|---|---|---|
| 0→12 | 100→60 | 0→40 |
| flow: 1.0 mL/min | | |

Example 2-3 (results)

The results are shown in Fig. 4 and Fig. 5. The results show that a clear change in the content ratio (%) relative to the content at the time of start of the storage was absent even at the concentration range of the citrate buffer of 0.005 M - 0.05 M at 60°C for 4 weeks when the single-stranded nucleic acid was prepared at 0.1 mg/mL. Therefrom it is suggested that the effect of the stability of nucleic acid can be maintained by controlling the concentration of the citrate buffer even when the concentration of the single-stranded nucleic acid increases.

Example 3 (preparation of PH-0009-containing composition (10 mg/mL))

A preparation method of a 10 mg/mL PH-0009-containing composition was performed. A 1 mg/mL PH-0009-containing composition can be prepared by changing the standard amount of nucleic acid to be charged to 1.0 g. In the case of 0.1 mg/mL, the amount is changed to 0.10 g.
Citric acid hydrate (21.0 g) was dissolved in water for injection (1 L) to give 0.1 M citric acid solution. Similarly, trisodium citrate dihydrate (29.4 g) was dissolved in water for injection (1 L) to give 0.1 M sodium citrate solution. The 0.1 M citric acid solution was added to the 0.1 M sodium citrate solution to adjust the pH to 6.5 to give 0.1 M citrate buffer (pH 6.5).
Separately, nucleic acid (PH-0009) (10 g) was dissolved in water for injection (500 mL). Thereto was added 0.1 M citrate buffer (pH 6.5) (500 mL) and the mixture was stirred and passed through a 0.22 μm polyvinylidene difluoride (PVDF) filter to give 10 g/L (10 mg/mL) PH-0009-containing composition. The composition can be utilized as inhalant preparation for IPF and the like.

[Table 9]

| Component name (trade name or grade) | Standard charge amount |
|---|---|
| PH-0009 (single-stranded nucleic acid) | 10.0 g |
| citric acid hydrate (the Japanese Pharmacopoeia) | 0.914 g |
| trisodium citrate dihydrate (JIS standard) | 29.4 g |
| water for injection (the Japanese Pharmacopoeia) | 1 L |

Example 4 (evaluation of temperature stability of PH-0009-containing composition)

Example 4-1 (test composition)

In the development of a preparation of a single-stranded nucleic acid, various formulations were evaluated for the stability of PH-0009-containing composition. As a result, test compositions 15 and 16 were judged to be the most superior formulations as nucleic acid pharmaceutical products, and the thermal stability of these was evaluated.
The test compositions 15 and 16 were obtained in the same manner as in Example 3 and test compositions 13, 14.

test composition 15:PH-0009 formulation 44 (0.05 M citrate buffer (pH 6.5), (1 mg/mL)
test composition 16:PH-0009 formulation 44 (0.05 M citrate buffer (pH 6.5), (10 mg/mL)

Example 4-2 (test method and diagnostic criteria)

The test compositions 15 and 16 were each stored in a stability test chamber at 25°C/60%RH, 40°C/75%RH and 60°C. Each stored product was taken out every week, the content was calculated by ion exchange HPLC, and the stability was evaluated based on a decrease in the content ratio (%) relative to the content at the time of start of the storage. The storage period and number of products stored are shown in Table 10.

Changes in the content ratio (%) relative to the content at the time of start of the storage were confirmed up to 4 weeks under each storage condition, and the evaluation was continued for the formulations judged to be superior in stability.

The test compositions 15 and 16 stored for 1 week, 2 weeks, 3 weeks and 4 weeks at 40°C/75%RH and 60°C, respectively were used as storage samples.

Separately, PH-0009 was prepared at 1 mg/mL by using water for injection and used as a calibration curve sample (100%). The calibration curve sample (100%) was taken by 90 μL, water for injection (10 μL) was added to 100 μL and used as a calibration curve sample (90%). The calibration curve sample (100%) was taken by 80 μL, water for injection (20 μL) was added to 100 μL and used as a calibration curve sample (80%). The calibration curve sample (100%) was taken by 70 μL, water for injection (30 μL) was added to 100 μL and used as a calibration curve sample (70%). The calibration curve sample (100%) was taken by 60 μL, water for injection (40 μL) were added to 100 μL and used as a calibration curve sample (60%).

Each calibration curve sample (60% - 100%) and each storage sample (each 10 μL) were measured by HPLC. As for the 10 mg/mL sample, 1 μL was measured by HPLC. With regard to the peak areas obtained by calibration curve samples (60% - 100%), the regression line (Y=aX+b) and correlation coefficient (r) thereof were determined by the least-squares method with the theoretical content (%) on the horizontal axis (X) and the peak area on the vertical axis (Y), and the content ratio (%) of each sample relative to the content at the time of start of the storage was calculated (excel 2013).

[Table 10]

| storage period and number of products stored | | | |
|---|---|---|---|
| sample | formulation | formulation 44 | |
| | | 0.05 M citrate buffer (pH 6.5) | |
| | nucleic acid | PH-0009 | |
| | mg/mL | 1 | 10 |
| test composition No. | | 15 | 16 |
| found | 0 | 1 | 1 |
| 40°C | 1 week | 1 | 1 |
| | 2 weeks | 1 | 1 |
| | 3 weeks | 1 | 1 |
| | 4 weeks | 1 | 1 |
| 60°C | 1 week | 1 | 1 |
| | 2 weeks | 1 | 1 |
| | 3 weeks | 1 | 1 |
| | 4 weeks | 1 | 1 |

Measurement method

The calibration curve samples (60% - 100%) and respective samples were measured under the following measurement conditions.

detector: ultraviolet absorptiometer (measurement wavelength: 254 nm)
column: X-Bridge OST C18 (2.5 μm, 4.6×50 mm)
column temperature: 40°C

mobile phase A: 50 mM TEAA (pH 7.0), 0.5% Acetonitrile
mobile phase B: 100% Acetonitrile
mobile phase feed: The mixing ratio of mobile phase A and
mobile phase B was changed as follows to control concentration gradient (Table 11).

[Table 11]

| time after injection (min) | mobile phase A (vol%) | mobile phase B (vol%) |
|---|---|---|
| 0→12 | 100→60 | 0→40 |
| flow: 1.0 mL/min | | |

Example 4-3 (results)

The results of the test composition 16 are shown in Fig. 6. The results show that a clear change in the content ratio (%) relative to the content at the time of start of the storage was absent in a 10 mg/mL PH-0009-containing composition even at 60°C for 4 weeks. Similar results were obtained with test composition 15 (1 mg/mL PH-0009-containing composition). It was shown thereby that the PH-0009-containing composition (inhalant of single-stranded nucleic acid liquid and the like) prepared according to this formulation has high storage stability.

Production Example 2

As the nucleic acid molecule of Example 5, the strand nucleic acid molecule shown below was synthesized by a nucleic acid synthesizer (trade name: ABI Expedite (registered trademark) 8909 Nucleic Acid Synthesis System, Applied Biosystems) based on the phosphoramidite method. For the aforementioned synthesis, RNA Phosphoramidites (2'-O-TBDMSi, trade name, Samchully Pharm. Co., Ltd.) was used as RNA amidite. The aforementioned amidite was deprotected by a conventional method, and the synthesized RNA was purified by HPLC. Each RNA after purification was freeze-dried.

The single-stranded nucleic acid molecule and double-stranded nucleic acid molecule (siRNA) of Example 5 were synthesized as mentioned above. In NK-7006 and NK-7007, the parts enclosed with parentheses are linker regions. The underlines in NK-7006, NK-7007, PK-7006, PK-7015, PH-7069, and PH-7081 show expression suppressive sequences of respective target genes, and Lx is a linker region. The linker region Lx having the following structural formula was formed using L-proline diamide amidite.

NkRNA (NK-7006) (target gene: Luciferase)

5'-ACCUACGCCGAGUACUUCGAUUCC(CCACACC)GGAAUCGAAGUACUCGGCGUAGGUUC(UUCG)G-3' (SEQ ID NO: 8)

NkRNA (NK-7007) (target gene: mouse GAPDH)

5'-ACCACGAGAAAUAUGACAACUCCC(CCACACC)GGGAGUUGUCAUAUUUCUCGUGGUUC(UUCG)G-3' (SEQ ID NO: 9)

PnkRNA (PK-7006) (target gene: mouse TGF-β1)
5'-GGAACUCUACCAGAAAUAUAGCCC-Lx-GGGCUAUAUUUCUGGUAGAGUUCCAC-Lx-G-3' (SEQ ID NO: 10)
PnkRNA (PK-7015) (target gene: mouse CCR3)
5'-AGCCUUGUACAGCGAGAUCUUUCC-Lx-GGAAAGAUCUCGCUGUACAAGGCUUC-Lx-G-3' (SEQ ID NO: 11)
PshRNA (PH-7069) (target gene: mouse Smad3)
5'-GGUGCUCCAUCUCCUACUACGACC-Lx-GGUCGUAGUAGGAGAUGGAGCACCA-3' (SEQ ID NO: 12)
antisense nucleic acid (Kynamro-7001) (antisense DNA against ApoB100 mRNA)

5'-GCCUCagtctgcttcGCACC-3' (SEQ ID NO: 1)
(lower case letters show DNA)
PshRNA (PH-7081) (target gene: firefly luciferase)
5'-CUUACGCUGAGUACUUCGAAACC-Lx-GGUUUCGAAGUACUCAGCGUAAGUG-3' (SEQ ID NO: 13)
siRNA (NI-7001) (mouse CTGF)
5'-GUGUGACCAAAAGUUACAUGU-3' (SEQ ID NO: 14)
5'-AUGUAACUUUUGGUCACACUC-3' (SEQ ID NO: 15)
miRNA (NM-7001) (human let7a-1 precursor)

5'-UGGGAUGAGGUAGUAGGUUGUAUAGUUUUAGGGUCACACCCACCACUGGGAGAUAACUAUACAAUCUACUGUCUUUCCUA-3' (SEQ ID NO: 2)

aptamer (Macugen-7001) (aptamer to VEGF protein)
5'-CGGAAUCAGUGAAUGCUUAUACAUCCGt-3' (SEQ ID NO: 3) (t is 3'3'-dT)

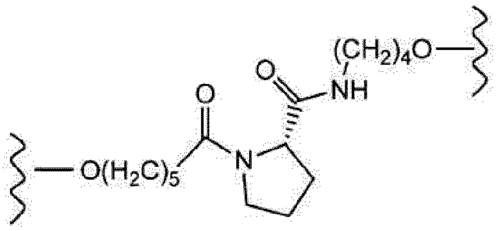

Example 5 (evaluation of stability of various nucleic acids by citrate buffer)

Example 5-1 (preparation of test composition)

The following test compositions 17 - 36 were obtained in the same manner as in Example 3 and test compositions 13, 14.

test composition 17: NK-7006 formulation 44 (0.05 M citrate buffer (pH 6.5)), (0.1 mg/mL)
test composition 18: NK-7006 water for injection, (0.1 mg/mL)
test composition 19: NK-7007 formulation 44 (0.05 M citrate buffer (pH 6.5)), (0.1 mg/mL)
test composition 20: NK-7007 water for injection, (0.1 mg/mL)
test composition 21: PK-7006 formulation 44 (0.05 M citrate buffer (pH 6.5)), (0.1 mg/mL)
test composition 22: PK-7006 water for injection, (0.1 mg/mL)
test composition 23: PK-7015 formulation 44 (0.05 M citrate buffer (pH 6.5)), (0.1 mg/mL)
test composition 24: PK-7015 water for injection, (0.1 mg/mL)
test composition 25: PH-7069 formulation 44 (0.05 M citrate buffer (pH 6.5)), (0.1 mg/mL)
test composition 26: PH-7069 water for injection, (0.1 mg/mL)
test composition 27: Kynamro-7001 formulation 44 (0.05 M citrate buffer (pH 6.5)), (0.1 mg/mL)
test composition 28: Kynamro-7001 water for injection, (0.1 mg/mL)
test composition 29: PH-7081 formulation 44 (0.05 M citrate buffer (pH 6.5)), (0.1 mg/mL)
test composition 30: PH-7081 water for injection, (0.1 mg/mL)
test composition 31: NI-7001 formulation 44 (0.05 M citrate buffer (pH 6.5)), (0.1 mg/mL)
test composition 32: NI-7001 water for injection, (0.1 mg/mL)
test composition 33: NM-7001 formulation 44 (0.05 M citrate buffer (pH 6.5)), (0.1 mg/mL)
test composition 34: NM-7001 water for injection, (0.1 mg/mL)
test composition 35: Macugen-7001 formulation 44 (0.05 M citrate buffer (pH 6.5)), (0.1 mg/mL)
test composition 36: Macugen-7001 water for injection, (0.1 mg/mL)

Example 5-2 (test method and diagnostic criteria)

The test compositions 17 - 36 were stored in a stability test chamber at 60°C. Each stored product was taken

out every week, the content was calculated by reversed-phase HPLC, and the stability was evaluated based on a decrease in the content ratio (%) relative to the content at the time of start of the storage. The storage period and number of products stored are shown in Table 12.

The test compositions 17 - 36 each stored for 1 week, 2 weeks, 3 weeks and 4 weeks at 60°C were used as storage samples.

Separately, the products of test compositions 17 - 36 stored at 4°C were used as calibration curve samples (100%). Each calibration curve sample (100%) was taken by 90 μL, water for injection (10 μL) was added to 100 μL and used as a calibration curve sample (90%). Each calibration curve sample (100%) was taken by 80 μL, water for injection (20 μL) was added to 100 μL and used as a calibration curve sample (80%). Each calibration curve sample (100%) was taken by 70 μL, water for injection (30 μL) was added to 100 μL and used as a calibration curve sample (70%). Each calibration curve sample (100%) was taken by 60 μL, water for injection (40 μL) was added to 100 μL and used as a calibration curve sample (60%). Preparation of the calibration curve samples is shown in Table 13.

Each calibration curve sample (60% - 100%) and each storage sample (each 10 μL) were measured by HPLC. With regard to the peak areas obtained by calibration curve samples (60% - 100%), the regression line (Y=aX+b) and correlation coefficient (r) thereof were determined by the least-squares method with the theoretical content (%) on the horizontal axis (X) and the peak area on the vertical axis (Y), and the content ratio (%) of each sample relative to the content at the time of start of the storage was calculated.

[Table 12]

| storage period and number of products stored | | | |
|---|---|---|---|
| stored sample | | 4°C | 60°C |
| | | time of start | 1 - 4 weeks |
| test substance No. | name | 1 | 4 |
| 17 | NK-7006 formulation 44 | 1 | 4 |
| 18 | NK-7006 water for injection, | 1 | 4 |
| 19 | NK-7007 formulation 44 | 1 | 4 |
| 20 | NK-7007 water for injection | 1 | 4 |
| 21 | PK-7006 formulation 44 | 1 | 4 |
| 22 | PK-7006 water for injection | 1 | 4 |
| 23 | PK-7015 formulation 44 | 1 | 4 |
| 24 | PK-7015 water for injection | 1 | 4 |
| 25 | PH-7069 formulation 44 | 1 | 4 |
| 26 | PH-7069 water for injection | 1 | 4 |
| 27 | Kynamro-7001 formulation 44 | 1 | 4 |
| 28 | Kynamro-7001 water for injection | 1 | 4 |
| 29 | PH-7081 formulation 44 | 1 | 4 |
| 30 | PH-7081 water for injection | 1 | 4 |
| 31 | NI-7001 formulation 44 | 1 | 4 |
| 32 | NI-7001 water for injection | 1 | 4 |
| 33 | NM-0001 formulation 44 | 1 | 4 |
| 34 | NM-0001 water for injection | 1 | 4 |
| 35 | Macugen-7001 formulation 44 | 1 | 4 |
| 36 | Macugen-7001 water for injection | 1 | 4 |

[Table 13]

| analytical curve sample preparation | | | | | |
|---|---|---|---|---|---|
| | each analytical curve sample (60% - 100%) | | | | |
| | 100% | 90% | 80% | 70% | 60% |
| preparation method | test substance | test substance 90 μL + water for injection 10 μL | test substance 80 μL + water for injection 20 μL | test substance 70 μL + water for injection 30 μL | test substance 60 μL + water for injection 40 μL |

Measurement method

The calibration curve samples (60% - 100%) and respective samples were measured under the following measurement conditions.

detector: ultraviolet absorptiometer (measurement wavelength: 254 nm)
column: X-Bridge OST C18 (2.5 μm, 4.6×50 mm)
column temperature: 40°C
mobile phase A: 50 mM TEAA (pH 7.0), 0.5% Acetonitrile
mobile phase B: 100% Acetonitrile
mobile phase feed: The mixing ratio of mobile phase A and
mobile phase B was changed as follows to control concentration gradient.

[Table 14]

| time after injection (min) | mobile phase A (vol%) | mobile phase B (vol%) |
|---|---|---|
| 0→12 | 100→60 | 0→40 |
| flow: 1.0 mL/min | | |

Example 5-3 (results)

The results are shown in Figs. 7 - 16.

The results show that a formulation (pH 6.5) of 0.05 M citrate buffer contributes to the thermal stability, irrespective of the kind of the nucleic acid, as compared to water for injection (WFI).

Production Example 3

As the nucleic acid molecule of Example 6, the strand nucleic acid molecule shown below is synthesized by a nucleic acid synthesizer (trade name: ABI Expedite (registered trademark) 8909 Nucleic Acid Synthesis System, Applied Biosystems) based on the phosphoramidite method. For the aforementioned synthesis, RNA Phosphoramidites (2'-O-TBDMSi, trade name, Samchully Pharm. Co., Ltd.) is used as RNA amidite. The aforementioned amidite is deprotected by a conventional method, and the synthesized RNA is purified by HPLC. Each RNA after purification is freeze-dried.

The single-stranded nucleic acid molecule and doublestranded nucleic acid molecule (siRNA) of Example 6 are synthesized as mentioned above. In NK-7006 and NK-7007, the parts enclosed with parentheses are linker regions. The underlines in NK-7006, NK-7007, PK-7006, PK-7015, PH-7069, and PH-7081 show expression suppressive sequences of respective target genes, and Lx is a linker region. The linker region Lx having the following structural formula is formed using L-proline diamide amidite.

NkRNA (NK-7006) (target gene: Luciferase)

5'-ACCUACGCCGAGUACUUCGAUUCC(CCACACC)GGAAUCGAAGUACUCGGCGUAGGUUC(UUCG)G-3' (SEQ ID NO: 8)

NkRNA (NK-7007) (target gene: mouse GAPDH)

5'-ACCACGAGAAAUAUGACAACUCCC(CCACACC)GGGAGUUGUCAUAUUUCUCGUGGUUC(UUCG)G-3' (SEQ ID NO: 9)

PnkRNA (PK-7006) (target gene: mouse TGF-β1)
5'-GGAACUCUACCAGAAAUAUAGCCC-Lx-GGGCUAUAUUUCUGGUAGAGUUCCAC-Lx-G-3' (SEQ ID NO: 10)
PnkRNA (PK-7015) (target gene: mouse CCR3)
5'-AGCCUUGUACAGCGAGAUCUUUCC-Lx-GGAAAGAUCUCGCUGUACAAGGCUUC-Lx-G-3' (SEQ ID NO: 11)
PshRNA (PH-7069) (target gene: mouse Smad3)
5'-GGUGCUCCAUCUCCUACUACGACC-Lx-GGUCGUAGUAGGAGAUGGAGCACCA-3' (SEQ ID NO: 12)
antisense nucleic acid (Kynamro-7001) (antisense DNA against ApoB100 mRNA)
5'-GCCUCagtctgcttcGCACC-3' (SEQ ID NO: 1)
(lower case letters show DNA)
PshRNA (PH-7081) (target gene: firefly luciferase)
5'-CUUACGCUGAGUACUUCGAAACC-Lx-GGUUUCGAAGUACUCAGCGUAAGUG-3' (SEQ ID NO: 13)
siRNA (NI-7001) (mouse CTGF)
5'-GUGUGACCAAAAGUUACAUGU-3' (SEQ ID NO: 14)
5'-AUGUAACUUUUGGUCACACUC-3' (SEQ ID NO: 15)
miRNA (NM-7001) (human let7a-1 precursor)

5'-UGGGAUGAGGUAGUAGGUUGUAUAGUUUUAGGGUCACACCCACCACUGGGAGAUAACUAUACAAUCUACUGUCUUUCCUA-3' (SEQ ID NO: 2)

aptamer (Macugen-7001) (aptamer to VEGF protein)
5'-CGGAAUCAGUGAAUGCUUAUACAUCCGt-3' (SEQ ID NO: 3) (t is 3'3'-dT)

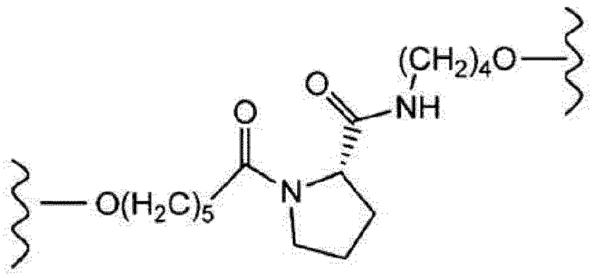

Example 6 (evaluation of stability of various nucleic acids by phosphate buffer)

Example 6-1 (preparation of test composition)

The following test compositions 37 - 56 are prepared as follows.

0.1 M phosphate buffer (pH 6.5) is prepared by mixing sodium dihydrogen phosphate dihydrate (13.006 g) and disodium hydrogen phosphate dodecahydrate (6.017 g), and diluting same to 1 L.

Nucleic acid (0.1 g) is dissolved in water for injection (500 mL). Thereto is added 0.1 M phosphate buffer (pH 6.5) (500 mL) and the mixture is stirred and passed through a 0.22 μm polyvinylidene difluoride (PVDF) filter to give 0.1 g/L (0.1 mg/mL) nucleic acid-containing composition.

test composition 37: NK-7006 formulation 44 (0.05 M phosphate buffer (pH 6.5)), (0.1 mg/mL)
test composition 38: NK-7006 water for injection, (0.1 mg/mL)
test composition 39: NK-7007 formulation 44 (0.05 M phosphate buffer (pH 6.5)), (0.1 mg/mL)
test composition 40: NK-7007 water for injection, (0.1 mg/mL)

test composition 41: PK-7006 formulation 44 (0.05 M phosphate buffer (pH 6.5)), (0.1 mg/mL)
test composition 42: PK-7006 water for injection, (0.1 mg/mL)
test composition 43: PK-7015 formulation 44 (0.05 M phosphate buffer (pH 6.5)), (0.1 mg/mL)
test composition 44: PK-7015 water for injection, (0.1 mg/mL)
test composition 45: PH-7069 formulation 44 (0.05 M phosphate buffer (pH 6.5)), (0.1 mg/mL)
test composition 46: PH-7069 water for injection, (0.1 mg/mL)
test composition 47: Kynamro-7001 formulation 44 (0.05 M phosphate buffer (pH 6.5)), (0.1 mg/mL)
test composition 48: Kynamro-7001 water for injection, (0.1 mg/mL)
test composition 49: PH-7081 formulation 44 (0.05 M phosphate buffer (pH 6.5)), (0.1 mg/mL)
test composition 50: PH-7081 water for injection, (0.1 mg/mL)
test composition 51: NI-7001 formulation 44 (0.05 M phosphate buffer (pH 6.5)), (0.1 mg/mL)
test composition 52: NI-7001 water for injection, (0.1 mg/mL)
test composition 53: NM-7001 formulation 44 (0.05 M phosphate buffer (pH 6.5)), (0.1 mg/mL)
test composition 54: NM-7001 water for injection, (0.1 mg/mL)
test composition 55: Macugen-7001 formulation 44 (0.05 M phosphate buffer (pH 6.5)), (0.1 mg/mL)
test composition 56: Macugen-7001 water for injection, (0.1 mg/mL)

Example 6-2 (test method and diagnostic criteria)

The test compositions 37 - 56 are stored in a stability test chamber at 60°C. Each stored product is taken out every week, the content is calculated by reversed-phase HPLC, and the stability is evaluated based on a decrease in the content ratio (%) relative to the content at the time of start of the storage. The storage period and number of products stored are shown in Table.

The test compositions 37 - 56 each stored for 1 week, 2 weeks, 3 weeks and 4 weeks at 60°C are used as storage samples.

Separately, the products of test compositions 17 - 36 stored at 4°C are used as calibration curve samples (100%). Each calibration curve sample (100%) is taken by 90 μL, water for injection (10 μL) is added to 100 μL and used as a calibration curve sample (90%). Each calibration curve sample (100%) is taken by 80 μL, water for injection (20 μL) is added to 100 μL and used as a calibration curve sample (80%). Each calibration curve sample (100%) is taken by 70 μL, water for injection (30 μL) is added to 100 μL and used as a calibration curve sample (70%). Each calibration curve sample (100%) is taken by 60 μL, water for injection (40 μL) is added to 100 μL and used as a calibration curve sample (60%).

Each calibration curve sample (60% - 100%) and each storage sample (each 10 μL) are measured by HPLC. With regard to the peak areas obtained by calibration curve samples (60% - 100%), the regression line (Y=aX+b) and correlation coefficient (r) thereof are determined by the least-squares method with the theoretical content (%) on the horizontal axis (X) and the peak area on the vertical axis (Y), and the content ratio (%) of each sample relative to the content at the time of start of the storage is calculated (excel 2013).

[Table 15]

| Table 15 storage period and number of products stored | | | |
|---|---|---|---|
| stored sample | | 4°C | 60°C |
| | | time of start | 1 - 4 weeks |
| test substance No. | name | 1 | 4 |
| 37 | NK-7006 formulation 44 | 1 | 4 |
| 38 | NK-7006 water for injection, | 1 | 4 |
| 39 | NK-7007 formulation 44 | 1 | 4 |
| 40 | NK-7007 water for injection | 1 | 4 |
| 41 | PK-7006 formulation 44 | 1 | 4 |
| 42 | PK-7006 water for injection | 1 | 4 |
| 43 | PK-7015 formulation 44 | 1 | 4 |
| 44 | PK-7015 water for injection | 1 | 4 |
| 45 | PH-7069 formulation 44 | 1 | 4 |

(continued)

| Table 15 storage period and number of products stored | | | |
|---|---|---|---|
| stored sample | | 4°C | 60°C |
| | | time of start | 1 - 4 weeks |
| test substance No. | name | 1 | 4 |
| 46 | PH-7069 water for injection | 1 | 4 |
| 47 | Kynamro-7001 formulation 44 | 1 | 4 |
| 48 | Kynamro-7001 water for injection | 1 | 4 |
| 49 | PH-7081 formulation 44 | 1 | 4 |
| 50 | PH-7081 water for injection | 1 | 4 |
| 51 | NI-7001 formulation 44 | 1 | 4 |
| 52 | NI-7001 water for injection | 1 | 4 |
| 53 | NM-0001 formulation 44 | 1 | 4 |
| 54 | NM-0001 water for injection | 1 | 4 |
| 55 | Macugen-7001 formulation 44 | 1 | 4 |
| 56 | Macugen-7001 water for injection | 1 | 4 |

Measurement method

The calibration curve samples (60% - 100%) and respective samples are measured under the following measurement conditions.

detector: ultraviolet absorptiometer (measurement wavelength: 254 nm)

column: X-Bridge OST C18 (2.5 μm, 4.6×50 mm)

column temperature: 40°C

mobile phase A: 50 mM TEAA (pH 7.0), 0.5% Acetonitrile

mobile phase B: 100% Acetonitrile

mobile phase feed: The mixing ratio of mobile phase A and

mobile phase B is changed as follows to control concentration gradient.

[Table 16]

| time after injection (min) | mobile phase A (vol%) | mobile phase B (vol%) |
|---|---|---|
| 0→12 | 100→60 | 0→40 |
| flow: 1.0 mL/min | | |

<u>Production Example 4</u>

As the nucleic acid molecules of Example 7, PK-7006, NK-7006, PH-7069, NI-7001, NM-7001, Kynamro-7001 and Macugen-7001 were synthesized by a method similar to that in Production Example 2.

Example 7 (evaluation of stability of various nucleic acids by citrate buffer and/or phosphate buffer)

Example 7-1 (preparation of test composition)

The test compositions 57 - 74, 105 - 122, 153 - 170 were prepared as follows.
0.1 M aqueous citric acid solution and 0.1 M trisodium citrate dihydrate solution were mixed and adjusted to pH4.0 - 8.0 to give 0.1 M citrate buffer at each pH.
A 25 mg/mL test composition (0.02 mL) prepared with water for injection, water for injection (2.48 mL), and 0.1 M citrate buffer (2.50 mL) at each pH were mixed to give 5 mL each of 0.1 mg/mL test composition.
The test compositions 201 - 218 were prepared as follows.
By a method similar to the above, 0.1 M citrate buffer was prepared. A 10 mg/mL test composition (0.05 mL) prepared with water for injection, water for injection (2.45 mL), and 0.1 M citrate buffer (2.50 mL) at each pH were mixed to give 5 mL each of 0.1 mg/mL test composition.
The test compositions 249 - 266, 297 - 314, 345 - 362 were prepared as follows.
By a method similar to the above, 0.1 M citrate buffer was prepared. A 20 mg/mL test composition (0.025 mL) prepared with water for injection, water for injection (2.475 mL), and 0.1 M citrate buffer (2.50 mL) at each pH were mixed to give 5 mL each of 0.1 mg/mL test composition.
The test compositions 75 - 95, 123 - 143, 171 - 191 were prepared as follows.
0.1 M aqueous sodium dihydrogen phosphate solution and 0.1 M disodium hydrogen phosphate solution were mixed and adjusted to pH4.0 - 8.0 to give 0.1 M phosphate buffer at each pH.
A 25 mg/mL test composition (0.02 mL) prepared with water for injection, water for injection (2.48 mL), and 0.1 M phosphate buffer (2.50 mL) at each pH were mixed to give 5 mL each of 0.1 mg/mL test composition.
The test compositions 219 - 239 were prepared as follows.
By a method similar to the above, 0.1 M phosphate buffer was prepared. A 10 mg/mL test composition (0.05 mL) prepared with water for injection, water for injection (2.45 mL), and 0.1 M phosphate buffer (2.50 mL) at each pH were mixed to give 5 mL each of 0.1 mg/mL test composition.
The test compositions 267 - 287, 315 - 335, 363 - 383 were prepared as follows.
By a method similar to the above, 0.1 M phosphate buffer was prepared. A 20 mg/mL test composition (0.025 mL) prepared with water for injection, water for injection (2.475 mL), and 0.1 M phosphate buffer (2.50 mL) at each pH were mixed to give 5 mL each of 0.1 mg/mL test composition.
The test compositions 96 - 104, 144 - 152, 192 - 200 were prepared as follows.
0.1 M aqueous sodium citrate solution and 0.1 M aqueous citric acid solution were mixed and adjusted to pH4.2 - 7.6 to give 0.1 M citrate buffer at each pH. Similarly, 0.1 M aqueous sodium dihydrogen phosphate solution and 0.1 M disodium hydrogen phosphate solution were mixed and adjusted to pH4.2 - 7.6 to give 0.1 M phosphate buffer at each pH. 0.1 M citrate buffer and 0.1 M phosphate buffer at the same pH were mixed at 5:5 (3 mL:3 mL) to give 0.1 M citrate-phosphate buffer (5:5) at each pH.
A 25 mg/mL test composition (0.02 mL) prepared with water for injection, water for injection (2.48 mL), and 0.1 M citrate-phosphate buffer (5:5) (2.50 mL) at each pH were mixed to give 5 mL each of 0.1 mg/mL test composition.
The test compositions 240 - 248 were prepared as follows.
By a method similar to the above, 0.1 M citrate-phosphate buffer (5:5) was prepared. A 10 mg/mL test composition (0.05 mL) prepared with water for injection, water for injection (2.45 mL), and 0.1 M citrate-phosphate buffer (5:5) (2.50 mL) at each pH were mixed to give 5 mL each of 0.1 mg/mL test composition.
The test compositions 288 - 296, 336 - 344, 384 - 392 were prepared as follows.
By a method similar to the above, 0.1 M citrate-phosphate buffer (5:5) was prepared. A 20 mg/mL test composition (0.025 mL) prepared with water for injection, water for injection (2.475 mL), and 0.1 M citrate-phosphate buffer (5:5) (2.50 mL) at each pH were mixed to give 5 mL each of 0.1 mg/mL test composition.
nucleic acid molecule: PK-7006
test composition 57: PK-7006 formulation 199 (0.05 M citrate buffer (pH 4.0)), (0.1 mg/mL)
test composition 58: PK-7006 formulation 200 (0.05 M citrate buffer (pH 4.2)), (0.1 mg/mL)
test composition 59: PK-7006 formulation 201 (0.05 M citrate buffer (pH 4.4)), (0.1 mg/mL)
test composition 60: PK-7006 formulation 202 (0.05 M citrate buffer (pH 4.6)), (0.1 mg/mL)
test composition 61: PK-7006 formulation 203 (0.05 M citrate buffer (pH 4.8)), (0.1 mg/mL)
test composition 62: PK-7006 formulation 204 (0.05 M citrate buffer (pH 5.0)), (0.1 mg/mL)
test composition 63: PK-7006 formulation 205 (0.05 M citrate buffer (pH 5.2)), (0.1 mg/mL)
test composition 64: PK-7006 formulation 206 (0.05 M citrate buffer (pH 5.4)), (0.1 mg/mL)
test composition 65: PK-7006 formulation 207 (0.05 M citrate buffer (pH 5.6)), (0.1 mg/mL)

test composition 66: PK-7006 formulation 208 (0.05 M citrate buffer (pH 5.8)), (0.1 mg/mL)
test composition 67: PK-7006 formulation 209 (0.05 M citrate buffer (pH 6.0)), (0.1 mg/mL)
test composition 68: PK-7006 formulation 210 (0.05 M citrate buffer (pH 6.2)), (0.1 mg/mL)
test composition 69: PK-7006 formulation 211 (0.05 M citrate buffer (pH 6.4)), (0.1 mg/mL)
test composition 70: PK-7006 formulation 212 (0.05 M citrate buffer (pH 6.6)), (0.1 mg/mL)
test composition 71: PK-7006 formulation 213 (0.05 M citrate buffer (pH 6.8)), (0.1 mg/mL)
test composition 72: PK-7006 formulation 214 (0.05 M citrate buffer (pH 7.0)), (0.1 mg/mL)
test composition 73: PK-7006 formulation 215 (0.05 M citrate buffer (pH 7.2)), (0.1 mg/mL)
test composition 74: PK-7006 formulation 216 (0.05 M citrate buffer (pH 7.4)), (0.1 mg/mL)
test composition 75: PK-7006 formulation 217 (0.05 M phosphate buffer (pH 4.0)), (0.1 mg/mL)
test composition 76: PK-7006 formulation 218 (0.05 M phosphate buffer (pH 4.2)), (0.1 mg/mL)
test composition 77: PK-7006 formulation 219 (0.05 M phosphate buffer (pH 4.4)), (0.1 mg/mL)
test composition 78: PK-7006 formulation 220 (0.05 M phosphate buffer (pH 4.6)), (0.1 mg/mL)
test composition 79: PK-7006 formulation 221 (0.05 M phosphate buffer (pH 4.8)), (0.1 mg/mL)
test composition 80: PK-7006 formulation 222 (0.05 M phosphate buffer (pH 5.0)), (0.1 mg/mL)
test composition 81: PK-7006 formulation 223 (0.05 M phosphate buffer (pH 5.2)), (0.1 mg/mL)
test composition 82: PK-7006 formulation 224 (0.05 M phosphate buffer (pH 5.4)), (0.1 mg/mL)
test composition 83: PK-7006 formulation 225 (0.05 M phosphate buffer (pH 5.6)), (0.1 mg/mL)
test composition 84: PK-7006 formulation 226 (0.05 M phosphate buffer (pH 5.8)), (0.1 mg/mL)
test composition 85: PK-7006 formulation 227 (0.05 M phosphate buffer (pH 6.0)), (0.1 mg/mL)
test composition 86: PK-7006 formulation 228 (0.05 M phosphate buffer (pH 6.2)), (0.1 mg/mL)
test composition 87: PK-7006 formulation 229 (0.05 M phosphate buffer (pH 6.4)), (0.1 mg/mL)
test composition 88: PK-7006 formulation 230 (0.05 M phosphate buffer (pH 6.6)), (0.1 mg/mL)
test composition 89: PK-7006 formulation 231 (0.05 M phosphate buffer (pH 6.8)), (0.1 mg/mL)
test composition 90: PK-7006 formulation 232 (0.05 M phosphate buffer (pH 7.0)), (0.1 mg/mL)
test composition 91: PK-7006 formulation 233 (0.05 M phosphate buffer (pH 7.2)), (0.1 mg/mL)
test composition 92: PK-7006 formulation 234 (0.05 M phosphate buffer (pH 7.4)), (0.1 mg/mL)
test composition 93: PK-7006 formulation 235 (0.05 M phosphate buffer (pH 7.6)), (0.1 mg/mL)
test composition 94: PK-7006 formulation 236 (0.05 M phosphate buffer (pH 7.8)), (0.1 mg/mL)
test composition 95: PK-7006 formulation 237 (0.05 M phosphate buffer (pH 8.0)), (0.1 mg/mL)
test composition 96: PK-7006 formulation 238 (0.05 M citrate-phosphate (5:5) buffer (pH 4.2)), (0.1 mg/mL)
test composition 97: PK-7006 formulation 239 (0.05 M citrate-phosphate (5:5) buffer (pH 4.4)), (0.1 mg/mL)
test composition 98: PK-7006 formulation 240 (0.05 M citrate-phosphate (5:5) buffer (pH 4.6)), (0.1 mg/mL)
test composition 99: PK-7006 formulation 241 (0.05 M citrate-phosphate (5:5) buffer (pH 5.0)), (0.1 mg/mL)
test composition 100: PK-7006 formulation 242 (0.05 M citrate-phosphate (5:5) buffer (pH 6.0)), (0.1 mg/mL)
test composition 101: PK-7006 formulation 243 (0.05 M citrate-phosphate (5:5) buffer (pH 6.6)), (0.1 mg/mL)
test composition 102: PK-7006 formulation 244 (0.05 M citrate-phosphate (5:5) buffer (pH 7.0)), (0.1 mg/mL)
test composition 103: PK-7006 formulation 245 (0.05 M citrate-phosphate (5:5) buffer (pH 7.4)), (0.1 mg/mL)
test composition 104: PK-7006 formulation 246 (0.05 M citrate-phosphate (5:5) buffer (pH 7.6)), (0.1 mg/mL)

nucleic acid molecule: NK-7006
test composition 105: NK-7006 formulation 247 (0.05 M citrate buffer (pH 4.0)), (0.1 mg/mL)
test composition 106: NK-7006 formulation 248 (0.05 M citrate buffer (pH 4.2)), (0.1 mg/mL)
test composition 107: NK-7006 formulation 249 (0.05 M citrate buffer (pH 4.4)), (0.1 mg/mL)
test composition 108: NK-7006 formulation 250 (0.05 M citrate buffer (pH 4.6)), (0.1 mg/mL)
test composition 109: NK-7006 formulation 251 (0.05 M citrate buffer (pH 4.8)), (0.1 mg/mL)
test composition 110: NK-7006 formulation 252 (0.05 M citrate buffer (pH 5.0)), (0.1 mg/mL)
test composition 111: NK-7006 formulation 253 (0.05 M citrate buffer (pH 5.2)), (0.1 mg/mL)
test composition 112: NK-7006 formulation 254 (0.05 M citrate buffer (pH 5.4)), (0.1 mg/mL)
test composition 113: NK-7006 formulation 255 (0.05 M citrate buffer (pH 5.6)), (0.1 mg/mL)
test composition 114: NK-7006 formulation 256 (0.05 M citrate buffer (pH 5.8)), (0.1 mg/mL)
test composition 115: NK-7006 formulation 257 (0.05 M citrate buffer (pH 6.0)), (0.1 mg/mL)
test composition 116: NK-7006 formulation 258 (0.05 M citrate buffer (pH 6.2)), (0.1 mg/mL)
test composition 117: NK-7006 formulation 259 (0.05 M citrate buffer (pH 6.4)), (0.1 mg/mL)
test composition 118: NK-7006 formulation 260 (0.05 M citrate buffer (pH 6.6)), (0.1 mg/mL)
test composition 119: NK-7006 formulation 261 (0.05 M citrate buffer (pH 6.8)), (0.1 mg/mL)

test composition 120: NK-7006 formulation 262 (0.05 M citrate buffer (pH 7.0)), (0.1 mg/mL)
test composition 121: NK-7006 formulation 263 (0.05 M citrate buffer (pH 7.2)), (0.1 mg/mL)
test composition 122: NK-7006 formulation 264 (0.05 M citrate buffer (pH 7.4)), (0.1 mg/mL)
test composition 123: NK-7006 formulation 265 (0.05 M phosphate buffer (pH 4.0)), (0.1 mg/mL)
test composition 124: NK-7006 formulation 266 (0.05 M phosphate buffer (pH 4.2)), (0.1 mg/mL)
test composition 125: NK-7006 formulation 267 (0.05 M phosphate buffer (pH 4.4)), (0.1 mg/mL)
test composition 126: NK-7006 formulation 268 (0.05 M phosphate buffer (pH 4.6)), (0.1 mg/mL)
test composition 127: NK-7006 formulation 269 (0.05 M phosphate buffer (pH 4.8)), (0.1 mg/mL)
test composition 128: NK-7006 formulation 270 (0.05 M phosphate buffer (pH 5.0)), (0.1 mg/mL)
test composition 129: NK-7006 formulation 271 (0.05 M phosphate buffer (pH 5.2)), (0.1 mg/mL)
test composition 130: NK-7006 formulation 272 (0.05 M phosphate buffer (pH 5.4)), (0.1 mg/mL)
test composition 131: NK-7006 formulation 273 (0.05 M phosphate buffer (pH 5.6)), (0.1 mg/mL)
test composition 132: NK-7006 formulation 274 (0.05 M phosphate buffer (pH 5.8)), (0.1 mg/mL)
test composition 133: NK-7006 formulation 275 (0.05 M phosphate buffer (pH 6.0)), (0.1 mg/mL)
test composition 134: NK-7006 formulation 276 (0.05 M phosphate buffer (pH 6.2)), (0.1 mg/mL)
test composition 135: NK-7006 formulation 277 (0.05 M phosphate buffer (pH 6.4)), (0.1 mg/mL)
test composition 136: NK-7006 formulation 278 (0.05 M phosphate buffer (pH 6.6)), (0.1 mg/mL)
test composition 137: NK-7006 formulation 279 (0.05 M phosphate buffer (pH 6.8)), (0.1 mg/mL)
test composition 138: NK-7006 formulation 280 (0.05 M phosphate buffer (pH 7.0)), (0.1 mg/mL)
test composition 139: NK-7006 formulation 281 (0.05 M phosphate buffer (pH 7.2)), (0.1 mg/mL)
test composition 140: NK-7006 formulation 282 (0.05 M phosphate buffer (pH 7.4)), (0.1 mg/mL)
test composition 141: NK-7006 formulation 283 (0.05 M phosphate buffer (pH 7.6)), (0.1 mg/mL)
test composition 142: NK-7006 formulation 284 (0.05 M phosphate buffer (pH 7.8)), (0.1 mg/mL)
test composition 143: NK-7006 formulation 285 (0.05 M phosphate buffer (pH 8.0)), (0.1 mg/mL)
test composition 144: NK-7006 formulation 286 (0.05 M citrate-phosphate (5:5) buffer (pH 4.2)), (0.1 mg/mL)
test composition 145: NK-7006 formulation 287 (0.05 M citrate-phosphate (5:5) buffer (pH 4.4)), (0.1 mg/mL)
test composition 146: NK-7006 formulation 288 (0.05 M citrate-phosphate (5:5) buffer (pH 4.6)), (0.1 mg/mL)
test composition 147: NK-7006 formulation 289 (0.05 M citrate-phosphate (5:5) buffer (pH 5.0)), (0.1 mg/mL)
test composition 148: NK-7006 formulation 290 (0.05 M citrate-phosphate (5:5) buffer (pH 6.0)), (0.1 mg/mL)
test composition 149: NK-7006 formulation 291 (0.05 M citrate-phosphate (5:5) buffer (pH 6.6)), (0.1 mg/mL)
test composition 150: NK-7006 formulation 292 (0.05 M citrate-phosphate (5:5) buffer (pH 7.0)), (0.1 mg/mL)
test composition 151: NK-7006 formulation 293 (0.05 M citrate-phosphate (5:5) buffer (pH 7.4)), (0.1 mg/mL)
test composition 152: NK-7006 formulation 294 (0.05 M citrate-phosphate (5:5) buffer (pH 7.6)), (0.1 mg/mL)

nucleic acid molecule: PH-7069
test composition 153: PH-7069 formulation 295 (0.05 M citrate buffer (pH 4.0)), (0.1 mg/mL)
test composition 154: PH-7069 formulation 296 (0.05 M citrate buffer (pH 4.2)), (0.1 mg/mL)
test composition 155: PH-7069 formulation 297 (0.05 M citrate buffer (pH 4.4)), (0.1 mg/mL)
test composition 156: PH-7069 formulation 298 (0.05 M citrate buffer (pH 4.6)), (0.1 mg/mL)
test composition 157: PH-7069 formulation 299 (0.05 M citrate buffer (pH 4.8)), (0.1 mg/mL)
test composition 158: PH-7069 formulation 300 (0.05 M citrate buffer (pH 5.0)), (0.1 mg/mL)
test composition 159: PH-7069 formulation 301 (0.05 M citrate buffer (pH 5.2)), (0.1 mg/mL)
test composition 160: PH-7069 formulation 302 (0.05 M citrate buffer (pH 5.4)), (0.1 mg/mL)
test composition 161: PH-7069 formulation 303 (0.05 M citrate buffer (pH 5.6)), (0.1 mg/mL)
test composition 162: PH-7069 formulation 304 (0.05 M citrate buffer (pH 5.8)), (0.1 mg/mL)
test composition 163: PH-7069 formulation 305 (0.05 M citrate buffer (pH 6.0)), (0.1 mg/mL)
test composition 164: PH-7069 formulation 306 (0.05 M citrate buffer (pH 6.2)), (0.1 mg/mL)
test composition 165: PH-7069 formulation 307 (0.05 M citrate buffer (pH 6.4)), (0.1 mg/mL)
test composition 166: PH-7069 formulation 308 (0.05 M citrate buffer (pH 6.6)), (0.1 mg/mL)
test composition 167: PH-7069 formulation 309 (0.05 M citrate buffer (pH 6.8)), (0.1 mg/mL)
test composition 168: PH-7069 formulation 310 (0.05 M citrate buffer (pH 7.0)), (0.1 mg/mL)
test composition 169: PH-7069 formulation 311 (0.05 M citrate buffer (pH 7.2)), (0.1 mg/mL)
test composition 170: PH-7069 formulation 312 (0.05 M citrate buffer (pH 7.4)), (0.1 mg/mL)
test composition 171: PH-7069 formulation 313 (0.05 M phosphate buffer (pH 4.0)), (0.1 mg/mL)
test composition 172: PH-7069 formulation 314 (0.05 M phosphate buffer (pH 4.2)), (0.1 mg/mL)
test composition 173: PH-7069 formulation 315 (0.05 M phosphate buffer (pH 4.4)), (0.1 mg/mL)

test composition 174: PH-7069 formulation 316 (0.05 M phosphate buffer (pH 4.6)), (0.1 mg/mL)
test composition 175: PH-7069 formulation 317 (0.05 M phosphate buffer (pH 4.8)), (0.1 mg/mL)
test composition 176: PH-7069 formulation 318 (0.05 M phosphate buffer (pH 5.0)), (0.1 mg/mL)
test composition 177: PH-7069 formulation 319 (0.05 M phosphate buffer (pH 5.2)), (0.1 mg/mL)
test composition 178: PH-7069 formulation 320 (0.05 M phosphate buffer (pH 5.4)), (0.1 mg/mL)
test composition 179: PH-7069 formulation 321 (0.05 M phosphate buffer (pH 5.6)), (0.1 mg/mL)
test composition 180: PH-7069 formulation 322 (0.05 M phosphate buffer (pH 5.8)), (0.1 mg/mL)
test composition 181: PH-7069 formulation 323 (0.05 M phosphate buffer (pH 6.0)), (0.1 mg/mL)
test composition 182: PH-7069 formulation 324 (0.05 M phosphate buffer (pH 6.2)), (0.1 mg/mL)
test composition 183: PH-7069 formulation 325 (0.05 M phosphate buffer (pH 6.4)), (0.1 mg/mL)
test composition 184: PH-7069 formulation 326 (0.05 M phosphate buffer (pH 6.6)), (0.1 mg/mL)
test composition 185: PH-7069 formulation 327 (0.05 M phosphate buffer (pH 6.8)), (0.1 mg/mL)
test composition 186: PH-7069 formulation 328 (0.05 M phosphate buffer (pH 7.0)), (0.1 mg/mL)
test composition 187: PH-7069 formulation 329 (0.05 M phosphate buffer (pH 7.2)), (0.1 mg/mL)
test composition 188: PH-7069 formulation 330 (0.05 M phosphate buffer (pH 7.4)), (0.1 mg/mL)
test composition 189: PH-7069 formulation 331 (0.05 M phosphate buffer (pH 7.6)), (0.1 mg/mL)
test composition 190: PH-7069 formulation 332 (0.05 M phosphate buffer (pH 7.8)), (0.1 mg/mL)
test composition 191: PH-7069 formulation 333 (0.05 M phosphate buffer (pH 8.0)), (0.1 mg/mL)
test composition 192: PH-7069 formulation 334 (0.05 M citrate-phosphate (5:5) buffer (pH 4.2)), (0.1 mg/mL)
test composition 193: PH-7069 formulation 335 (0.05 M citrate-phosphate (5:5) buffer (pH 4.4)), (0.1 mg/mL)
test composition 194: PH-7069 formulation 336 (0.05 M citrate-phosphate (5:5) buffer (pH 4.6)), (0.1 mg/mL)
test composition 195: PH-7069 formulation 337 (0.05 M citrate-phosphate (5:5) buffer (pH 5.0)), (0.1 mg/mL)
test composition 196: PH-7069 formulation 338 (0.05 M citrate-phosphate (5:5) buffer (pH 6.0)), (0.1 mg/mL)
test composition 197: PH-7069 formulation 339 (0.05 M citrate-phosphate (5:5) buffer (pH 6.6)), (0.1 mg/mL)
test composition 198: PH-7069 formulation 340 (0.05 M citrate-phosphate (5:5) buffer (pH 7.0)), (0.1 mg/mL)
test composition 199: PH-7069 formulation 341 (0.05 M citrate-phosphate (5:5) buffer (pH 7.4)), (0.1 mg/mL)
test composition 200: PH-7069 formulation 342 (0.05 M citrate-phosphate (5:5) buffer (pH 7.6)), (0.1 mg/mL)

nucleic acid molecule: NI-7001
test composition 201: NI-7001 formulation 343 (0.05 M citrate buffer (pH 4.0)), (0.1 mg/mL)
test composition 202: NI-7001 formulation 344 (0.05 M citrate buffer (pH 4.2)), (0.1 mg/mL)
test composition 203: NI-7001 formulation 345 (0.05 M citrate buffer (pH 4.4)), (0.1 mg/mL)
test composition 204: NI-7001 formulation 346 (0.05 M citrate buffer (pH 4.6)), (0.1 mg/mL)
test composition 205: NI-7001 formulation 347 (0.05 M citrate buffer (pH 4.8)), (0.1 mg/mL)
test composition 206: NI-7001 formulation 348 (0.05 M citrate buffer (pH 5.0)), (0.1 mg/mL)
test composition 207: NI-7001 formulation 349 (0.05 M citrate buffer (pH 5.2)), (0.1 mg/mL)
test composition 208: NI-7001 formulation 350 (0.05 M citrate buffer (pH 5.4)), (0.1 mg/mL)
test composition 209: NI-7001 formulation 351 (0.05 M citrate buffer (pH 5.6)), (0.1 mg/mL)
test composition 210: NI-7001 formulation 352 (0.05 M citrate buffer (pH 5.8)), (0.1 mg/mL)
test composition 211: NI-7001 formulation 353 (0.05 M citrate buffer (pH 6.0)), (0.1 mg/mL)
test composition 212: NI-7001 formulation 354 (0.05 M citrate buffer (pH 6.2)), (0.1 mg/mL)
test composition 213: NI-7001 formulation 355 (0.05 M citrate buffer (pH 6.4)), (0.1 mg/mL)
test composition 214: NI-7001 formulation 356 (0.05 M citrate buffer (pH 6.6)), (0.1 mg/mL)
test composition 215: NI-7001 formulation 357 (0.05 M citrate buffer (pH 6.8)), (0.1 mg/mL)
test composition 216: NI-7001 formulation 358 (0.05 M citrate buffer (pH 7.0)), (0.1 mg/mL)
test composition 217: NI-7001 formulation 359 (0.05 M citrate buffer (pH 7.2)), (0.1 mg/mL)
test composition 218: NI-7001 formulation 360 (0.05 M citrate buffer (pH 7.4)), (0.1 mg/mL)
test composition 219: NI-7001 formulation 361 (0.05 M phosphate buffer (pH 4.0)), (0.1 mg/mL)
test composition 220: NI-7001 formulation 362 (0.05 M phosphate buffer (pH 4.2)), (0.1 mg/mL)
test composition 221: NI-7001 formulation 363 (0.05 M phosphate buffer (pH 4.4)), (0.1 mg/mL)
test composition 222: NI-7001 formulation 364 (0.05 M phosphate buffer (pH 4.6)), (0.1 mg/mL)
test composition 2223: NI-7001 formulation 365 (0.05 M phosphate buffer (pH 4.8)), (0.1 mg/mL)
test composition 224: NI-7001 formulation 366 (0.05 M phosphate buffer (pH 5.0)), (0.1 mg/mL)
test composition 225: NI-7001 formulation 367 (0.05 M phosphate buffer (pH 5.2)), (0.1 mg/mL)
test composition 226: NI-7001 formulation 368 (0.05 M phosphate buffer (pH 5.4)), (0.1 mg/mL)
test composition 227: NI-7001 formulation 369 (0.05 M phosphate buffer (pH 5.6)), (0.1 mg/mL)

test composition 228: NI-7001 formulation 370 (0.05 M phosphate buffer (pH 5.8)), (0.1 mg/mL)
test composition 229: NI-7001 formulation 371 (0.05 M phosphate buffer (pH 6.0)), (0.1 mg/mL)
test composition 230: NI-7001 formulation 372 (0.05 M phosphate buffer (pH 6.2)), (0.1 mg/mL)
test composition 231: NI-7001 formulation 373 (0.05 M phosphate buffer (pH 6.4)), (0.1 mg/mL)
test composition 232: NI-7001 formulation 374 (0.05 M phosphate buffer (pH 6.6)), (0.1 mg/mL)
test composition 233: NI-7001 formulation 375 (0.05 M phosphate buffer (pH 6.8)), (0.1 mg/mL)
test composition 234: NI-7001 formulation 376 (0.05 M phosphate buffer (pH 7.0)), (0.1 mg/mL)
test composition 235: NI-7001 formulation 377 (0.05 M phosphate buffer (pH 7.2)), (0.1 mg/mL)
test composition 236: NI-7001 formulation 378 (0.05 M phosphate buffer (pH 7.4)), (0.1 mg/mL)
test composition 237: NI-7001 formulation 379 (0.05 M phosphate buffer (pH 7.6)), (0.1 mg/mL)
test composition 238: NI-7001 formulation 380 (0.05 M phosphate buffer (pH 7.8)), (0.1 mg/mL)
test composition 239: NI-7001 formulation 381 (0.05 M phosphate buffer (pH 8.0)), (0.1 mg/mL)
test composition 240: NI-7001 formulation 382 (0.05 M citrate-phosphate (5:5) buffer (pH 4.2)), (0.1 mg/mL)
test composition 241: NI-7001 formulation 383 (0.05 M citrate-phosphate (5:5) buffer (pH 4.4)), (0.1 mg/mL)
test composition 242: NI-7001 formulation 384 (0.05 M citrate-phosphate (5:5) buffer (pH 4.6)), (0.1 mg/mL)
test composition 243: NI-7001 formulation 385 (0.05 M citrate-phosphate (5:5) buffer (pH 5.0)), (0.1 mg/mL)
test composition 244: NI-7001 formulation 386 (0.05 M citrate-phosphate (5:5) buffer (pH 6.0)), (0.1 mg/mL)
test composition 245: NI-7001 formulation 387 (0.05 M citrate-phosphate (5:5) buffer (pH 6.6)), (0.1 mg/mL)
test composition 246: NI-7001 formulation 388 (0.05 M citrate-phosphate (5:5) buffer (pH 7.0)), (0.1 mg/mL)
test composition 247: NI-7001 formulation 389 (0.05 M citrate-phosphate (5:5) buffer (pH 7.4)), (0.1 mg/mL)
test composition 248: NI-7001 formulation 390 (0.05 M citrate-phosphate (5:5) buffer (pH 7.6)), (0.1 mg/mL)

nucleic acid molecule: NM-7001
test composition 249: NM-7001 formulation 391 (0.05 M citrate buffer (pH 4.0)), (0.1 mg/mL)
test composition 250: NM-7001 formulation 392 (0.05 M citrate buffer (pH 4.2)), (0.1 mg/mL)
test composition 251: NM-7001 formulation 393 (0.05 M citrate buffer (pH 4.4)), (0.1 mg/mL)
test composition 252: NM-7001 formulation 394 (0.05 M citrate buffer (pH 4.6)), (0.1 mg/mL)
test composition 253: NM-7001 formulation 395 (0.05 M citrate buffer (pH 4.8)), (0.1 mg/mL)
test composition 254: NM-7001 formulation 396 (0.05 M citrate buffer (pH 5.0)), (0.1 mg/mL)
test composition 255: NM-7001 formulation 397 (0.05 M citrate buffer (pH 5.2)), (0.1 mg/mL)
test composition 256: NM-7001 formulation 398 (0.05 M citrate buffer (pH 5.4)), (0.1 mg/mL)
test composition 257: NM-7001 formulation 399 (0.05 M citrate buffer (pH 5.6)), (0.1 mg/mL)
test composition 258: NM-7001 formulation 400 (0.05 M citrate buffer (pH 5.8)), (0.1 mg/mL)
test composition 259: NM-7001 formulation 401 (0.05 M citrate buffer (pH 6.0)), (0.1 mg/mL)
test composition 260: NM-7001 formulation 402 (0.05 M citrate buffer (pH 6.2)), (0.1 mg/mL)
test composition 261: NM-7001 formulation 403 (0.05 M citrate buffer (pH 6.4)), (0.1 mg/mL)
test composition 262: NM-7001 formulation 404 (0.05 M citrate buffer (pH 6.6)), (0.1 mg/mL)
test composition 263: NM-7001 formulation 405 (0.05 M citrate buffer (pH 6.8)), (0.1 mg/mL)
test composition 264: NM-7001 formulation 406 (0.05 M citrate buffer (pH 7.0)), (0.1 mg/mL)
test composition 265: NM-7001 formulation 407 (0.05 M citrate buffer (pH 7.2)), (0.1 mg/mL)
test composition 266: NM-7001 formulation 408 (0.05 M citrate buffer (pH 7.4)), (0.1 mg/mL)
test composition 267: NM-7001 formulation 409 (0.05 M phosphate buffer (pH 4.0)), (0.1 mg/mL)
test composition 268: NM-7001 formulation 410 (0.05 M phosphate buffer (pH 4.2)), (0.1 mg/mL)
test composition 269: NM-7001 formulation 411 (0.05 M phosphate buffer (pH 4.4)), (0.1 mg/mL)
test composition 270: NM-7001 formulation 412 (0.05 M phosphate buffer (pH 4.6)), (0.1 mg/mL)
test composition 271: NM-7001 formulation 413 (0.05 M phosphate buffer (pH 4.8)), (0.1 mg/mL)
test composition 272: NM-7001 formulation 414 (0.05 M phosphate buffer (pH 5.0)), (0.1 mg/mL)
test composition 273: NM-7001 formulation 415 (0.05 M phosphate buffer (pH 5.2)), (0.1 mg/mL)
test composition 274: NM-7001 formulation 416 (0.05 M phosphate buffer (pH 5.4)), (0.1 mg/mL)
test composition 275: NM-7001 formulation 417 (0.05 M phosphate buffer (pH 5.6)), (0.1 mg/mL)
test composition 276: NM-7001 formulation 418 (0.05 M phosphate buffer (pH 5.8)), (0.1 mg/mL)
test composition 277: NM-7001 formulation 419 (0.05 M phosphate buffer (pH 6.0)), (0.1 mg/mL)
test composition 278: NM-7001 formulation 420 (0.05 M phosphate buffer (pH 6.2)), (0.1 mg/mL)
test composition 279: NM-7001 formulation 421 (0.05 M phosphate buffer (pH 6.4)), (0.1 mg/mL)
test composition 280: NM-7001 formulation 422 (0.05 M phosphate buffer (pH 6.6)), (0.1 mg/mL)
test composition 281: NM-7001 formulation 423 (0.05 M phosphate buffer (pH 6.8)), (0.1 mg/mL)

test composition 282: NM-7001 formulation 424 (0.05 M phosphate buffer (pH 7.0)), (0.1 mg/mL)
test composition 283: NM-7001 formulation 425 (0.05 M phosphate buffer (pH 7.2)), (0.1 mg/mL)
test composition 284: NM-7001 formulation 426 (0.05 M phosphate buffer (pH 7.4)), (0.1 mg/mL)
test composition 285: NM-7001 formulation 427 (0.05 M phosphate buffer (pH 7.6)), (0.1 mg/mL)
test composition 286: NM-7001 formulation 428 (0.05 M phosphate buffer (pH 7.8)), (0.1 mg/mL)
test composition 287: NM-7001 formulation 429 (0.05 M phosphate buffer (pH 8.0)), (0.1 mg/mL)
test composition 288: NM-7001 formulation 430 (0.05 M citrate-phosphate (5:5) buffer (pH 4.2)), (0.1 mg/mL)
test composition 289: NM-7001 formulation 431 (0.05 M citrate-phosphate (5:5) buffer (pH 4.4)), (0.1 mg/mL)
test composition 290: NM-7001 formulation 432 (0.05 M citrate-phosphate (5:5) buffer (pH 4.6)), (0.1 mg/mL)
test composition 291: NM-7001 formulation 433 (0.05 M citrate-phosphate (5:5) buffer (pH 5.0)), (0.1 mg/mL)
test composition 292: NM-7001 formulation 434 (0.05 M citrate-phosphate (5:5) buffer (pH 6.0)), (0.1 mg/mL)
test composition 293: NM-7001 formulation 435 (0.05 M citrate-phosphate (5:5) buffer (pH 6.6)), (0.1 mg/mL)
test composition 294: NM-7001 formulation 436 (0.05 M citrate-phosphate (5:5) buffer (pH 7.0)), (0.1 mg/mL)
test composition 295: NM-7001 formulation 437 (0.05 M citrate-phosphate (5:5) buffer (pH 7.4)), (0.1 mg/mL)
test composition 296: NM-7001 formulation 438 (0.05 M citrate-phosphate (5:5) buffer (pH 7.6)), (0.1 mg/mL)

nucleic acid molecule: Kynamro-7001
test composition 297: Kynamro-7001 formulation 439 (0.05 M citrate buffer (pH 4.0)), (0.1 mg/mL)
test composition 298: Kynamro-7001 formulation 440 (0.05 M citrate buffer (pH 4.2)), (0.1 mg/mL)
test composition 299: Kynamro-7001 formulation 441 (0.05 M citrate buffer (pH 4.4)), (0.1 mg/mL)
test composition 300: Kynamro-7001 formulation 442 (0.05 M citrate buffer (pH 4.6)), (0.1 mg/mL)
test composition 301: Kynamro-7001 formulation 443 (0.05 M citrate buffer (pH 4.8)), (0.1 mg/mL)
test composition 302: Kynamro-7001 formulation 444 (0.05 M citrate buffer (pH 5.0)), (0.1 mg/mL)
test composition 303: Kynamro-7001 formulation 445 (0.05 M citrate buffer (pH 5.2)), (0.1 mg/mL)
test composition 304: Kynamro-7001 formulation 446 (0.05 M citrate buffer (pH 5.4)), (0.1 mg/mL)
test composition 305: Kynamro-7001 formulation 447 (0.05 M citrate buffer (pH 5.6)), (0.1 mg/mL)
test composition 306: Kynamro-7001 formulation 448 (0.05 M citrate buffer (pH 5.8)), (0.1 mg/mL)
test composition 307: Kynamro-7001 formulation 449 (0.05 M citrate buffer (pH 6.0)), (0.1 mg/mL)
test composition 308: Kynamro-7001 formulation 450 (0.05 M citrate buffer (pH 6.2)), (0.1 mg/mL)
test composition 309: Kynamro-7001 formulation 451 (0.05 M citrate buffer (pH 6.4)), (0.1 mg/mL)
test composition 310: Kynamro-7001 formulation 452 (0.05 M citrate buffer (pH 6.6)), (0.1 mg/mL)
test composition 311: Kynamro-7001 formulation 453 (0.05 M citrate buffer (pH 6.8)), (0.1 mg/mL)
test composition 312: Kynamro-7001 formulation 454 (0.05 M citrate buffer (pH 7.0)), (0.1 mg/mL)
test composition 313: Kynamro-7001 formulation 455 (0.05 M citrate buffer (pH 7.2)), (0.1 mg/mL)
test composition 314: Kynamro-7001 formulation 456 (0.05 M citrate buffer (pH 7.4)), (0.1 mg/mL)
test composition 315: Kynamro-7001 formulation 457 (0.05 M phosphate buffer (pH 4.0)), (0.1 mg/mL)
test composition 316: Kynamro-7001 formulation 458 (0.05 M phosphate buffer (pH 4.2)), (0.1 mg/mL)
test composition 317: Kynamro-7001 formulation 459 (0.05 M phosphate buffer (pH 4.4)), (0.1 mg/mL)
test composition 318: Kynamro-7001 formulation 460 (0.05 M phosphate buffer (pH 4.6)), (0.1 mg/mL)
test composition 319: Kynamro-7001 formulation 461 (0.05 M phosphate buffer (pH 4.8)), (0.1 mg/mL)
test composition 320: Kynamro-7001 formulation 462 (0.05 M phosphate buffer (pH 5.0)), (0.1 mg/mL)
test composition 321: Kynamro-7001 formulation 463 (0.05 M phosphate buffer (pH 5.2)), (0.1 mg/mL)
test composition 322: Kynamro-7001 formulation 464 (0.05 M phosphate buffer (pH 5.4)), (0.1 mg/mL)
test composition 323: Kynamro-7001 formulation 465 (0.05 M phosphate buffer (pH 5.6)), (0.1 mg/mL)
test composition 324: Kynamro-7001 formulation 466 (0.05 M phosphate buffer (pH 5.8)), (0.1 mg/mL)
test composition 325: Kynamro-7001 formulation 467 (0.05 M phosphate buffer (pH 6.0)), (0.1 mg/mL)
test composition 326: Kynamro-7001 formulation 468 (0.05 M phosphate buffer (pH 6.2)), (0.1 mg/mL)
test composition 327: Kynamro-7001 formulation 469 (0.05 M phosphate buffer (pH 6.4)), (0.1 mg/mL)
test composition 328: Kynamro-7001 formulation 470 (0.05 M phosphate buffer (pH 6.6)), (0.1 mg/mL)
test composition 329: Kynamro-7001 formulation 471 (0.05 M phosphate buffer (pH 6.8)), (0.1 mg/mL)
test composition 330: Kynamro-7001 formulation 472 (0.05 M phosphate buffer (pH 7.0)), (0.1 mg/mL)
test composition 331: Kynamro-7001 formulation 473 (0.05 M phosphate buffer (pH 7.2)), (0.1 mg/mL)
test composition 332: Kynamro-7001 formulation 474 (0.05 M phosphate buffer (pH 7.4)), (0.1 mg/mL)
test composition 333: Kynamro- 7001 formulation 475 (0.05 M phosphate buffer (pH 7.6)), (0.1 mg/mL)
test composition 334: Kynamro-7001 formulation 476 (0.05 M phosphate buffer (pH 7.8)), (0.1 mg/mL)
test composition 335: Kynamro-7001 formulation 477 (0.05 M phosphate buffer (pH 8.0)), (0.1 mg/mL)

test composition 336: Kynamro-7001 formulation 478 (0.05 M citrate-phosphate (5:5) buffer (pH 4.2)), (0.1 mg/mL)
test composition 337: Kynamro-7001 formulation 479 (0.05 M citrate-phosphate (5:5) buffer (pH 4.4)), (0.1 mg/mL)
test composition 338: Kynamro-7001 formulation 480 (0.05 M citrate-phosphate (5:5) buffer (pH 4.6)), (0.1 mg/mL)
test composition 339: Kynamro-7001 formulation 481 (0.05 M citrate-phosphate (5:5) buffer (pH 5.0)), (0.1 mg/mL)
test composition 340: Kynamro-7001 formulation 482 (0.05 M citrate-phosphate (5:5) buffer (pH 6.0)), (0.1 mg/mL)
test composition 341: Kynamro-7001 formulation 483 (0.05 M citrate-phosphate (5:5) buffer (pH 6.6)), (0.1 mg/mL)
test composition 342: Kynamro-7001 formulation 484 (0.05 M citrate-phosphate (5:5) buffer (pH 7.0)), (0.1 mg/mL)
test composition 343: Kynamro-7001 formulation 485 (0.05 M citrate-phosphate (5:5) buffer (pH 7.4)), (0.1 mg/mL)
test composition 344: Kynamro-7001 formulation 486 (0.05 M citrate-phosphate (5:5) buffer (pH 7.6)), (0.1 mg/mL)

nucleic acid molecule: Macugen-700
test composition 345: Macugen-7001 formulation 487 (0.05 M citrate buffer (pH 4.0)), (0.1 mg/mL)
test composition 346: Macugen-7001 formulation 488 (0.05 M citrate buffer (pH 4.2)), (0.1 mg/mL)
test composition 347: Macugen-7001 formulation 489 (0.05 M citrate buffer (pH 4.4)), (0.1 mg/mL)
test composition 348: Macugen-7001 formulation 490 (0.05 M citrate buffer (pH 4.6)), (0.1 mg/mL)
test composition 349: Macugen-7001 formulation 491 (0.05 M citrate buffer (pH 4.8)), (0.1 mg/mL)
test composition 350: Macugen-7001 formulation 492 (0.05 M citrate buffer (pH 5.0)), (0.1 mg/mL)
test composition 351: Macugen-7001 formulation 493 (0.05 M citrate buffer (pH 5.2)), (0.1 mg/mL)
test composition 352: Macugen-7001 formulation 494 (0.05 M citrate buffer (pH 5.4)), (0.1 mg/mL)
test composition 353: Macugen-7001 formulation 495 (0.05 M citrate buffer (pH 5.6)), (0.1 mg/mL)
test composition 354: Macugen-7001 formulation 496 (0.05 M citrate buffer (pH 5.8)), (0.1 mg/mL)
test composition 355: Macugen-7001 formulation 497 (0.05 M citrate buffer (pH 6.0)), (0.1 mg/mL)
test composition 356: Macugen-7001 formulation 498 (0.05 M citrate buffer (pH 6.2)), (0.1 mg/mL)
test composition 357: Macugen-7001 formulation 499 (0.05 M citrate buffer (pH 6.4)), (0.1 mg/mL)
test composition 358: Macugen-7001 formulation 500 (0.05 M citrate buffer (pH 6.6)), (0.1 mg/mL)
test composition 359: Macugen-7001 formulation 501 (0.05 M citrate buffer (pH 6.8)), (0.1 mg/mL)
test composition 360: Macugen-7001 formulation 502 (0.05 M citrate buffer (pH 7.0)), (0.1 mg/mL)
test composition 361: Macugen-7001 formulation 503 (0.05 M citrate buffer (pH 7.2)), (0.1 mg/mL)
test composition 362: Macugen-7001 formulation 504 (0.05 M citrate buffer (pH 7.4)), (0.1 mg/mL)
test composition 363: Macugen-7001 formulation 505 (0.05 M phosphate buffer (pH 4.0)), (0.1 mg/mL)
test composition 364: Macugen-7001 formulation 506 (0.05 M phosphate buffer (pH 4.2)), (0.1 mg/mL)
test composition 365: Macugen-7001 formulation 507 (0.05 M phosphate buffer (pH 4.4)), (0.1 mg/mL)
test composition 366: Macugen-7001 formulation 508 (0.05 M phosphate buffer (pH 4.6)), (0.1 mg/mL)
test composition 367: Macugen-7001 formulation 509 (0.05 M phosphate buffer (pH 4.8)), (0.1 mg/mL)
test composition 368: Macugen-7001 formulation 510 (0.05 M phosphate buffer (pH 5.0)), (0.1 mg/mL)
test composition 369: Macugen-7001 formulation 511 (0.05 M phosphate buffer (pH 5.2)), (0.1 mg/mL)
test composition 370: Macugen-7001 formulation 512 (0.05 M phosphate buffer (pH 5.4)), (0.1 mg/mL)
test composition 371: Macugen-7001 formulation 513 (0.05 M phosphate buffer (pH 5.6)), (0.1 mg/mL)
test composition 372: Macugen-7001 formulation 514 (0.05 M phosphate buffer (pH 5.8)), (0.1 mg/mL)
test composition 373: Macugen-7001 formulation 515 (0.05 M phosphate buffer (pH 6.0)), (0.1 mg/mL)
test composition 374: Macugen-7001 formulation 516 (0.05 M phosphate buffer (pH 6.2)), (0.1 mg/mL)
test composition 375: Macugen-7001 formulation 517 (0.05 M phosphate buffer (pH 6.4)), (0.1 mg/mL)
test composition 376: Macugen-7001 formulation 518 (0.05 M phosphate buffer (pH 6.6)), (0.1 mg/mL)
test composition 377: Macugen-7001 formulation 519 (0.05 M phosphate buffer (pH 6.8)), (0.1 mg/mL)
test composition 378: Macugen-7001 formulation 520 (0.05 M phosphate buffer (pH 7.0)), (0.1 mg/mL)
test composition 379: Macugen-7001 formulation 521 (0.05 M phosphate buffer (pH 7.2)), (0.1 mg/mL)
test composition 380: Macugen-7001 formulation 522 (0.05 M phosphate buffer (pH 7.4)), (0.1 mg/mL)
test composition 381: Macugen-7001 formulation 523 (0.05 M phosphate buffer (pH 7.6)), (0.1 mg/mL)
test composition 382: Macugen-7001 formulation 524 (0.05 M phosphate buffer (pH 7.8)), (0.1 mg/mL)
test composition 383: Macugen-7001 formulation 525 (0.05 M phosphate buffer (pH 8.0)), (0.1 mg/mL)
test composition 384: Macugen-7001 formulation 526 (0.05 M citrate-phosphate (5:5) buffer (pH 4.2)), (0.1 mg/mL)
test composition 385: Macugen-7001 formulation 527 (0.05 M citrate-phosphate (5:5) buffer (pH 4.4)), (0.1 mg/mL)
test composition 386: Macugen-7001 formulation 528 (0.05 M citrate-phosphate (5:5) buffer (pH 4.6)), (0.1 mg/mL)
test composition 387: Macugen-7001 formulation 529 (0.05 M citrate-phosphate (5:5) buffer (pH 5.0)), (0.1 mg/mL)
test composition 388: Macugen-7001 formulation 530 (0.05 M citrate-phosphate (5:5) buffer (pH 6.0)), (0.1 mg/mL)
test composition 389: Macugen-7001 formulation 531 (0.05 M citrate-phosphate (5:5) buffer (pH 6.6)), (0.1 mg/mL)

test composition 390: Macugen-7001 formulation 532 (0.05 M citrate-phosphate (5:5) buffer (pH 7.0)), (0.1 mg/mL)
test composition 391: Macugen-7001 formulation 533 (0.05 M citrate-phosphate (5:5) buffer (pH 7.4)), (0.1 mg/mL)
test composition 392: Macugen-7001 formulation 534 (0.05 M citrate-phosphate (5:5) buffer (pH 7.6)), (0.1 mg/mL)

Example 7-2 (test method and diagnostic criteria)

One each of the test compositions 57 - 392 was stored at 4°C and four each were stored in a stability test chamber at 60°C. Each stored product at 4°C was taken out at the time of start and each stored product at 60°C was taken out every week, the content was calculated by reversed-phase HPLC, and the stability was evaluated based on a decrease in the content ratio (%) relative to the content at the time of start of the storage.

Using each test composition at the time of start as the calibration curve sample (100%), calibration curve samples (60% - 100%) were prepared by a method similar to that in Example 1-2. The calibration curve samples (60% - 100%) and each storage sample (each 30 μL) were measured by HPLC according to a method similar to that in Example 1-2.

Example 7-3 (results)

The results are shown in Figs. 17 - 23. The results show that PK-7006, NK-7006 and PH-7069 have high storage stability even at 60°C, 4 weeks.

[Industrial Applicability]

According to the present invention, a novel liquid nucleic acid-containing composition, particularly a pharmaceutical composition, showing improved stability of nucleic acid molecule can be provided. Therefore, storage, transportation and the like at ambient temperature become possible, and the composition is highly useful since a nucleic acid-containing composition with superior handleability can be provided.

This application is based on patent application Nos. 2014-267087 filed in Japan (filing date: December 29, 2014) and 2015-081298 filed in Japan (filing date: April 10, 2015), the contents of which are incorporated in full herein.

## Claims

1. A composition comprising a nucleic acid molecule and a buffer, and having the following features:

   (a) being in the form of a solution at ambient temperature; and
   (b) a content of the nucleic acid molecule after storage at 25°C, relative humidity 60% for 4 weeks, of not less than 80% relative to the content at the time of start of the storage.

2. The composition according to claim 1, wherein the content of the nucleic acid molecule after storage at 40°C, relative humidity 75% for 4 weeks is not less than 80% relative to the content at the time of start of the storage.

3. The composition according to claim 1 or 2, wherein the content of the nucleic acid molecule after storage at 60°C for 4 weeks is not less than 60% relative to the content at the time of start of the storage.

4. The composition according to any one of claims 1 to 3, wherein the buffer adjusts the pH of the composition to not less than 4.0 and not more than 9.0.

5. The composition according to any one of claims 1 to 3, wherein the buffer adjusts the pH of the composition to not less than 5.5 and not more than 7.5.

6. The composition according to any one of claims 1 to 3, wherein the buffer adjusts the pH of the composition to not less than 6.0 and not more than 7.0.

7. The composition according to any one of claims 1 to 6, wherein the buffer comprises one or more buffering agents selected from sodium hydrogen phosphate, sodium dihydrogen phosphate, disodium hydrogen phosphate, sodium chloride, arginine hydrochloride, sodium citrate, trisodium citrate dihydrate, monosodium L-glutamate, sodium acetate, sodium carbonate, sodium hydrogen carbonate, sodium lactate, monopotassium phosphate, sodium hydroxide, meglumine, glycine, citric acid, and acetic acid.

8. The composition according to any one of claims 1 to 7, wherein the buffer comprises citric acid and/or phosphoric acid.

9. The composition according to any one of claims 1 to 8, wherein said nucleic acid molecule is a single-stranded nucleic acid molecule or a double-stranded nucleic acid molecule.

10. The composition according to any one of claims 1 to 9, wherein said nucleic acid molecule is a DNA molecule, an RNA molecule, or a chimeric nucleic acid molecule of DNA and RNA.

11. The composition according to any one of claims 1 to 10, wherein the nucleotide number of said nucleic acid molecule is 10 - 300.

12. The composition according to any one of claims 1 to 11, wherein said nucleic acid molecule comprises a sequence that controls expression of a target gene or function of a target protein.

**13.** The composition according to any one of claims 1 to 11, comprising a nucleic acid molecule comprising a sequence that controls expression of a target gene.

**14.** The composition according to any one of claims 1 to 13, wherein said nucleic acid molecule is antisense nucleic acid, siRNA or shRNA, miRNA, ribozyme, decoy nucleic acid or aptamer.

**15.** The composition according to any one of claims 1 to 14, which is a pharmaceutical composition.

**16.** A method of producing the composition according to any one of claims 1 to 15, comprising dissolving said nucleic acid molecule in a buffer adjusting a pH of the composition to not less than 6.0 and not more than 7.0, and storing the solution at ambient temperature.

**17.** A method for stabilizing a nucleic acid molecule in a composition, comprising dissolving the nucleic acid molecule in a buffer adjusting a pH of the composition to not less than 6.0 and not more than 7.0, and storing the solution at ambient temperature.

**18.** The method according to claim 16 or 17, wherein the buffer comprises citric acid and/or phosphoric acid.

**19.** The method according to any one of claims 16 to 18, wherein the composition is a pharmaceutical composition.

FIG 1

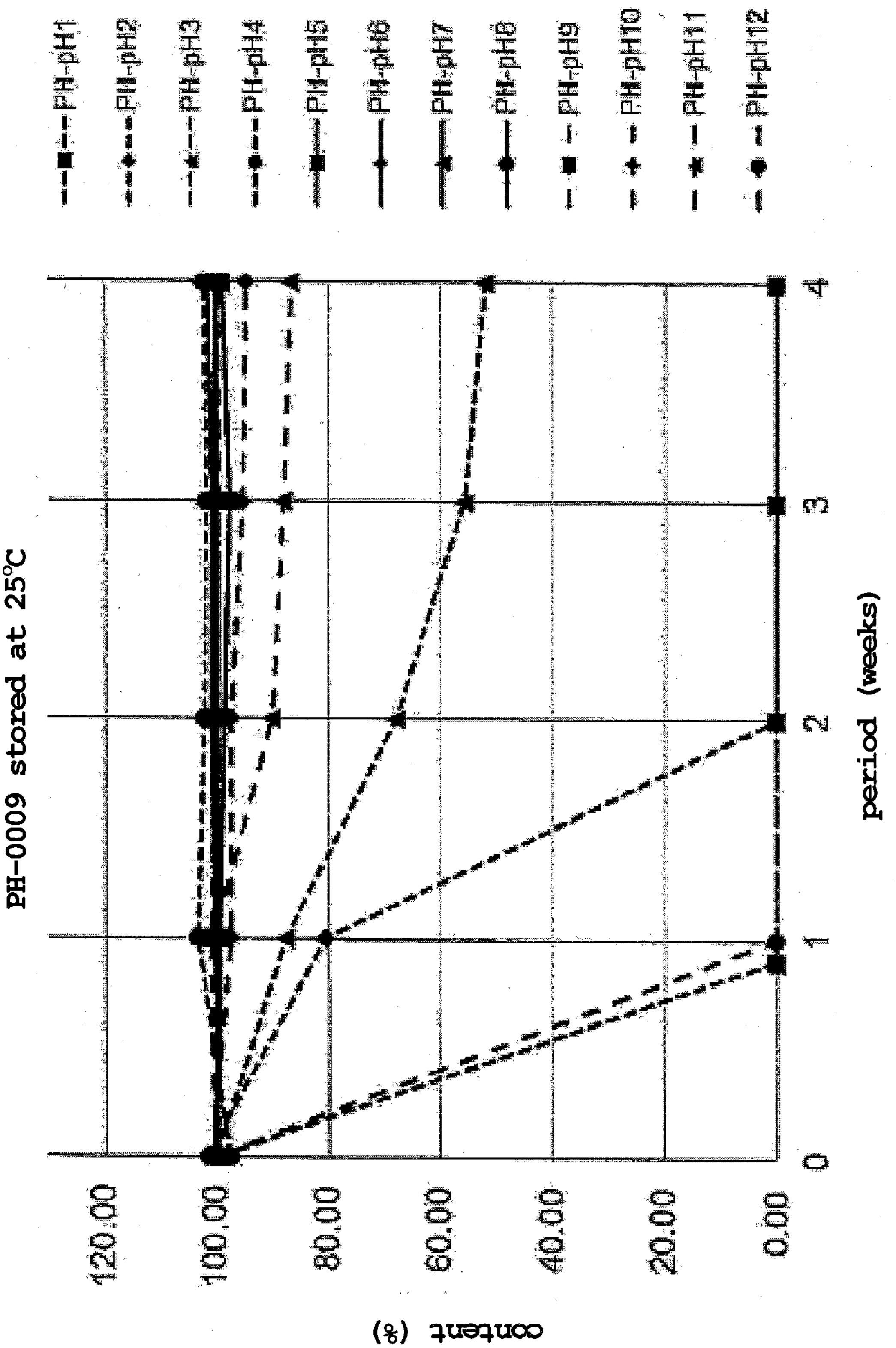
PH-0009 stored at 25°C
content (%)
120.00
100.00
80.00
60.00
40.00
20.00
0.00
0
1
2
3
4
period (weeks)
PH-pH1
PH-pH2
PH-pH3
PH-pH4
PH-pH5
PH-pH6
PH-pH7
PH-pH8
PH-pH9
PH-pH10
PH-pH11
PH-pH12

FIG 2

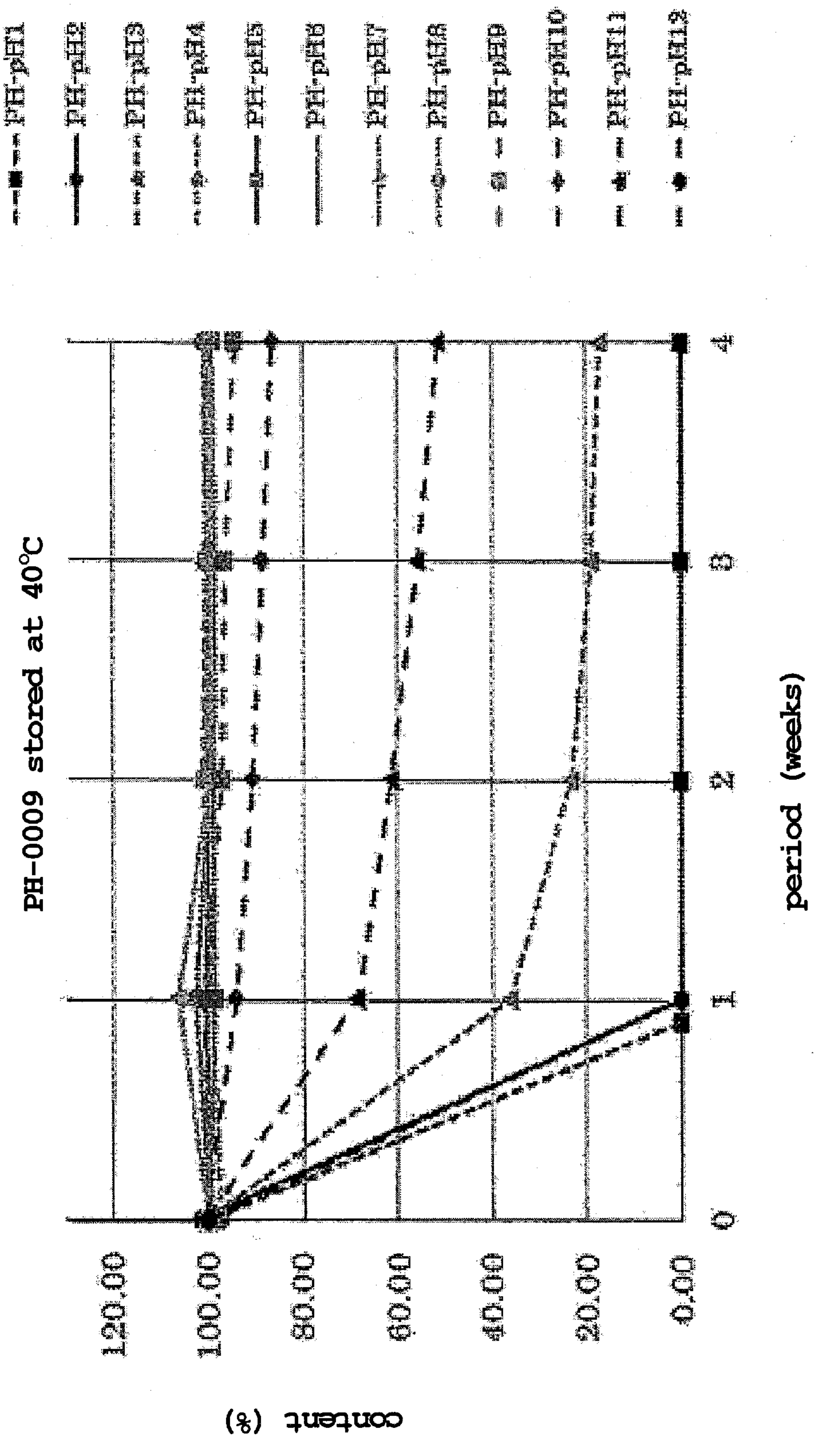
PH-0009 stored at 40°C
content (%)
120.00
100.00
80.00
60.00
40.00
20.00
0.00
0
1
2
3
4
period (weeks)
PH-pH1
PH-pH2
PH-pH3
PH-pH4
PH-pH5
PH-pH6
PH-pH7
PH-pH8
PH-pH9
PH-pH10
PH-pH11
PH-pH12

FIG 3

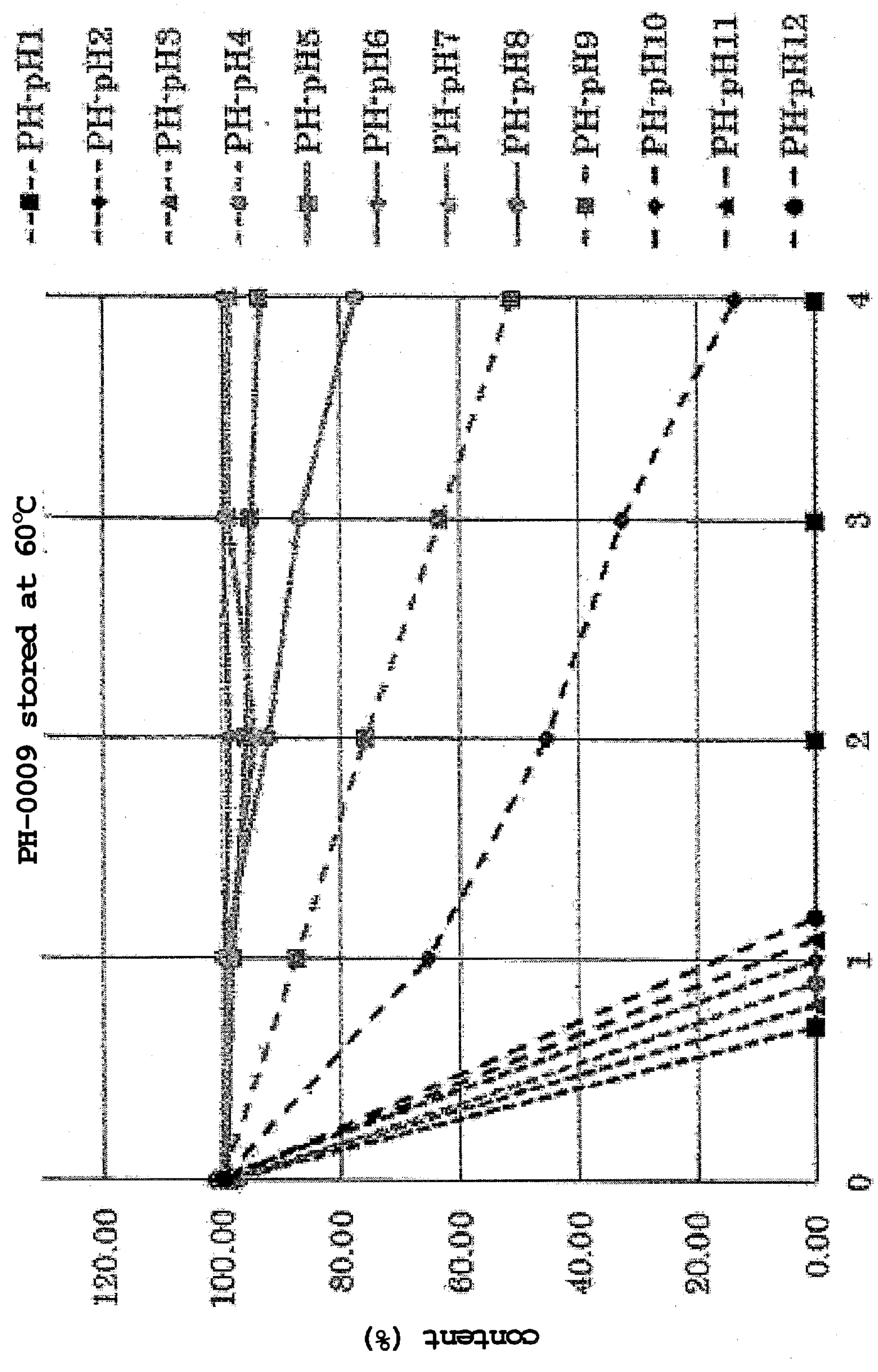
PH-0009 stored at 60°C
content (%)
120.00
100.00
80.00
60.00
40.00
20.00
0.00
0
1
2
3
4
period (weeks)
PH-pH1
PH-pH2
PH-pH3
PH-pH4
PH-pH5
PH-pH6
PH-pH7
PH-pH8
PH-pH9
PH-pH10
PH-pH11
PH-pH12

FIG 4

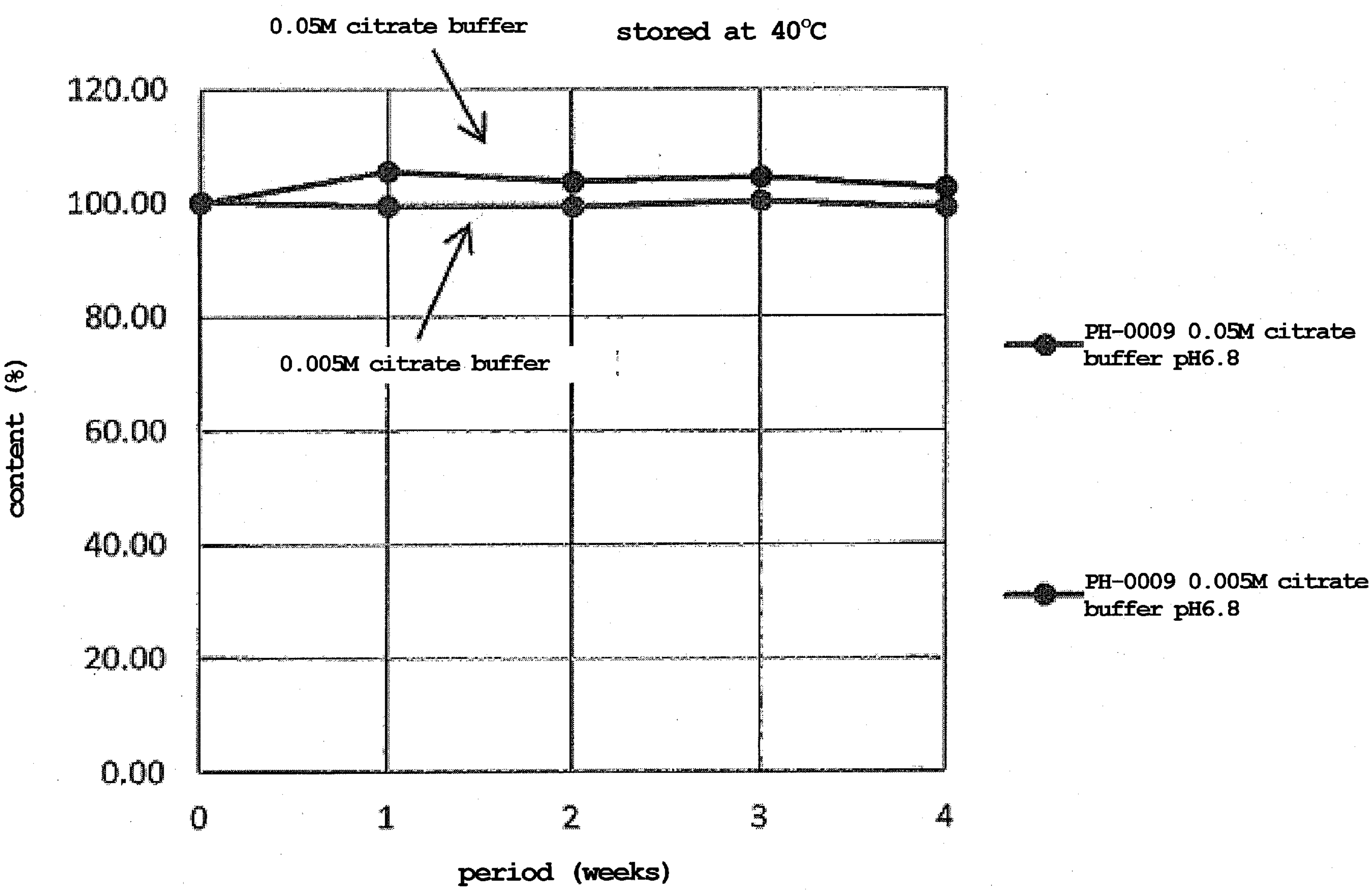
stored at 40°C
0.05M citrate buffer
0.005M citrate buffer
120.00
100.00
80.00
60.00
40.00
20.00
0.00
content (%)
0
1
2
3
4
period (weeks)
PH-0009 0.05M citrate buffer pH6.8
PH-0009 0.005M citrate buffer pH6.8

FIG 5

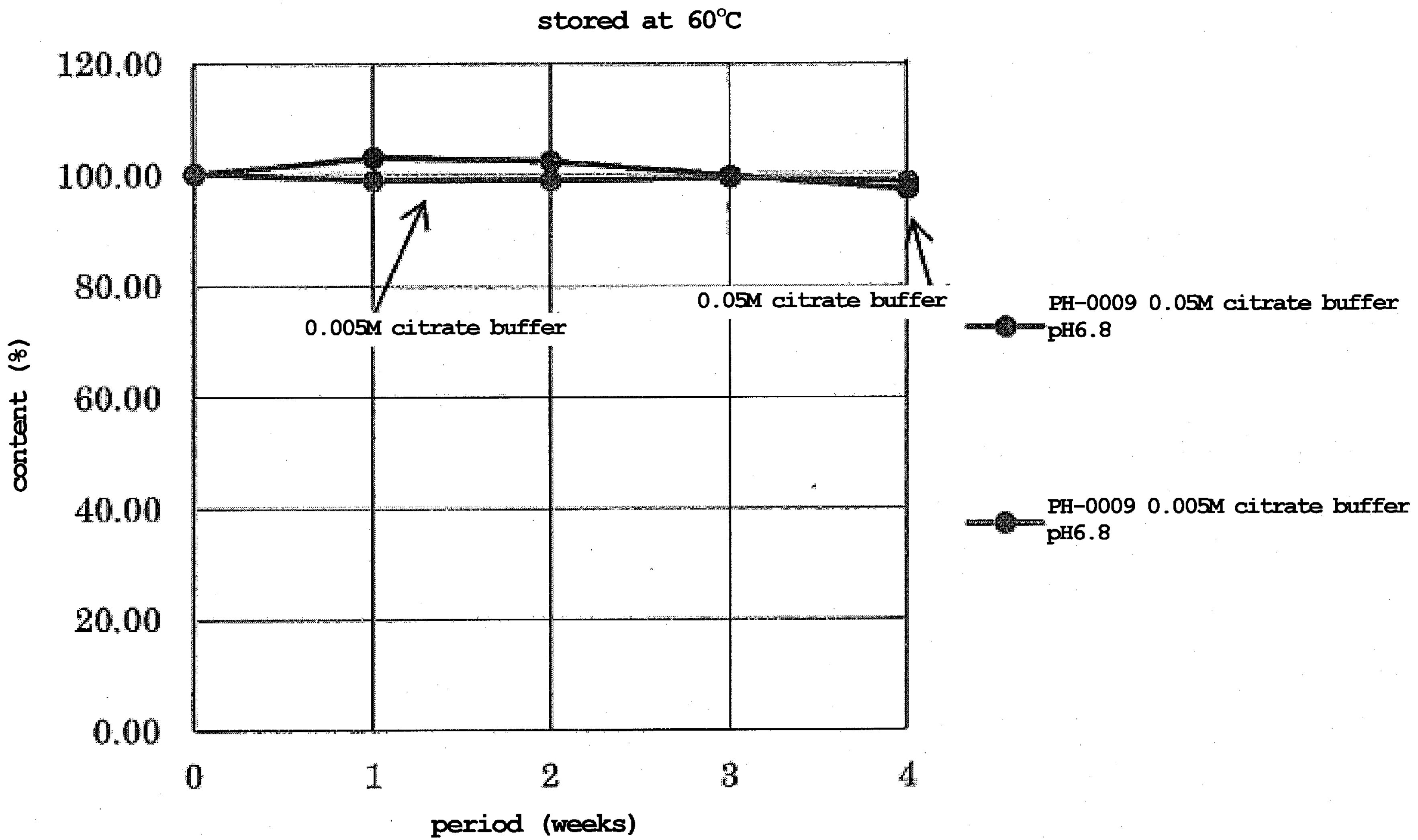
stored at 60°C
120.00
100.00
80.00
60.00
40.00
20.00
0.00
content (%)
0
1
2
3
4
period (weeks)
0.005M citrate buffer
0.05M citrate buffer
PH-0009 0.05M citrate buffer pH6.8
PH-0009 0.005M citrate buffer pH6.8

FIG 6

PH-0009 formulation 44 (0.05M citrate buffer (pH6.5) (10 mg/mL)
storage stability

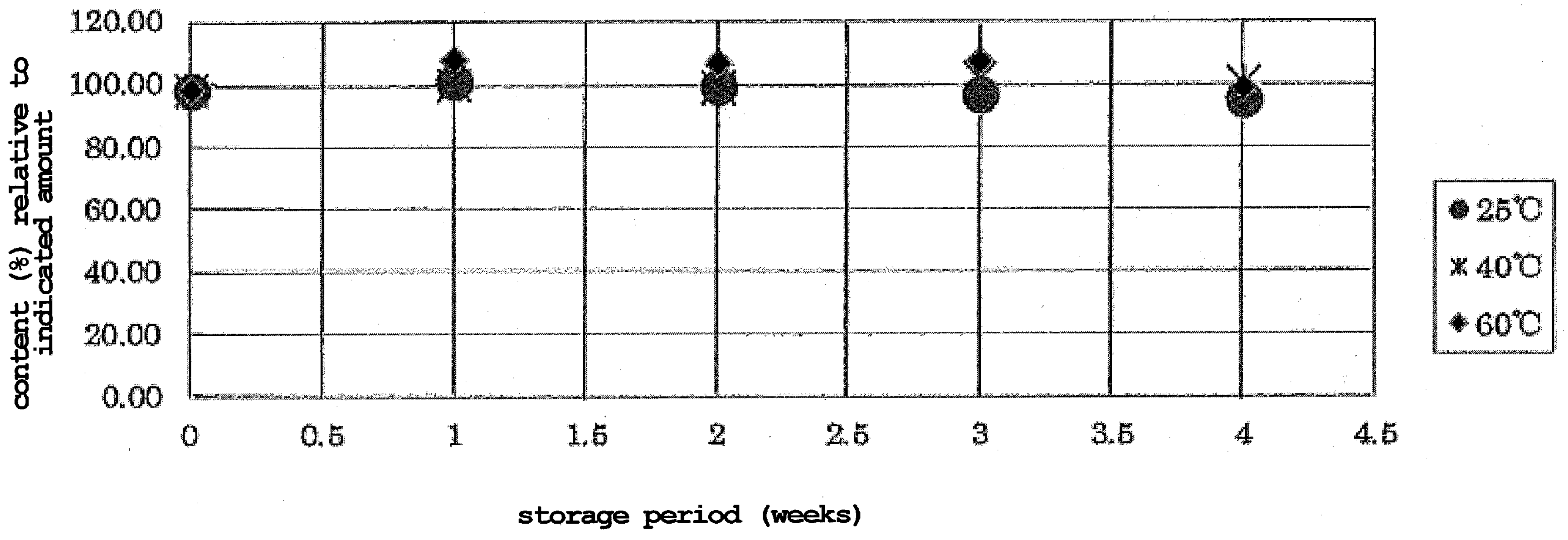

FIG 7

NK-7006 (0.1 mg/mL) storage stability

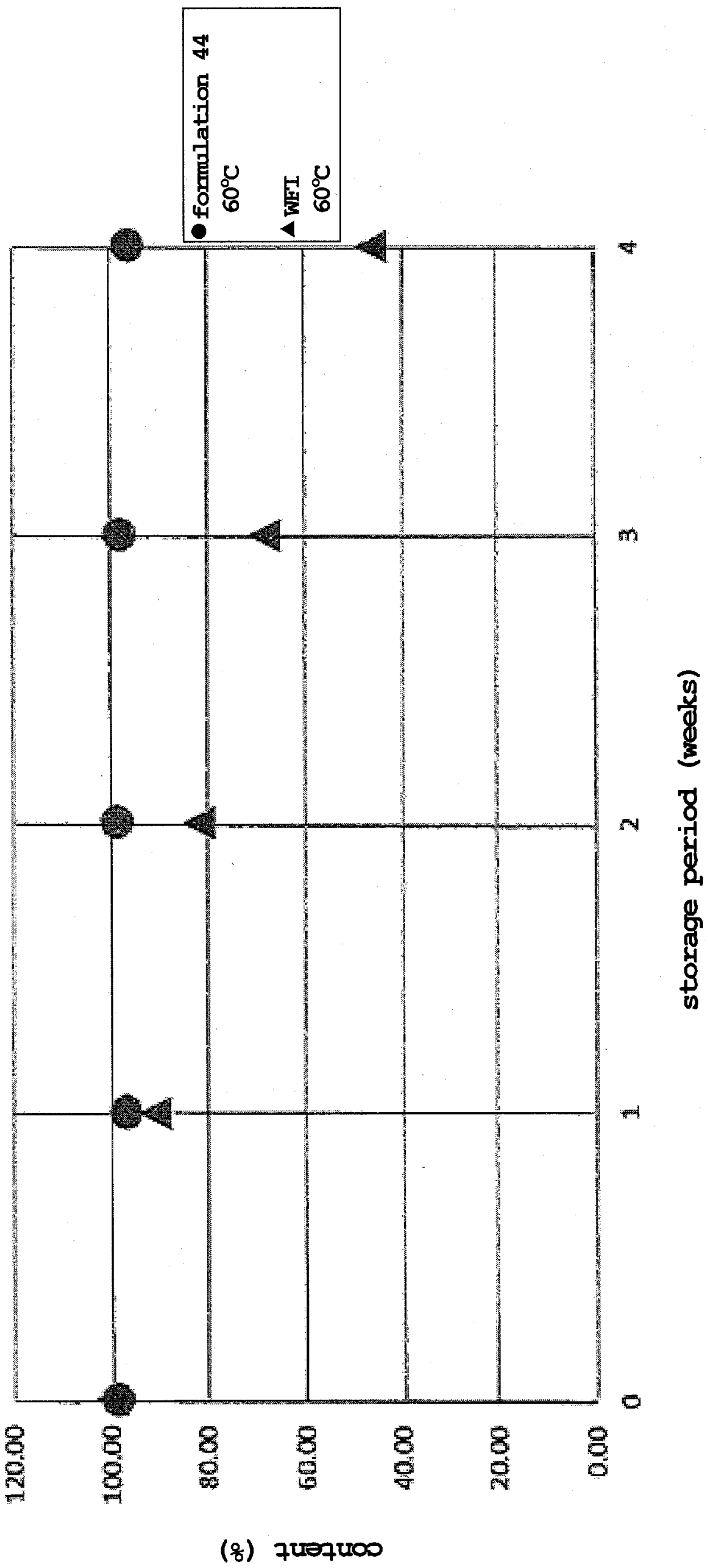


Nk: NK-7006 (luciferase, L2 sequence) 62 bases

FIG 8

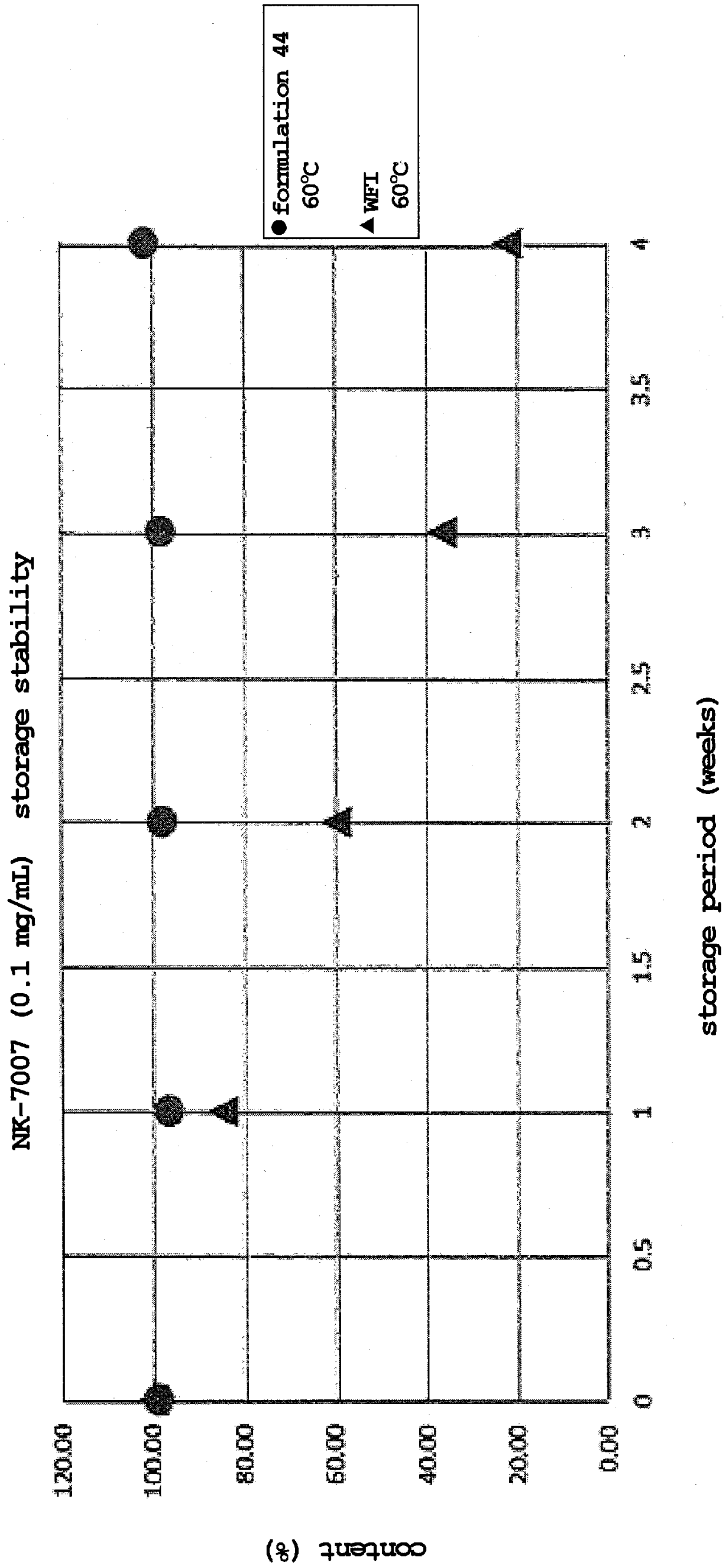


Nk: NK-7007 (mGAPDH sequence) 62 bases

FIG 9

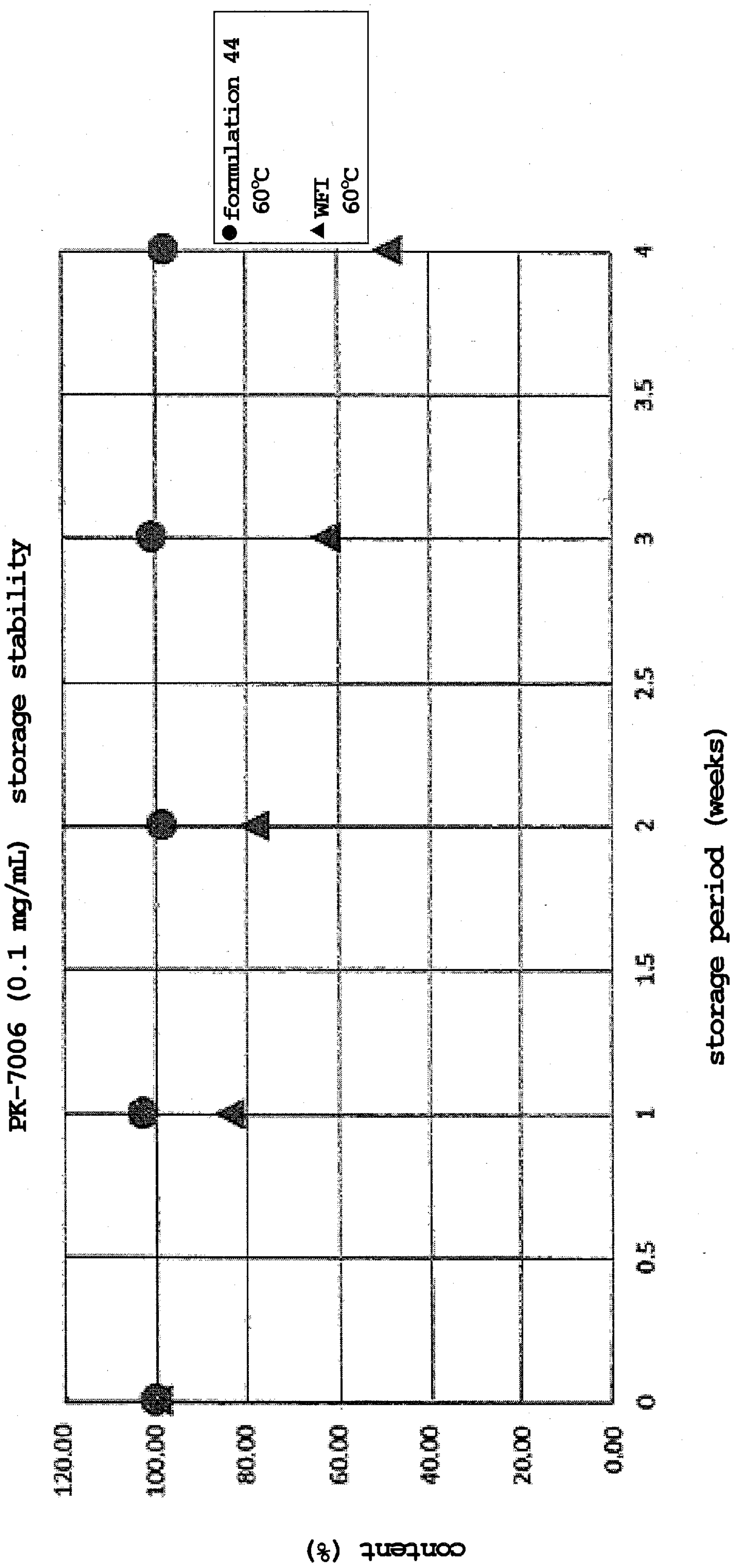


Pnk: PK-7006 (mTGF-β1 sequence) 53 bases

FIG 10

PK-7015 (0.1 mg/mL) storage stability

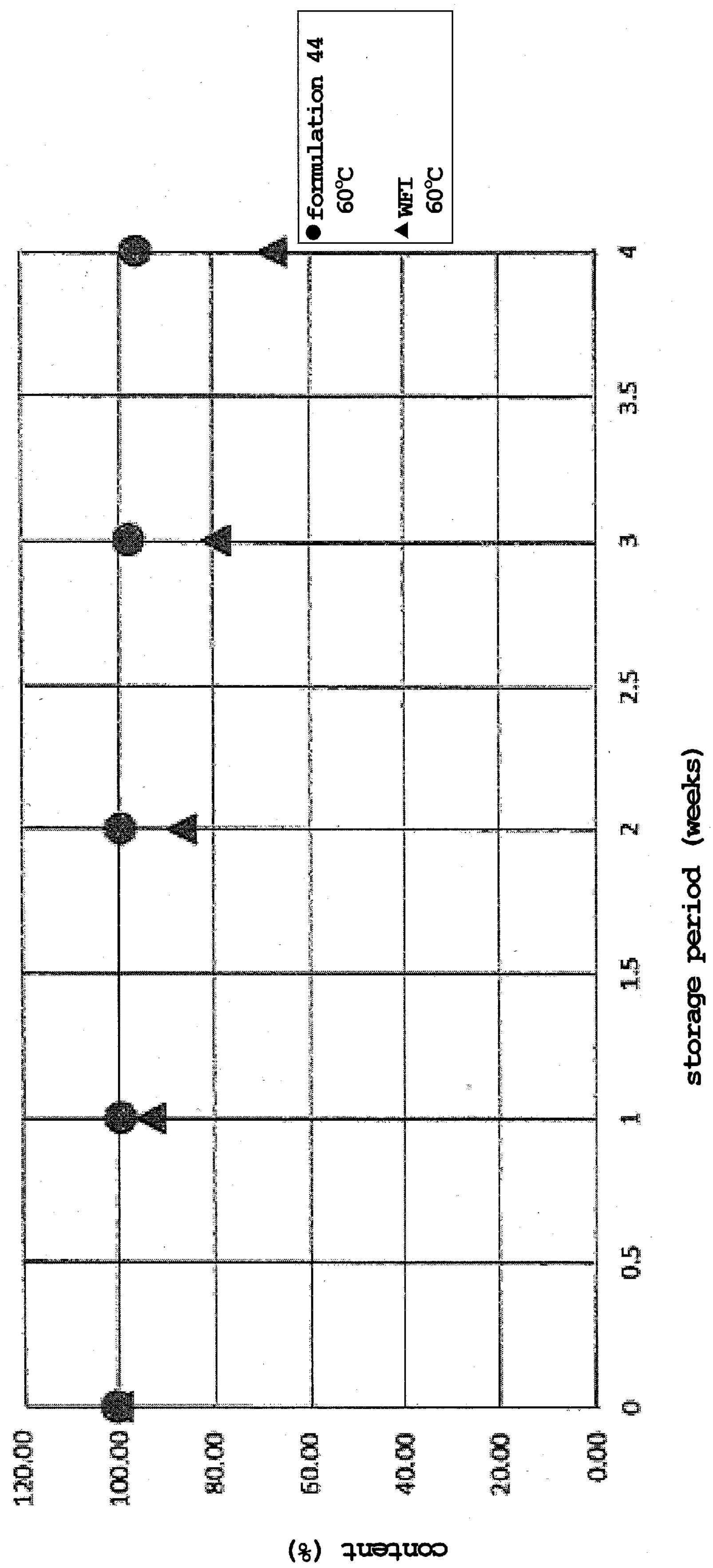


Psh: PK-7015 (mCCR3 sequence) 53 bases

FIG 11

PH-7069 (0.1 mg/mL) storage stability

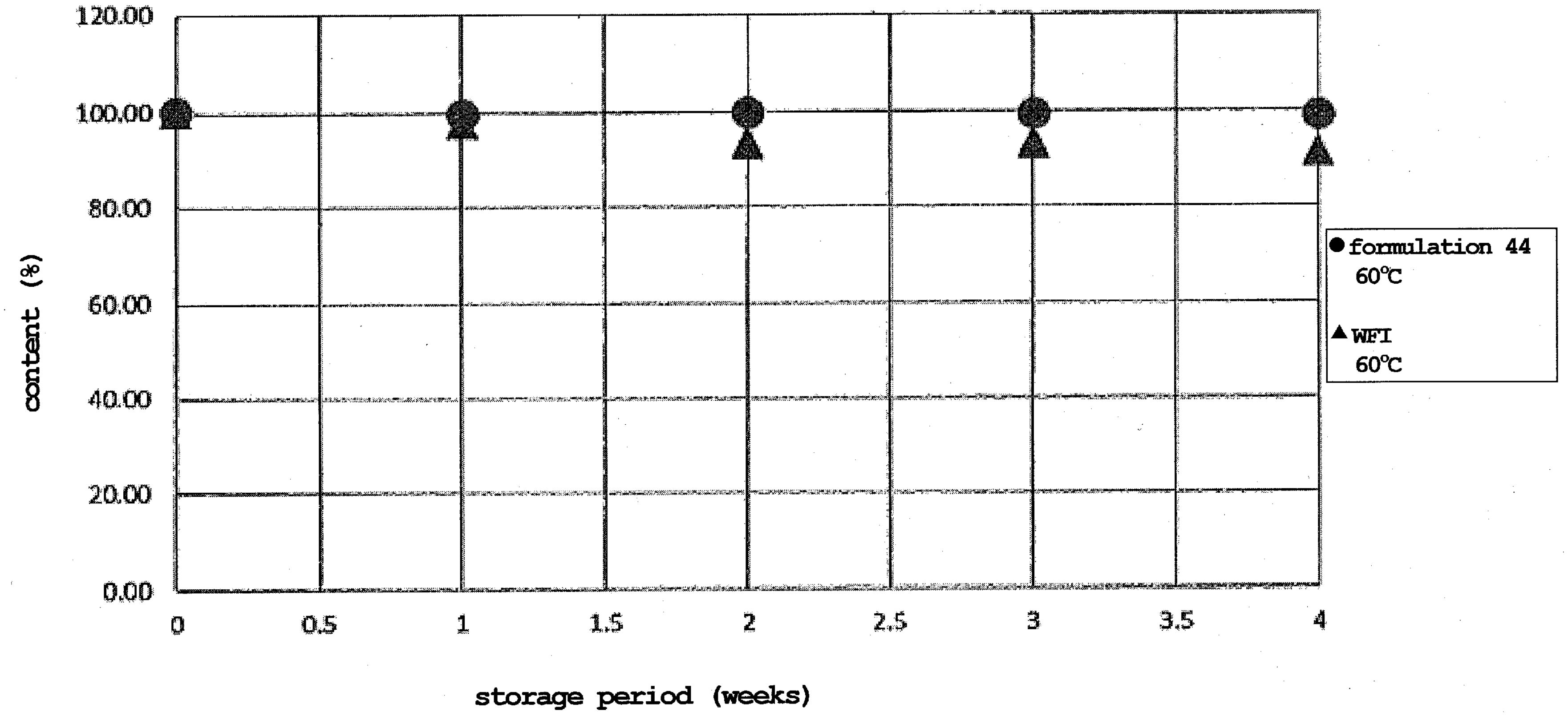


Psh: PH-7069 (mSmad3 sequence) 50 bases

FIG 12

Kynamro7001 (0.1 mg/mL) storage stability

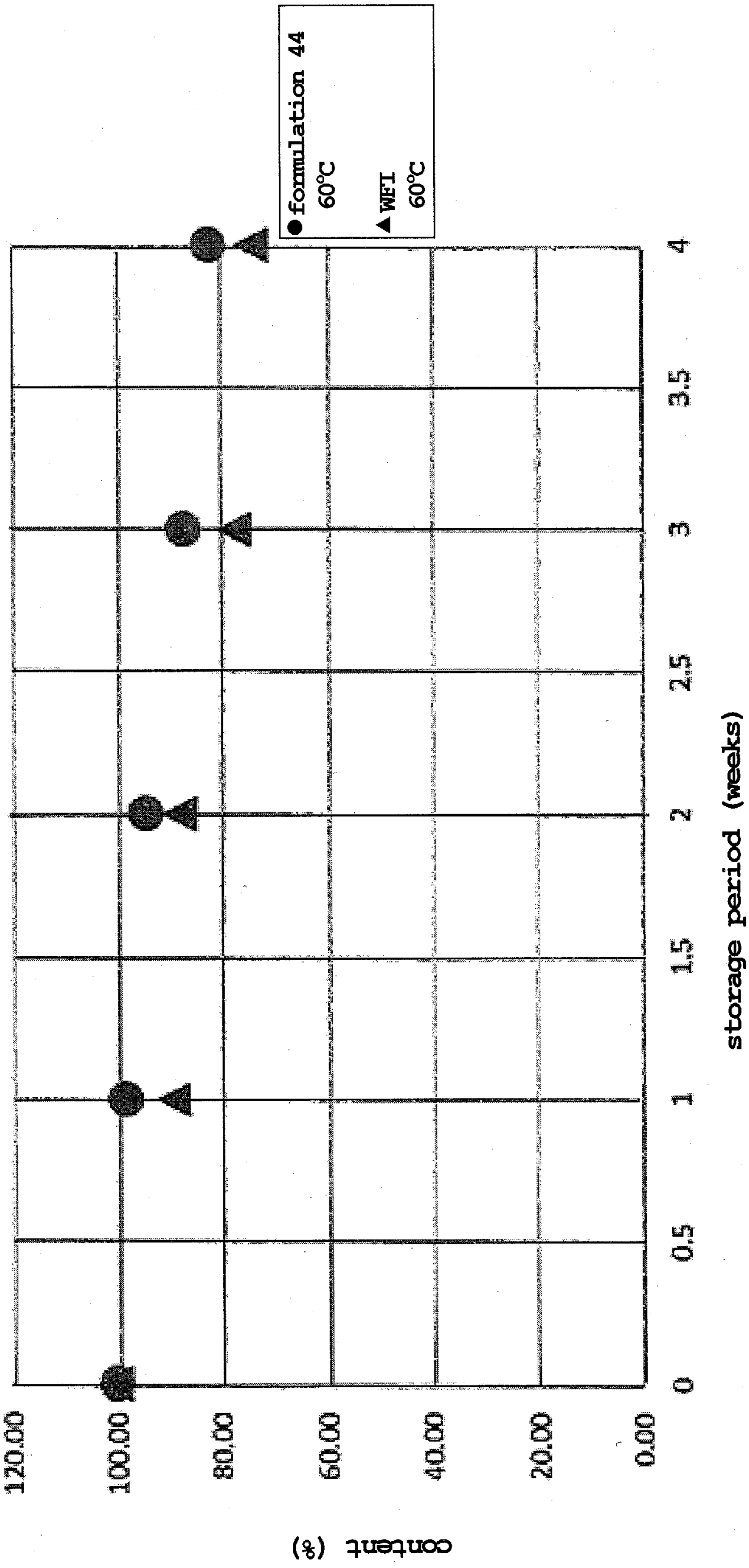


antisense: Kynamuro (mipomersen) unmodified sequence 20 bases

FIG 13

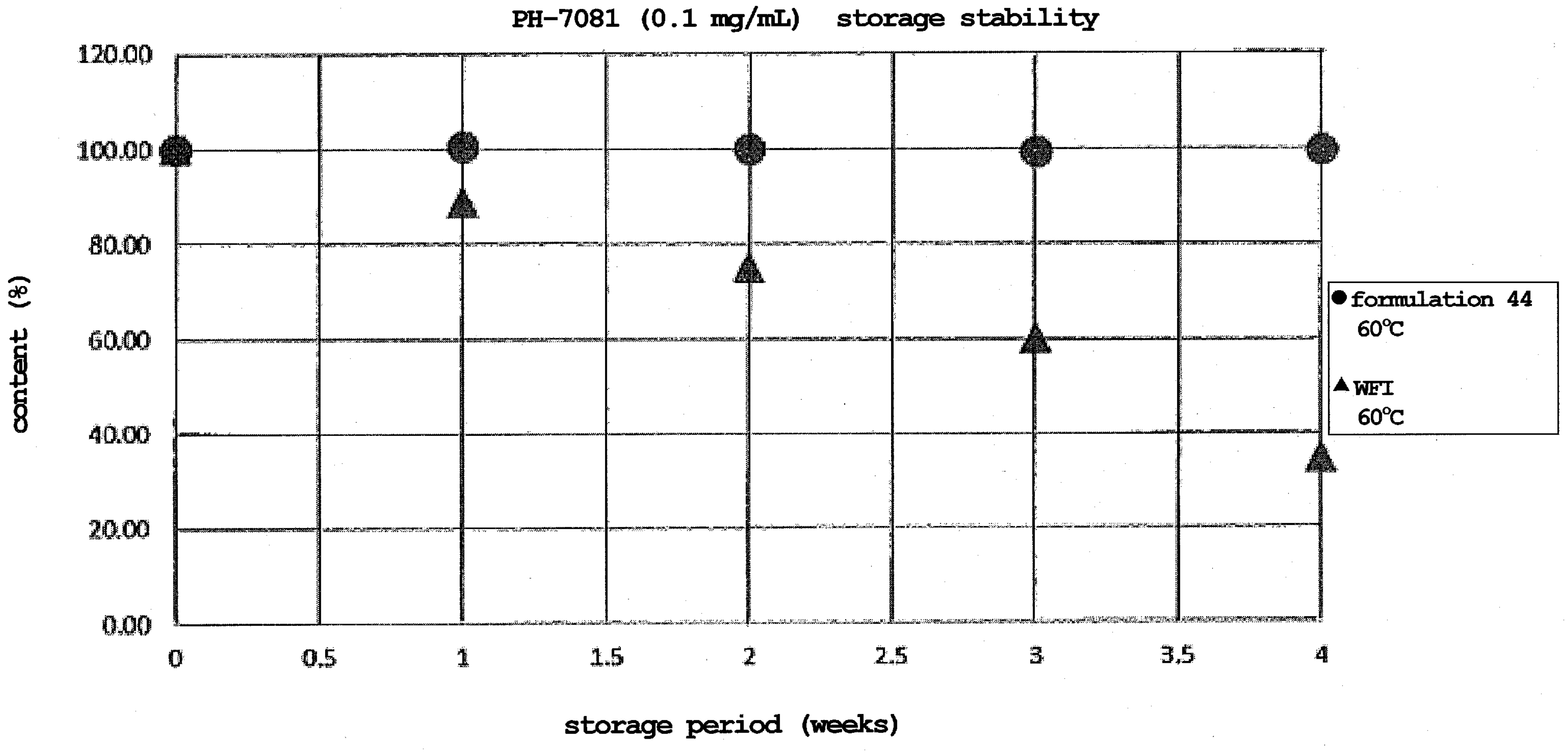


Psh: PH-7081 (firefly luciferase sequence) 49 bases

FIG 14

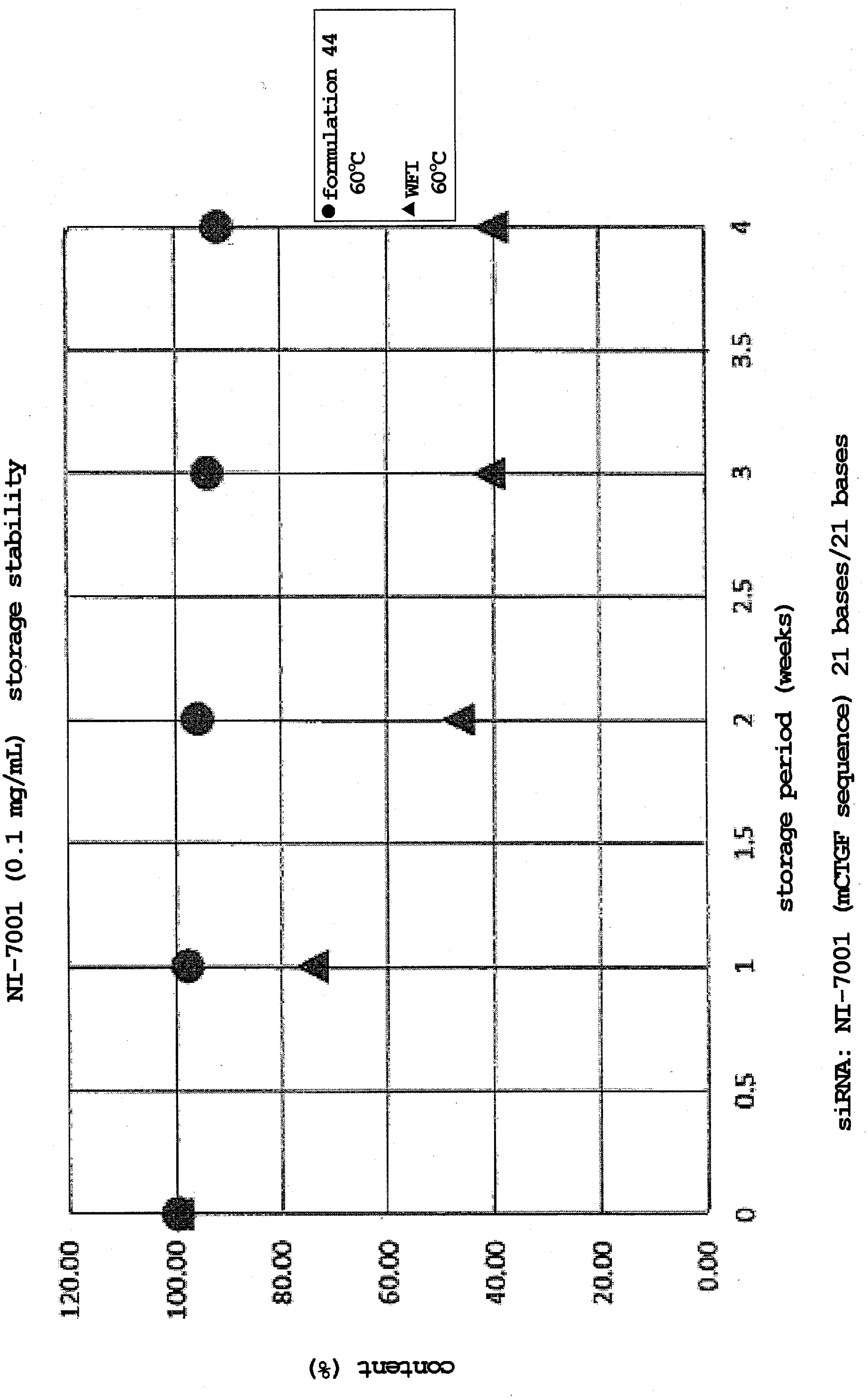
NI-7001 (0.1 mg/mL) storage stability
formulation 44 60°C
WFI 60°C
content (%)
120.00
100.00
80.00
60.00
40.00
20.00
0.00
0
0.5
1
1.5
2
2.5
3
3.5
4
storage period (weeks)
siRNA: NI-7001 (mCTGF sequence) 21 bases/21 bases

FIG 15

NM-7001 (0.1 mg/mL) storage stability

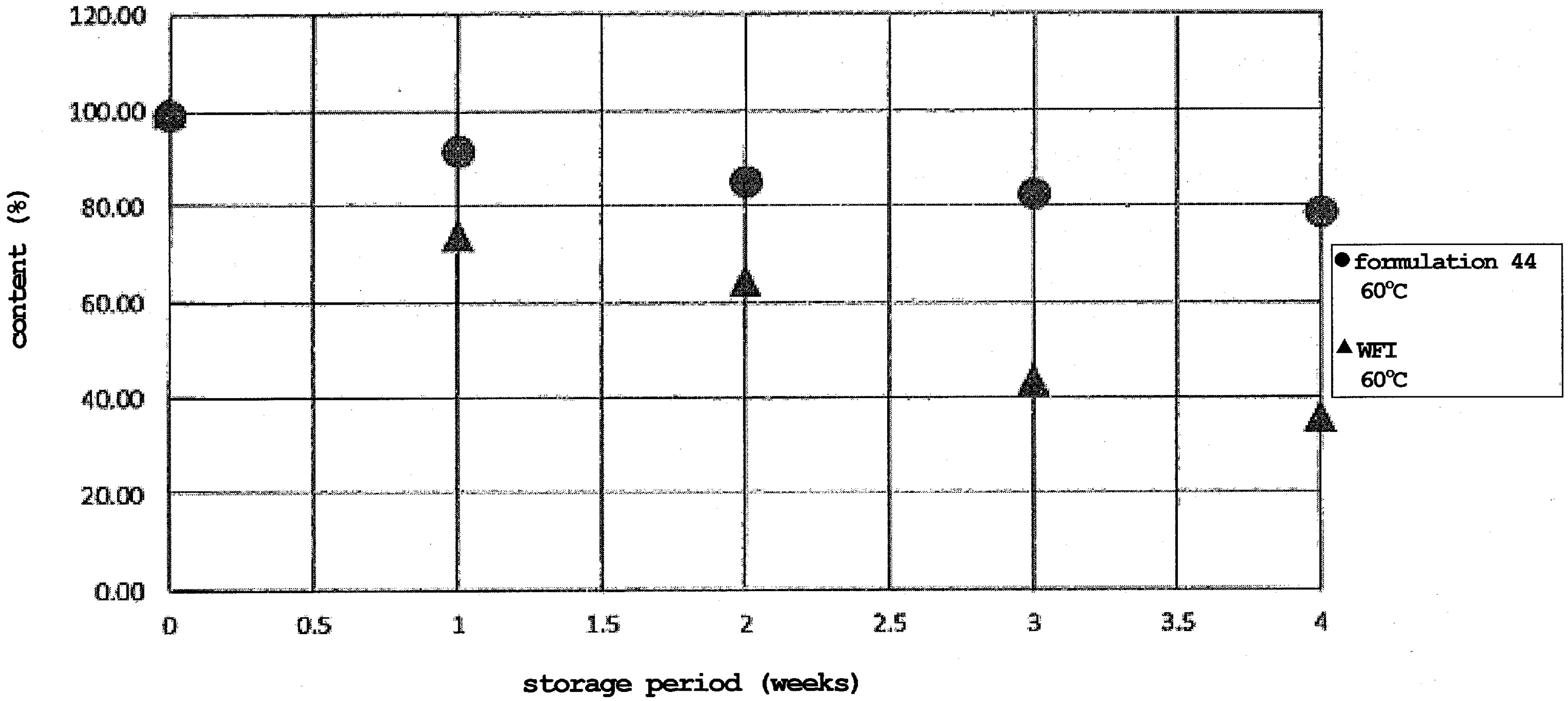


miRNA: NM-7001 (human-let7a-1 precursor sequence) 80 bases

FIG 16

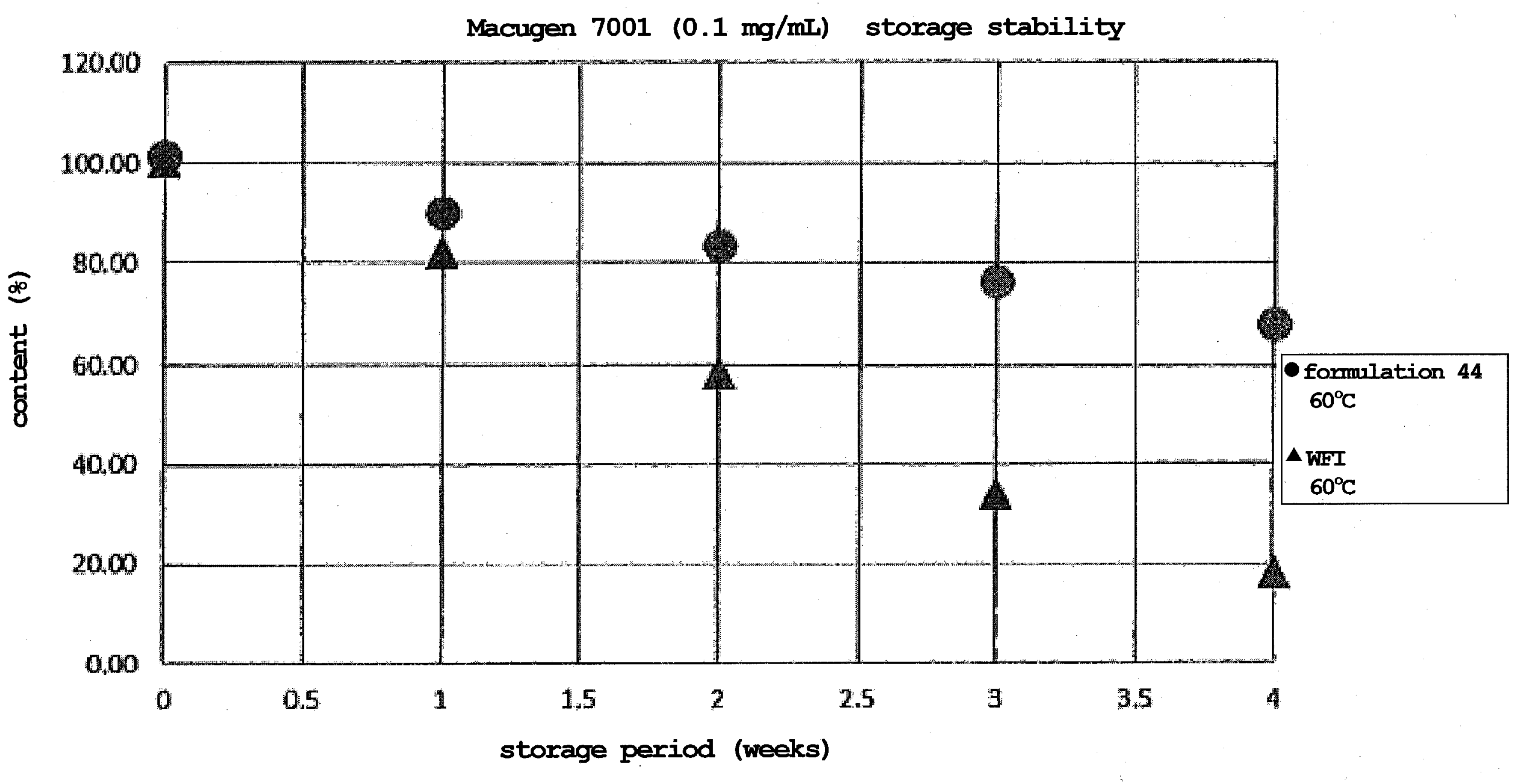


aptamer: Macugen (pegaptanib sodium) unmodified sequence 28 bases

FIG 17

PK-7006 content at each pH of buffer (60°C, on week 4)

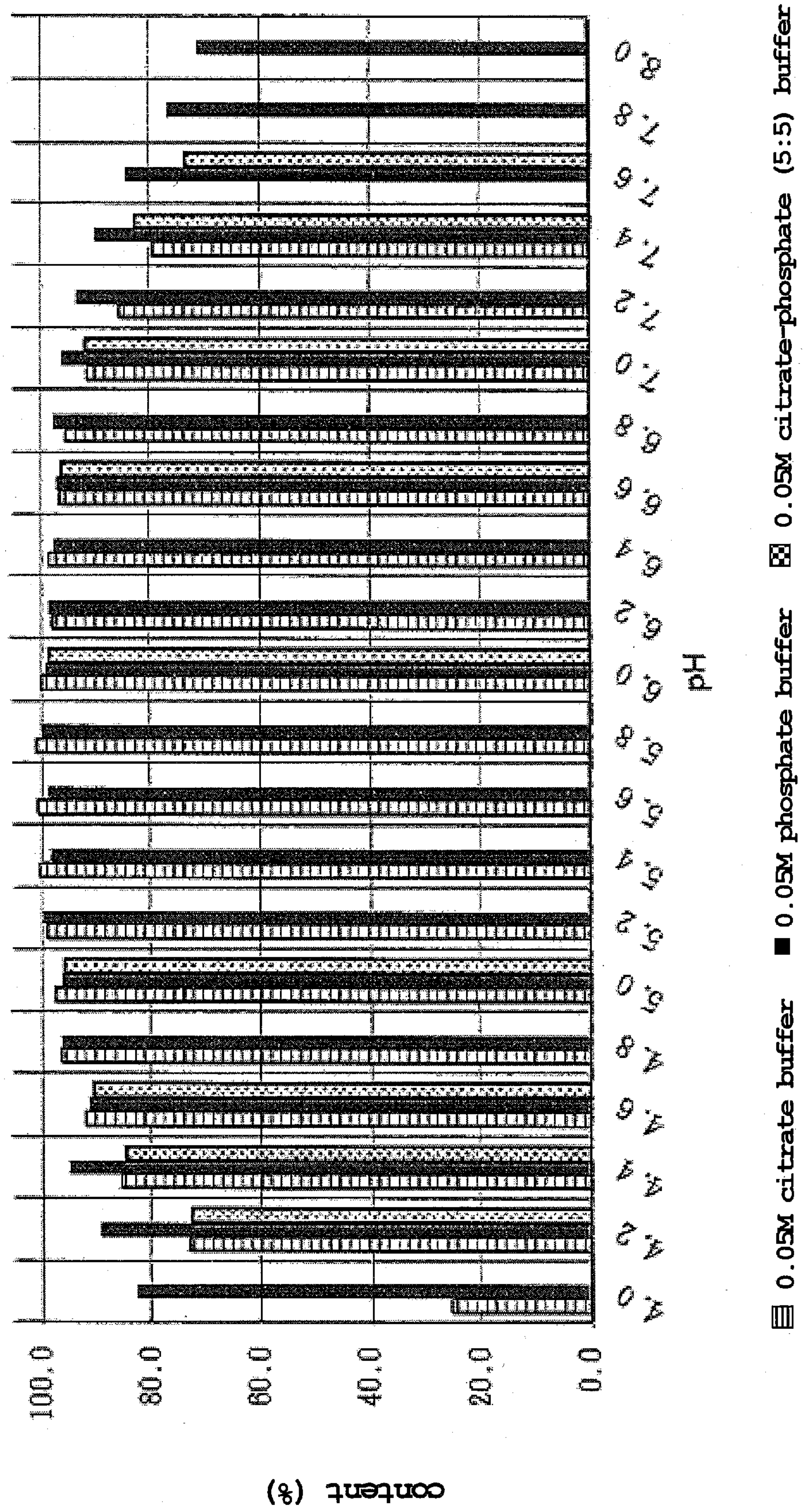

FIG 18

NK-7006 content at each pH of buffer (60°C, on week 4)

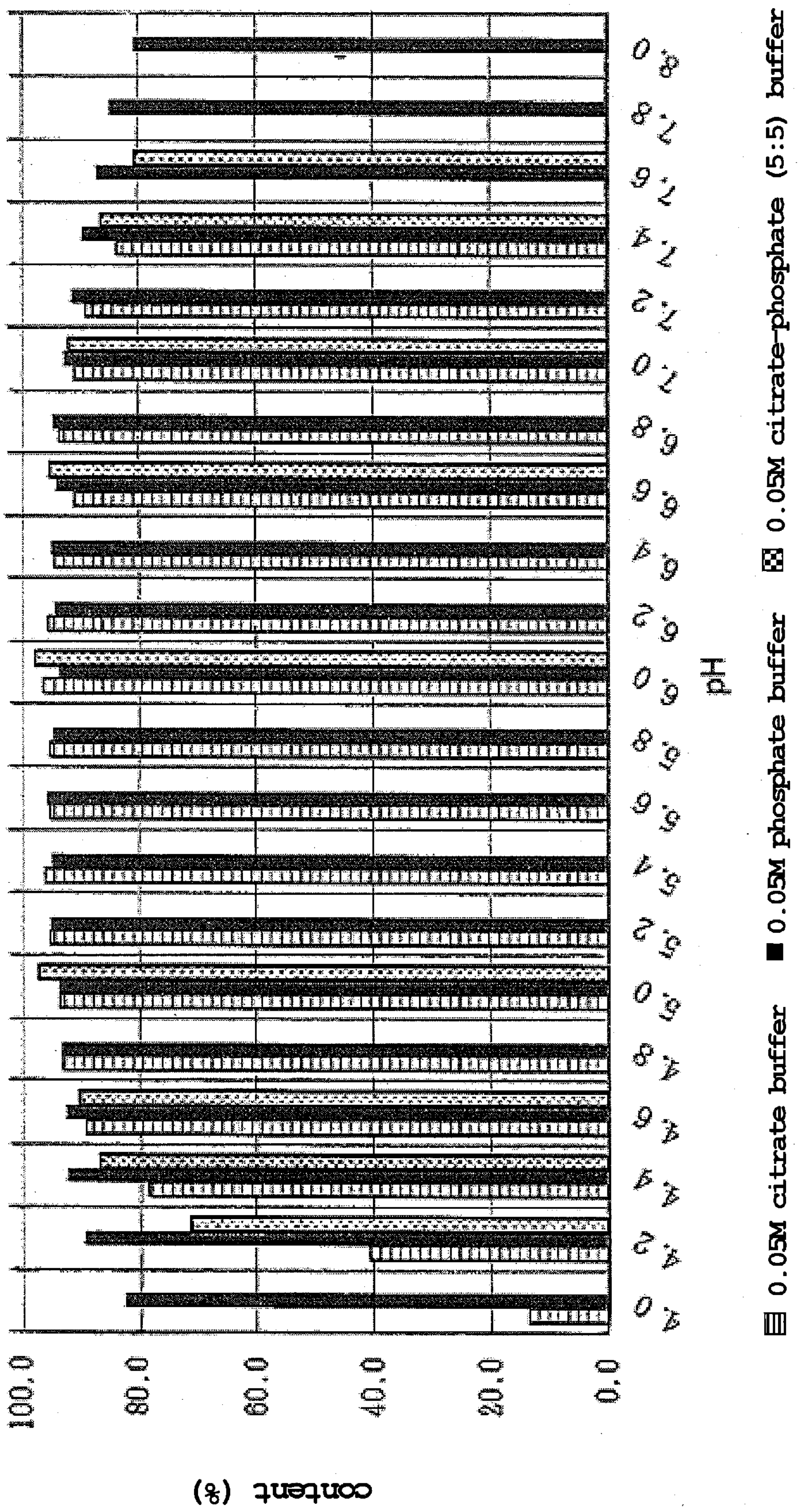

FIG 19

PH-7069 content at each pH of buffer (60°C, on week 4)

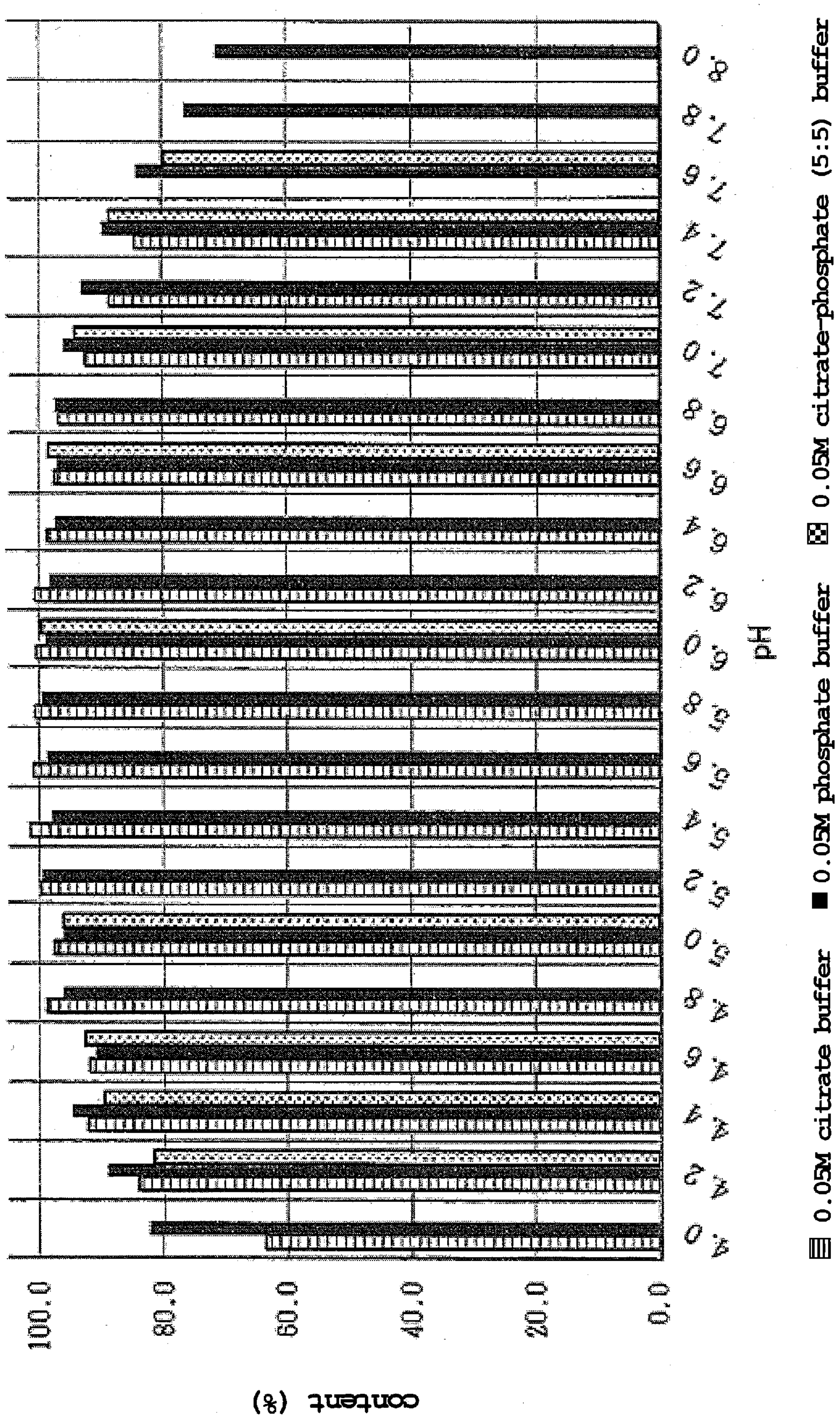

FIG 20

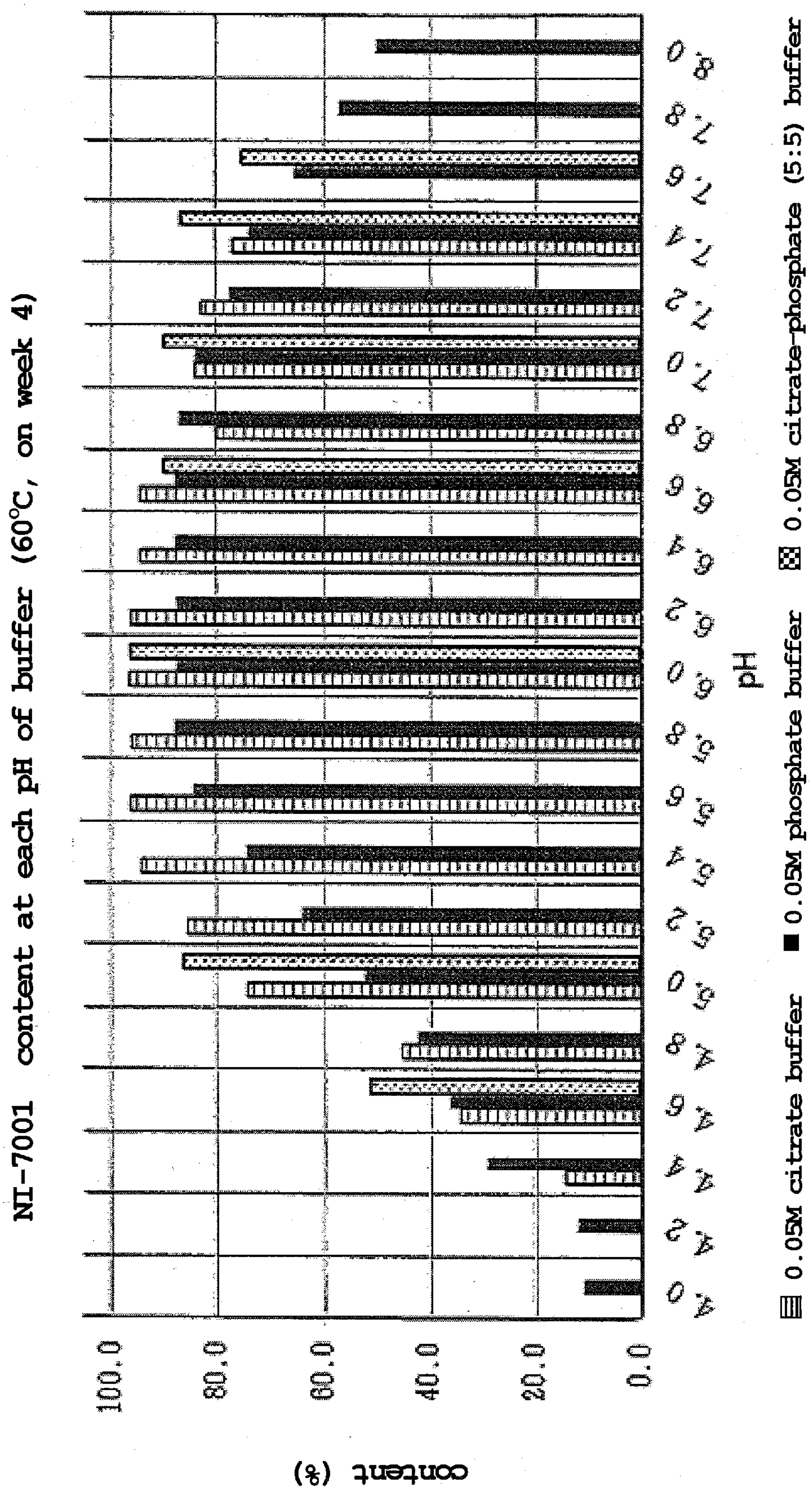
NI-7001 content at each pH of buffer (60°C, on week 4)
content (%)
100.0
80.0
60.0
40.0
20.0
0.0
4.0
4.2
4.4
4.6
4.8
5.0
5.2
5.4
5.6
5.8
6.0
6.2
6.4
6.6
6.8
7.0
7.2
7.4
7.6
7.8
8.0
pH
0.05M citrate buffer
0.05M phosphate buffer
0.05M citrate-phosphate (5:5) buffer

FIG 21

NM-7001 content at each pH of buffer (60°C, on week 4)

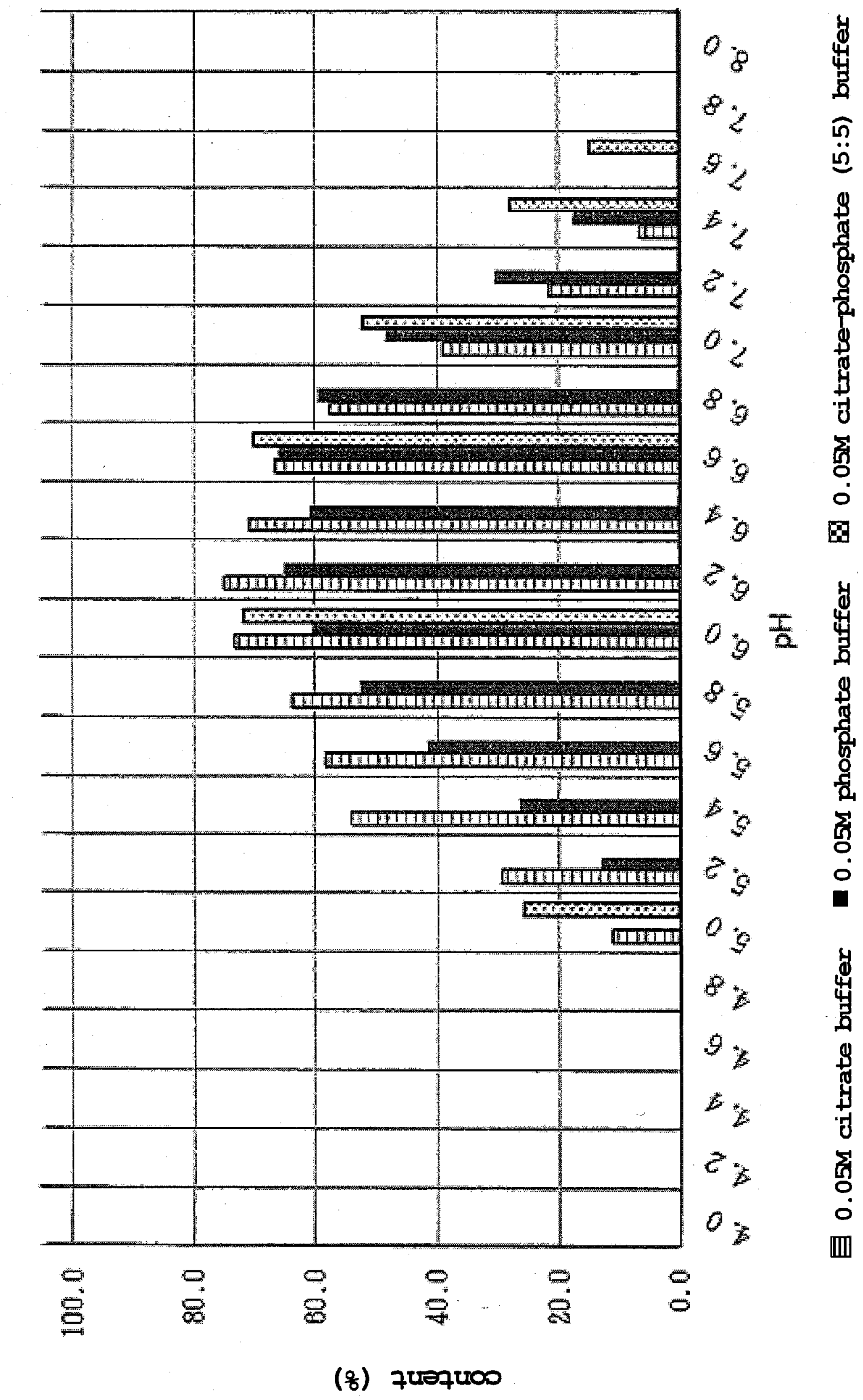

FIG 22

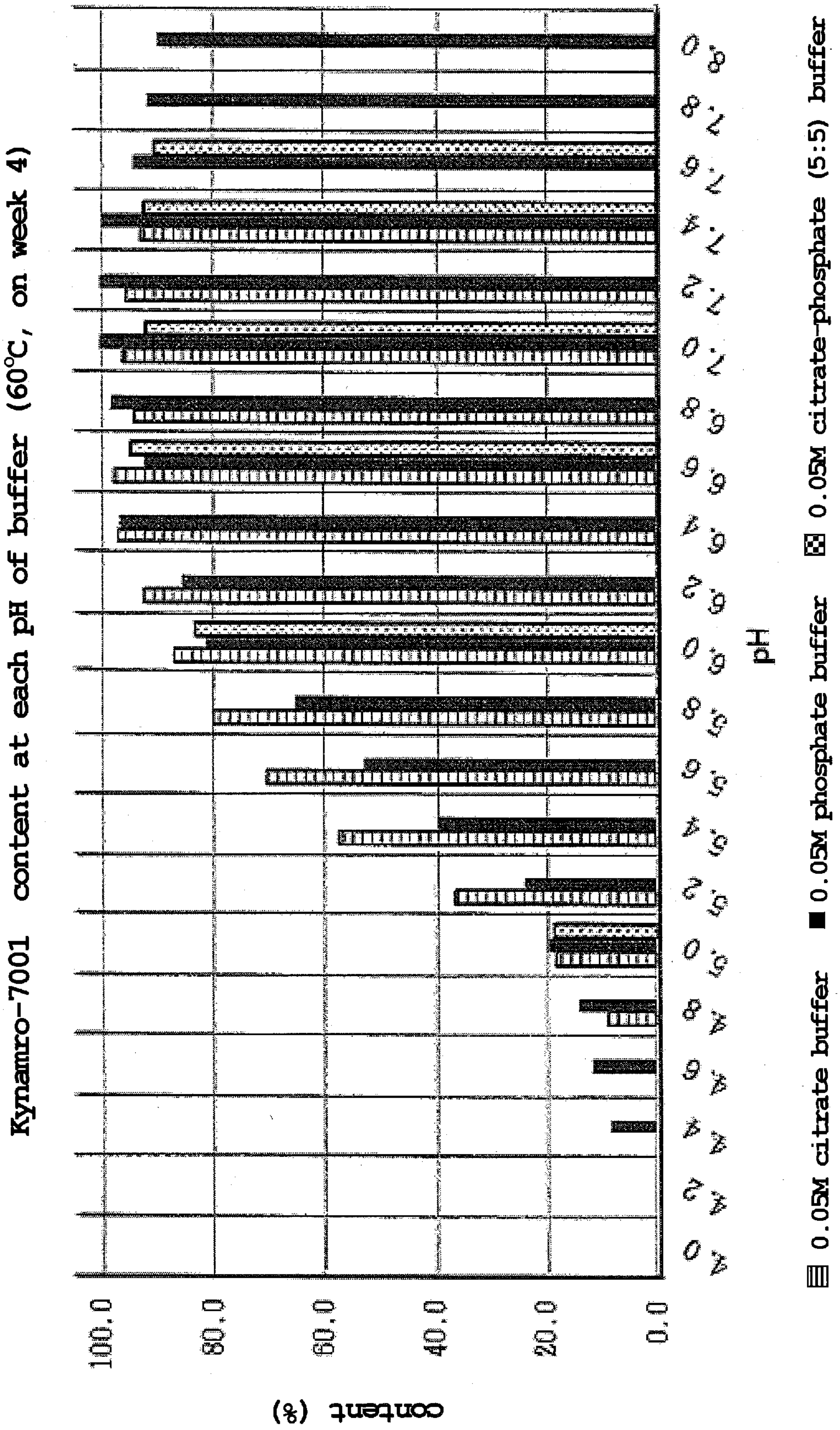
Kynamro-7001 content at each pH of buffer (60°C, on week 4)
content (%)
100.0
80.0
60.0
40.0
20.0
0.0
4.0
4.2
4.4
4.6
4.8
5.0
5.2
5.4
5.6
5.8
6.0
6.2
6.4
6.6
6.8
7.0
7.2
7.4
7.6
7.8
8.0
pH
0.05M citrate buffer
0.05M phosphate buffer
0.05M citrate-phosphate (5:5) buffer

FIG 23

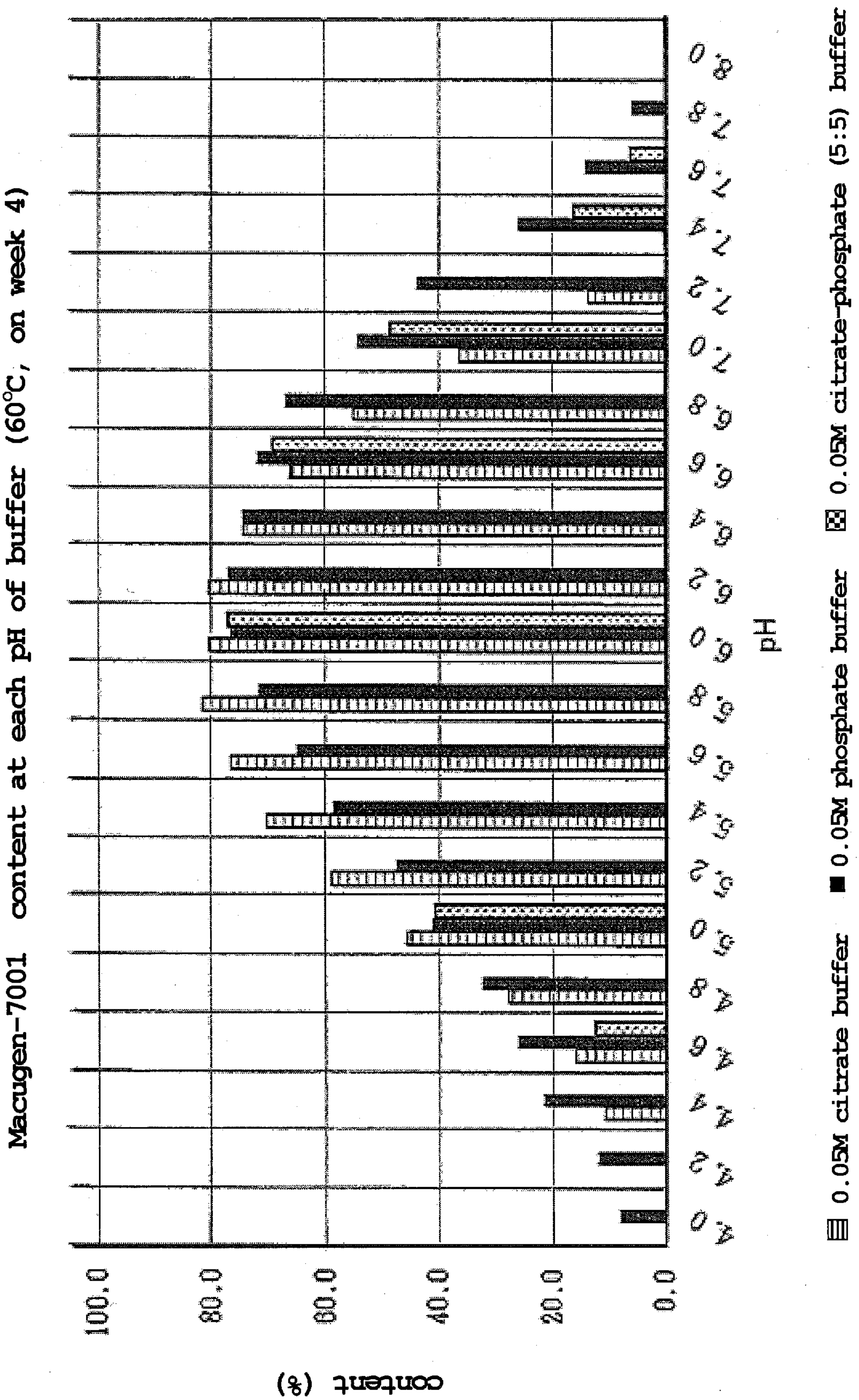
Macugen-7001 content at each pH of buffer (60°C, on week 4)
content (%)
100.0
80.0
60.0
40.0
20.0
0.0
4.0
4.2
4.4
4.6
4.8
5.0
5.2
5.4
5.6
5.8
6.0
6.2
6.4
6.6
6.8
7.0
7.2
7.4
7.6
7.8
8.0
pH
0.05M citrate buffer
0.05M phosphate buffer
0.05M citrate-phosphate (5:5) buffer

## INTERNATIONAL SEARCH REPORT

International application No. PCT/JP2015/080849

A. CLASSIFICATION OF SUBJECT MATTER
*C12N15/113*(2010.01)i, *A61K31/7088*(2006.01)i, *A61K47/12*(2006.01)i, *A61K48/00*(2006.01)i

According to International Patent Classification (IPC) or to both national classification and IPC

B. FIELDS SEARCHED

Minimum documentation searched (classification system followed by classification symbols)
C12N15/113, A61K31/7088, A61K47/12, A61K48/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)
JSTPlus/JMEDPlus/JST7580(JDreamIII), CAplus/MEDLINE/EMBASE/BIOSIS/WPIDS/WPIX(STN)

C. DOCUMENTS CONSIDERED TO BE RELEVANT

| Category* | Citation of document, with indication, where appropriate, of the relevant passages | Relevant to claim No. |
|---|---|---|
| X/Y | JP 2003-524782 A (Antigene Biotech GmbH), 19 August 2003 (19.08.2003), claims 1 to 5; paragraph [0031]; example 5 & US 2003/0143566 A1 paragraph [0031]; example 5; claims 1 to 5 & WO 2001/060517 A2 & EP 1257364 B1 & CN 1434746 A & KR 10-2003-0009361 A | 1-10,16-18/ 1-19 |
| X/Y | JP 2002-522092 A (Antigen GmbH), 23 July 2002 (23.07.2002), example 8 & US 6776959 B1 example 8 & WO 2000/009746 A1 & EP 1105533 A1 & CN 1321200 A | 1-10,16-18/ 1-19 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 January 2016 (06.01.16) | 19 January 2016 (19.01.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No.
PCT/JP2015/080849

C (Continuation). DOCUMENTS CONSIDERED TO BE RELEVANT

| Category* | Citation of document, with indication, where appropriate, of the relevant passages | Relevant to claim No. |
|---|---|---|
| X/Y | JP 2010-233505 A (Toyobo Co., Ltd.), 21 October 2010 (21.10.2010), example 4; fig. 13, 14 (Family: none) | 1-6,9-11,16, 17/1-19 |
| Y | JP 2002-506048 A (American Home Products Corp.), 26 February 2002 (26.02.2002), paragraph [0031] & US 7276359 B1 column 7 & WO 1999/045966 A1 & EP 1061955 B1 & CN 1294520 A | 1-19 |
| A | WO 99/039000 A1 (AKZO NOBEL N.V.), 05 August 1999 (05.08.1999), & JP 2002-501759 A & US 6465639 B1 & EP 1051520 A1 | 1-19 |
| A | JP 2001-242139 A (Hymo Corp.), 07 September 2001 (07.09.2001), & US 6726821 B1 & WO 2001/040789 A1 & EP 1167962 A1 & CN 1327537 A | 1-19 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2708960 B
- JP 3626503 B
- US 7511131 B
- WO 2012017919 A
- WO 2013103146 A
- WO 2012005368 A
- WO 2013077446 A
- WO 2013133393 A
- US 3687808 A
- JP 2014267087 A
- JP 2015081298 A


### Non-patent literature cited in the description


- **J. F. W. MCOMIE.** Protecting Groups in Organic Chemistry. Plenum Press, 1973
- **LIMBACH et al.** Summary: the modified nucleosides of RNA. *Nucleic Acids Res.,* 1994, vol. 22, 2183-2196
- Concise Encyclopedia of Polymer Science and Engineering. John Wiley & Sons, 1990, 858-859
- **ENGLISCH et al.** *Angewandte Chemie, International Edition,* 1991, vol. 30, 613

SEQUENCE LISTING

<110>  BONAC CORPORATION

<120>  Composition stably containing nucleic acid molecule

<130>  092374

<150>  JP 2014-267087
<151>  2014-12-29

<150>  JP 2015-081298
<151>  2015-04-10

<160>  15

<170>  PatentIn version 3.5

<210>  1
<211>  20
<212>  DNA
<213>  Artificial Sequence

<220>
<223>  antisense, Nucleotide at positions 1-5 and 16-20 are RNA.

<400>  1
gccucagtct gcttcgcacc                                                    20

<210>  2
<211>  80
<212>  RNA
<213>  Artificial Sequence

<220>
<223>  miRNA

<400>  2
ugggaugagg uaguagguug uauaguuuua gggucacacc caccacuggg agauaacuau      60

acaaucuacu gucuuuccua                                                    80

<210>  3
<211>  28
<212>  DNA
<213>  Artificial Sequence

<220>
<223>  aptamer, T in position 28 is a 3'-3'-linked deoxythymidine.

<400>  3
cggaaucagu gaaugcuuau acauccgt                                           28

<210>  4
<211>  18
<212>  RNA
<213>  Artificial Sequence

<220>
<223>  expression controling region

<400> 4
uaugcugugu guacucug 18

<210> 5
<211> 19
<212> RNA
<213> Artificial Sequence

<220>
<223> expression controling region

<400> 5
ucgaaguacu cggcguagg 19

<210> 6
<211> 19
<212> RNA
<213> Artificial Sequence

<220>
<223> expression controling region

<400> 6
guugucauau uucucgugg 19

<210> 7
<211> 48
<212> RNA
<213> Artificial Sequence

<220>
<223> nucleic acid molecule

<400> 7
gcagaguaca cacagcauau accgguauau gcugugugua cucugcuu 48

<210> 8
<211> 62
<212> RNA
<213> Artificial Sequence

<220>
<223> nucleic acid molecule

<400> 8
accuacgccg aguacuucga uuccccacac cggaaucgaa guacucggcg uagguucuuc 60

gg 62

<210> 9
<211> 62
<212> RNA
<213> Artificial Sequence

<220>
<223> nucleic acid molecule

<400> 9
accacgagaa auaugacaac ucccccacac cgggaguugu cauauuucuc gugguucuuc 60

gg 62

<210> 10
<211> 51
<212> RNA
<213> Artificial Sequence

<220>
<223> nucleic acid molecule

<400> 10
ggaacucuac cagaaauaua gcccgggcua uauuucuggu agaguuccac g 51

<210> 11
<211> 51
<212> RNA
<213> Artificial Sequence

<220>
<223> nucleic acid molecule

<400> 11
agccuuguac agcgagaucu uuccggaaag aucucgcugu acaaggcuuc g 51

<210> 12
<211> 49
<212> RNA
<213> Artificial Sequence

<220>
<223> nucleic acid molecule

<400> 12
ggugcuccau cuccuacuac gaccggucgu aguaggagau ggagcacca 49

<210> 13
<211> 48
<212> RNA
<213> Artificial Sequence

<220>
<223> nucleic acid molecule

<400> 13
cuuacgcuga guacuucgaa accgguuucg aaguacucag cguaagug 48

<210> 14
<211> 21
<212> RNA
<213> Artificial Sequence

<220>
<223> siRNA

<400> 14

```
gugugaccaa aaguuacaug u                                                21

<210>  15
<211>  21
<212>  RNA
<213>  Artificial Sequence

<220>
<223>  siRNA

<400>  15
auguaacuuu uggucacacu c                                                21
```